# EUROPEAN PATENT APPLICATION

(11) **EP 2 537 934 A2**
(43) Date of publication of application: **26.12.2012**
(21) Application number: 12171878.7
(22) Date of filing: 15.05.2007
(51) Int. Cl.: C12N 15/63, A61K 31/65, C12N 15/85

(54) **Methods of regulating expression of genes or of gene products using substituted tetracycline compounds**

(30) Priority: 15.05.2006 US 800662 P
(62) Divisional of application: 07809091.7
(71) Applicant: Paratek Pharmaceuticals, Inc., Boston, MA 02111 (US)
(72) Inventor: Draper, Michael, Windham, New Hampshire 03087 (US); Nelson, Mark L., Norfolk, MA 02056 (US)
(74) Representative: Johnston, Magnus George

(57) **Abstract**

The present invention relates, at least in part, to the use of substituted tetracycline compounds for regulation of expression of nucleic acids operably linked to a tetracycline operator system. The invention pertains to the compounds used in a regulatory system which utilizes components of the Tet repressor/operator/inducer system of prokaryotes to regulate gene expression in cells. Use of these tetracycline compounds, as featured in the methods of the invention, result in improved dose-response results when compared to those for e.g., tetracycline and doxycycline. Certain methods of the invention thus allow for enhanced control of the Tet repressor/operator/inducer system in regulating gene expression in cells.

## Description

### Related Applications

This application claims priority to U.S. Provisional Patent Application No. 60/800662, filed on May 15, 2006. This application is also related to U. S. Patent Application No. XX/XXX,XXX, filed May 15, 2007. The contents of the aforementioned applications are hereby incorporated in their entirety.

### Background of the Invention

The ability to change the expression level of genes or the timing of their synthesis has great utility for many applications from, for example, analysis of gene function to gene therapy. For this approach, inducible expression systems controlled by an external stimulus are very desirable. Such systems provide an "on/off" status for gene expression and also permit limited expression of a gene at a defined level.

Components of the prokaryotic tetracycline (tet) resistance operon function in eukaryotic cells and have been used to regulate gene expression. A regulatory system which utilizes components of the Tet repressor/operator/inducer system of prokaryotes has been widely used to regulate gene expression in eukaryotic cells. Such systems are known in the art and are described e.g., in U.S. Pat. Nos. 5,888,981; 5,866,755; 5,789,156; 5,654,168; 5,650,298; 6,004,941; 6,271,348; 6,271,341; 6,783,756; 5,464,758; 6,252,136; 5,922,927; 5,912,411; 5,859,310.

Recently, multidrug resistance has become a major problem, with the incidence of resistance increasing dramatically. This problem may make the use of tetracycline less desirable to modulate gene transcription in mammalian subjects, in particular where high doses of the antibiotic must be used in order to effect the desired result. Determination of which substituted tetracycline compounds are most useful in effecting gene regulation would also be of value, as such compounds may not be the same as those most useful in treatment of microbial infection. In addition, not all tetracyclines have the same tissue distribution properties, for example, not all tetracyclines have the ability to cross the blood brain barrier. Accordingly, the development of novel tetracycline derivatives or analogs with improved properties (e.g., altered dose-response profiles or desirable tissue distribution profiles in tetracycline-responsive expression systems) would be of great benefit for *in vitro* and *in vivo* applications.

### Summary of the Invention

The invention is based, at least in part, on the finding that novel substituted tetracycline compounds have improved properties which make them superior for use in modulation of tetracycline controlled gene transcription. The invention pertains to compounds used in a regulatory system which utilizes components of the Tet repressor/operator/inducer system of prokaryotes to regulate expression of gene or gene products in cells and methods of regulating expression of genes or gene products using such compounds. Regulation of expression of genes or gene products by a system of the invention generally involves at least two components: a target nucleic acid sequence which is operatively linked to a regulatory sequence responsive to tetracycline and a protein which, in either the presence or absence of tetracycline, binds to the regulatory sequence and either activates or inhibits transcription of the gene or gene product.

The Tet/repressor/operator/inducer system is functional in cultured cells from a wide variety of organisms (including eukaryotic and prokaryotic cells). In addition, the system works in practically all organisms in which it has been tested, for example, animals plants, unicellular organisms, yeast, fungi, and parasites.

The substituted tetracycline compounds of the invention have been found to be particularly effective for modulation of transcription and may be used in applications for regulation of transcription of genes or gene products known in the art. In particular, in one aspect the invention pertains to methods for using the novel substituted tetracycline compounds of the invention for regulating expression of a target nucleic acid sequence that is under transcriptional control of a tetracycline-responsive promoter element (TRE) in a cell of a subject. In one embodiment, the methods of the invention involve introducing into the cell a first nucleic acid molecule encoding a fusion protein which activates transcription or inhibits transcription in the presence of a substituted tetracycline compound and a second target nucleic acid sequence under the control of a tetracycline-responsive promoter element and modulating the level of tetracycline to which the cell is exposed.

In one embodiment, a fusion protein comprising a Tet repressor (TetR) from the Tc resistance operon of *Escherichia coli* transposon Tn10 fused to a transactivating domain (e.g., that of VP16 from Herpes simplex virus) or a domain (e.g., a dimerization domain) which recruits a transcriptional activator (e.g., an endogenous transcriptional activator) to interact with the fusion protein by a protein-protein interaction (e.g., a non-covalent interaction) is introduced into a cell. This construct is referred to as a tet-controlled transactivator (tTA) and it regulates expression of a target nucleic acid sequence that is under transcriptional control of a tetracycline-responsive promoter element (TRE). The TRE is made up of at least one Tet operator (tetO) sequence (e.g. one or more, including, e.g., concatemerized or multimerized tetO sequences) fused to a minimal promoter (for example, to a minimal RNA polymerase II promoter or modified promoter of RNA polymerases I and III, which are transcriptionally silent in the absence of tTA). One example for minimal promoter sequence was derived from the human cytomegalovirus (hCMV) immediate-early promoter. In the absence of tetracycline, tTA binds to the TRE and activates transcription of the target nucleic acid sequence. In the presence of tetracycline, tTA can not bind to the TRE, and expression from the target nucleic acid sequence remains inactive.

In another embodiment, a fusion protein comprised of a modified form of the tet repressor (TetR) and a transactivation domain or a domain (e.g., a dimerization domain) which recruits a transcriptional activator (e.g., an endogenous transcriptional activator) to interact with the fusion protein by a protein-protein interaction (e.g., a non-covalent interaction) is introduced into a cell. This construct is referred to as a reverse tetracycline-controlled transactivator (rtTA). The modification of TetR generally involves amino acid changes in TetR which alter its DNA binding characteristics so that it can only recognize the tetO sequences in the target transgene in the presence of tetracyclines. Thus, transcription of the TRE-regulated target nucleic acid sequence is stimulated by rtTA only in the presence of tetracycline.

In another embodiment, a tetracycline compound of the invention can be used to regulate transcription of a gene or gene product under the control of a native Tet operator and in the presence of a native TetR in a cell. In one embodiment, the invention pertains to such a cell. For example, JE305K is a strain that has disrupted acrB and waaP genes and comprises a plasmid which contains tetR, and the luxCDABE operon (from *P. luminescens*) under the regulation of the tetA promotor/operator.

In one embodiment, the first and second nucleic acid molecule can be within a single molecule (e.g., in the same vector). In another embodiment, the first and second nucleic acid molecule are present on separate molecules.

The methods of the invention allow for regulation of a gene or gene product which is an endogenous gene of the cell which has been operatively linked to at least one TRE. Alternatively, the TRE-linked gene can be an exogenous gene or gene products which has been introduced into the cells.

According to the methods of the invention gene transcription can be regulated in vitro or in vivo.

In another embodiment, the method involves obtaining a cell from a subject, modifying the cell ex vivo to contain one or more of the aforementioned nucleic acid molecules, administering the modified cell to the subject and modulating the concentration of substituted tetracycline compound of the invention in the subject.

### Brief Description of the Drawings

Figure 1A is a graph showing the dose response curve for doxycycline.
Figure 1B is a graph showing the dose response curve for 5-cyclobutanoate doxycycline.
Figure 1C is a graph showing the dose response curve for 5-cyclohexanoate doxycycline.
Figure 1D is a graph showing the dose response curve for 5-propionyl-7-cyclopentylacetylamino doxycycline.
Figure 1E is a graph showing the dose response curve for 7-acetylamino doxycycline.
Figure 1F is a graph showing the dose response curve for 9-1'-methylcyclopentyl doxycycline.
Figure 1G is a graph showing the dose response curve for 9-1'-methylcyclobutyl -doxycycline.
Figure 1H is a graph showing the dose response curve for 9-t-butyl-7-methyl doxycycline.
Figure 2A is a depiction of the effect of doxycycline on 34R mutants.
Figure 2B is a depiction of the effect of doxycyline on MT2 mutants.
Figures 2C is a depiction of the effect of 9-t-butyl doxycycline on 34R mutants.
Figure 2D is a depiction of the effect of 9-t-butyl doxycycline on MT2 mutants.
Figure 3A is a graph showing the dose response curve for doxycycline.
Figure 3B is a graph showing the dose response curve for 5-cyclobutanoate doxycycline.
Figure 3C is a graph showing the dose response curve for 5-cyclohexanoate doxycycline.
Figure 3D is a graph showing the dose response curve for 5-propionyl-7-cyclopentylacetylamino doxycycline.
Figure 3E is a graph showing the dose response curve for 7-acetylamino doxycycline.
Figure 3F is a graph showing the dose response curve for 9-1'-methylcyclopentyl doxycycline.
Figure 3G is a graph showing the dose response curve for 9-1'-methylcyclobutyl doxycycline.
Figure 3H is a graph showing the dose response curve for 9-t-butyl-7-methylthiomethyl doxycycline.
Figure 3I is a graph showing the dose response curve for 9-t-butyl-7-methyl doxycycline.
Figure 4 is a digital image of luciferase expression in the mice.

### Detailed Description of the Invention

This invention pertains, at least in part, to the use of substituted tetracycline compounds to modulate a tetracycline-responsive expression system that can be used to regulate the expression of genes or gene products in cells or organisms in a highly controlled manner. Exemplary systems in which the subject compound may be employed are known in the art. In one embodiment, regulation of expression by a system of the invention involves at least two components: a gene which is operatively linked to a regulatory sequence and a protein which, in either the presence or absence of an inducible agent, binds to the regulatory sequence and either activates or inhibits transcription of the gene. The invention utilizes components of the Tet repressor/operator/inducer system of prokaryotes to modulate gene expression in eukaryotic cells.

Various aspects of the invention pertain to substituted tetracycline compounds that modulate the activity of fusion proteins which are capable of either activating or inhibiting transcription of a gene linked to a TRE. Such fusion proteins bind to tetracycline responsive elements only in the presence or, alternatively, in the absence of substituted tetracycline compounds. Thus, in a host cell, transcription of a gene operatively linked to a TRE may be stimulated or inhibited by a fusion protein of the invention by altering the concentration of a substituted tetracycline compound in contact with the host cell (e.g., adding or removing or changing the concentration of a substituted tetracycline compound from a culture medium, or administering or ceasing to administer or changing the concentration administered of a substituted tetracycline compound to a host organism, etc.).

The instant invention pertains to substituted tetracycline compounds that have beneficial properties (e.g., are non-antibiotic and/or have enhanced activity) in gene regulatory systems responsive to tetracyclines. Such regulatory systems are known in the art (e.g., are described in US patents 5,888,981; 5,866,755; 5,789,156; 5,654,168; 5,650,298; 6,004,941; 6,271,348; 6,271,341; 6,783,756; 5,464,758; 6,252,136; 5,922,927; 5,912,411; 5,859,310, the contents of each of these patents are incorporated herein by reference in their entirety).

Methods for stimulating or inhibiting transcription of a gene using substituted tetracycline compounds, and kits which contain the components of the regulatory system described herein, are also encompassed by the invention. Various aspects of the invention and exemplary regulatory systems are discussed in greater detail below. It will be understood by those skilled in the art that the subject tetracycline compounds may be used in other application in which tetracycline has been used to regulate expression of genes and gene products in the art.

### Definitions

The term "tetracycline"" includes unsubstituted and substituted tetracycline compounds.

The term "substituted tetracycline compound" includes derivatives or analogs of tetracycline compounds or compounds with a similar ring structure to tetracycline. Examples of substituted tetracycline compounds include: chlortetracycline, oxytetracycline, demeclocycline, methacycline, sancycline, chelocardin, rolitetracycline, lymecycline, apicycline; clomocycline, guamecycline, meglucycline, mepylcycline, penimepicycline, pipacycline, etamocycline, penimocycline, etc. Other derivatives and analogues comprising a similar four ring structure are also included (See Rogalski, "Chemical Modifications of Tetracyclines," the entire contents of which are hereby incorporated herein by reference). Table 1 depicts tetracycline and several known tetracycline derivatives.

**Table 1**

| | | |
|---|---|---|
| 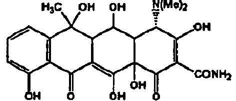 | 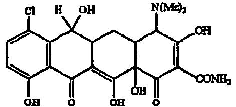 | 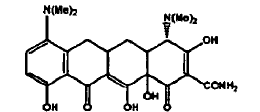 |
| Oxytetrycline | Demeclocyne | Minocycline |
| 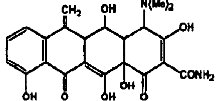 | 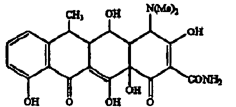 | 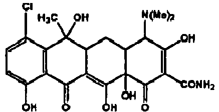 |
| methacycline | Doxycycline | Chlortetracycline |
| 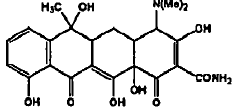 | 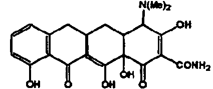 | 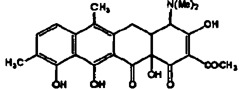 |
| Tetracycline | Sancycline | Chelocardin |

Other substituted tetracycline compounds which may be used in the methods of the invention include, but are not limited to, 6-demethyl-6-deoxy-4-dedimethylaminotetracycline; tetracyclino-pyrazole; 7-chloro-4-dedimethylaminotetracycline; 4-hydroxy-4-dedimethylaminotetracycline; 12α-deoxy-4-dedimethylaminotetracycline; 5-hydroxy-6α-deoxy-4-dedimethylaminotetracycline; 4-dedimethylamino-12α-deoxyanhydrotetracycline; 7-dimethylamino-6-demethyl-6-deoxy-4-dedimethylaminotetracycline; tetracyclinonitrile; 4-oxo-4-dedimethylaminotetracycline 4,6-hemiketal; 4-oxo-11a Cl-4-dedimethylaminotetracycline-4,6-hemiketal; 5a,6-anhydro-4-hydrazon-4-dedimethylamino tetracycline; 4-hydroxyimino-4-dedimethylamino tetracyclines; 4-hydroxyimino-4-dedimethylamino 5a,6-anhydrotetracyclines; 4-amino-4-dedimethylamino-5a, 6 anhydrotetracycline; 4-methylamino-4-dedimethylamino tetracycline; 4-hydrazono-11a-chloro-6-deoxy-6-demethyl-6-methylene-4-dedimethylamino tetracycline; tetracycline quaternary ammonium compounds; anhydrotetracycline betaines; 4-hydroxy-6-methyl pretetramides; 4-keto tetracyclines; 5-keto tetracyclines; 5a, 11a dehydro tetracyclines; 11a Cl-6, 12 hemiketal tetracyclines; 11a Cl-6-methylene tetracyclines; 6, 13 diol tetracyclines; 6-benzylthiomethylene tetracyclines; 7, 11a-dichloro-6-fluoro-methyl-6-deoxy tetracyclines; 6-fluoro (α)-6-demethyl-6-deoxy tetracyclines; 6-fluoro (β)-6-demethyl-6-deoxy tetracyclines;6-α acetoxy-6-demethyl tetracyclines; 6-β acetoxy-6-demethyl tetracyclines; 7, 13-epithiotetracyclines; oxytetracyclines; pyrazolotetracyclines; 11a halogens of tetracyclines; 12a formyl and other esters of tetracyclines; 5, 12a esters of tetracyclines; 10, 12a- diesters of tetracyclines; isotetracycline; 12-a-deoxyanhydro tetracyclines; 6-demethyl-12a-deoxy-7-chloroanhydrotetracyclines; B-nortetracyclines; 7-methoxy-6-demethyl-6-deoxytetracyclines; 6-demethyl-6-deoxy-5a-epitetracyclines; 8-hydroxy-6-demethyl-6-deoxy tetracyclines; monardene; chromocycline; 5a methyl-6-demethyl-6-deoxy tetracyclines; 6-oxa tetracyclines, and 6 thia tetracyclines.

The term "substituted tetracycline compound" is intended to include compounds which are structurally related to tetracycline and which bind to the Tet repressor with a Kₐ of at least about 10⁶ M⁻¹. Substituted tetracycline compounds are generally of formula (I) and (II). Preferably, the substituted tetracycline compound binds with an affinity of about 10⁹ M⁻¹ or greater. In one embodiment, the term "substituted tetracycline compound" does not include unsubstituted tetracycline compounds such as minocycline, doxycycline, tetracycline, anhydrotetracycline, doxycycline, chlorotetracycline, oxytetracycline and others disclosed by Hlavka and Boothe, "The Tetracyclines," in Handbook of Experimental Pharmacology 78, R K. Blackwood et al. (eds.), Springer-Verlag, Berlin-New York, 1985; L. A. Mitscher, "The Chemistry of the Tetracycline Antibiotics", Medicinal Research 9, Dekker, New York, 1978; Noyee Development Corporation, "Tetracycline Manufacturing Processes" Chemical Process Reviews, Park Ridge, N.J., 2 volumes, 1969; R C. Evans, "The Technology of the Tetracyclines", Biochemical Reference Series 1, Quadrangle Press, New York, 1968; and H. F. Dowling, "Tetracycline", Antibiotic Monographs, no. 3, Medical Encyclopedia, New York, 1955. A substituted tetracycline compound may have a reduced antibiotic activity compared to tetracycline.

The term "substituted tetracycline compound" includes tetracycline compounds with one or more additional substituents, e.g., at the 1, 2, 3, 4, 5, 6, 7, 8, 9, 10, 11, 11 a, 12, 12a or 13 position or at any other position which allows the substituted tetracycline compound of the invention to perform its intended function, e.g., modulate gene expression or gene products.

Examples of substituted tetracycline compounds include compounds described in U.S. Patents Nos. 6,165,999; 5,834,450; 5,886,175; 5,567,697; 5,567,692; 5,530,557; 5,512,553; 5,430,162, each of which is incorporated herein by reference in its entirety. Other examples of substituted tetracycline compounds include those described in, for example, WO 03/079984, WO 03/075857, WO 03/057169, WO 02/072545, WO 02/072532, WO 99/37307, WO 02/12170, WO 02/04407, WO 02/04406, WO 02/04404, WO 01/98260, WO 01/98259, WO 01/98236, WO 01/87824, WO 01/74761, WO 01/52858, WO 01/19784, WO 84/01895, U.S.S.N. 60/367,050, U.S.S.N. 09/895,797, U.S.S.N. 60/305,546, U.S.S.N. 60/346,930, U.S.S.N. 60/346,929, U.S.S.N. 60/347,065, U.S.S.N. 60/346,956, U.S.S.N. 60/367,049, U.S.S.N. 10/097,095, U.S.S.N. 10/097,135, U.S.S.N. 60/362,654, U.S.S.N. 60/367,045, U.S.S.N. 60/366,915, U.S.S.N. 60/367,048, U.S.S.N. 11/258,622, U.S.S.N. 11/258,613, U.S.S.N. 11/348,608, U.S.S.N. 60/701,730 and U.S.S.N. 11/490,867. Other examples of substituted tetracycline compounds are described in EP 0582810 B1;EP. 0536 515B1; EP 0582 789B1; EP 0582 829B1; EP 0582788B1; US 5,530,117; US 5,495,030; US 5,495,018; US 5,494,903; US 5,466,684; EP 0535 346B1; US 5,457,096; US 5,442,059; US 5,430,162; US 5,420,272; US 5,401,863; US 5,401,729; US 5,386,041; US 5,380,888; US 5,371,076; EP 618 190; US 5,326,759; EP 582 829; EP 528 810; EP 582 790; EP 582 789; EP 582 788; US 5,281,628; EP 536 515; EP 535 346; WO 96/34852; WO 95/22529A1; US 4,066,694; US 3,862,225; US 3,622,627; WO 01/87823A1; WO 00/28983A1; WO 07/014,154; WO 06/084,265; WO 06/047,671; WO 06/047,756; US 2006-0287283; WO 06/549,717; and US 2005-0143352. Each of these aforementioned applications and patents are hereby incorporated herein by reference in its entirety.

The term "fusion protein" includes a polypeptide comprising an amino acid sequence derived from two different polypeptides, typically from different sources (e.g., different cells and/or different organisms) which are operatively linked. For such polypeptides, the term "operatively linked" is intended to mean that the two polypeptides are connected in manner such that each polypeptide can serve its intended function. Typically, the two polypeptides are covalently attached through peptide bonds. The fusion protein is generally produced by standard recombinant DNA techniques. For example, a DNA molecule encoding the first polypeptide is ligated to another DNA molecule encoding the second polypeptide, and the resultant hybrid DNA molecule is expressed in a host cell to produce the fusion protein. The DNA molecules are ligated to each other in a 5' to 3' orientation such that, after ligation, the translational frame of the encoded polypeptides is not altered (i.e., the DNA molecules are ligated to each other in-frame).

The term "heterologous" is intended to mean that the second polypeptide is derived from a different protein than the first polypeptide. Like the transactivator fusion proteins, the transcriptional silencer fusion proteins can be prepared using standard recombinant DNA techniques as described herein.

A transcriptional regulator of the methods of the invention can be used to regulate transcription of an exogenous nucleotide sequence introduced into the host cell or animal. An "exogenous" nucleotide sequence is a nucleotide sequence which is introduced into the host cell and typically is inserted into the genome of the host. The exogenous nucleotide sequence may not be present elsewhere in the genome of the host (e.g., a foreign nucleotide sequence) or may be an additional copy of a sequence which is present within the genome of the host but which is integrated at a different site in the genome. An exogenous nucleotide sequence to be transcribed and an operatively linked tet operator sequence(s) can be contained within a single nucleic acid molecule which is introduced into the host cell or animal.

Alternatively, a transcriptional regulator of the methods of the invention can be used to regulate transcription of an endogenous nucleotide sequence to which a tet operator sequence(s) has been linked. An "endogenous" nucleotide sequence is a nucleotide sequence which is present within the genome of the host. An endogenous gene can be operatively linked to a tet operator sequence(s) by homologous recombination between a recombination vector comprising a TRE and sequences of the endogeneous gene. For example, a homologous recombination vector can be prepared which includes at least one tet operator sequence and a miminal promoter sequence flanked at its 3' end by sequences representing the coding region of the endogenous gene and flanked at its 5' end by sequences from the upstream region of the endogenous gene by excluding the actual promoter region of the endogenous gene. The flanking sequences are of sufficient length for successful homologous recombination of the vector DNA with the endogenous gene. Preferably, several kilobases of flanking DNA are included in the homologous recombination vector. Upon homologous recombination between the vector DNA and the endogenous gene in a host cell, a region of the endogenous promoter is replaced by the vector DNA containing one or more tet operator sequences operably linked to a minimal promoter. Thus, expression of the endogenous gene is no longer under the control of its endogenous promoter but rather is placed under the control of the tet operator sequence(s) and the minimal promoter.

The term "tet operator sequence" is intended to encompass all classes of tet operators (e.g., A, B, C, D and E). A nucleotide sequence to be transcribed can be operatively linked to a single tet operator sequence, or for an enhanced range of regulation, it can be operatively linked to multiple copies of a tet operator sequence or multiple tet operator sequences (e.g., two, three, four, five, six, seven, eight, nine, ten or more operator sequences). In a preferred embodiment, the sequence to be transcribed is operatively linked to seven tet operator sequences.

As used herein, the terms "tet operator" and "tet operator sequence" encompass all classes oftet operator sequences, e.g. class A, B, C, D, and E. Nucleotide sequences of these five classes oftet operators are presented in US 6,271,348, and are additionally described in Waters, S. H. et al. (1983) Nucleic Acid Research 11(17):6089-6105, Hillen, W. and Schollenmeier, K. (1983) Nucleic Acid Research 11(2):525-539, Stuber, D. and Bujard, H. (1981) Proc. Natl. Acad. Sci. USA 78:167-171, Unger, B. et al. (1984) nucleic Acid Research 12(20):7693-7703 and Tovar, K. et al. (1988) Mol. Gen. Genet. 215:76-80. In certain embodiments, the mutated Tet repressor is a Tn10-encoded repressor (i.e., class B) and the tet operator sequence is a class B tet operator sequence. Alternatively, a mutated class A Tet repressor can be used with a class A tet operator sequence, and so on for the other classes of Tet repressor/operators.

As used herein, "repression" of transcription is intended to mean a diminution in the level or amount of transcription of a target nucleic acid sequence compared to the level or amount of transcription prior to regulation by the transcriptional silencer protein. Transcriptional inhibition may be partial or complete.

The term "Tet repressor" includes a protein occurring in nature or modified forms thereof which regulate transcription from Tet operator sequences in prokaryotic cells in the absence or presence of tetracycline. The term "wild-type Tet repressor" is intended to describe a protein occurring in nature which represses transcription from Tet operator sequences in prokaryotic cells in the absence of tetracycline. The term "mutated Tet repressor" is intended to include polypeptides having an amino acid sequence which is similar to a wild-type Tet repressor but which has at least one amino acid difference from the wild-type Tet repressor. Such mutated Tet repressors may be modified in function such that transcription of a TRE-regulated target nucleic acid sequence is stimulated by the repressor in the presence of tetracycline

The phrase "operably linked" or "operatively linked", when used in reference to nucleotide sequences, means that the nucleotide sequence of interest (*e.g.,* the sequence that encodes a polypeptide to be expressed in a tetracycline-responsive manner) is linked to the regulatory sequence(s) (e.g., the tet operator, for nucleotide sequences that are "tet operator-linked nucleotide sequences") in a manner which allows for expression of the nucleotide sequence (*e.g.,* in a host cell when the construct is introduced into the host cell or in an *in vitro* transcription/translation system). The term "regulatory sequence" is art-recognized and intended to include promoters, enhancers and other expression control elements (e.g., polyadenylation signals). Such regulatory sequences are known to those skilled in the art and are described in Goeddel, Gene Expression Technology: Methods in Enzymology 185, Academic Press, San Diego, Calif. (1990). Regulators sequences include those which direct constitutive expression of a nucleotide sequence in many types of host cell and those which direct expression of the nucleotide sequence only in certain host cells (*e.g.,* tissue-specific regulatory sequences). Other elements included in the design of a particular expression vector can depend on such factors as the choice of the host cell to be transformed, the level of expression of protein desired, *etc.* The expression constructs of the invention can be introduced into host cells to thereby produce proteins or peptides, including fusion proteins or peptides, encoded by nucleic acids as described herein.

Within a transcription unit, the "nucleotide sequence to be transcribed" typically includes a minimal promoter sequence of which only a downstream part is transcribed and which serves (at least in part) to position the transcriptional machinery for transcription. The minimal promoter sequence is linked to the transcribed sequence of interest in a 5' to 3' direction by phosphodiester bonds (i.e., the promoter is located upstream of the transcribed sequence of interest) to form a contiguous nucleotide sequence. Accordingly, as used herein, the terms "nucleotide sequence to be transcribed" or "target nucleotide sequence" are intended to include both the nucleotide sequence which is transcribed into mRNA and an operatively linked upstream minimal promoter sequence. The term "minimal promoter" includes partial promoter sequences which define the start site of transcription for the linked sequence to be transcribed but which by itself is not capable of initiating transcription efficiently, if at all. Thus, the activity of such a minimal promoter is dependent upon the binding of a transcriptional activator (such as the tetracycline-inducible fusion protein of the invention) to an operatively linked regulatory sequence (such as one or more tet operator sequences). In one embodiment, the minimal promoter is from the human cytomegalovirus (as described in Boshart et al. (1985) Cell 41:521-530). Preferably, nucleotide positions between about +75 to -53 and +75 to -31 are used. Other suitable minimal promoters are known in the art or can be identified by standard techniques. For example, a functional promoter which activates transcription of a contiguously linked reporter gene (e.g., chloramphenicol acetyl transferase, β-galactosidase or luciferase) can be progressively deleted until it no longer activates expression of the reporter gene alone but rather requires the presence of an additional regulatory sequence(s).

The term "a polypeptide which activates transcription in eukaryotic cells" as used herein includes polypeptides which either directly or indirectly activate transcription.

The term "in a form suitable for expression of the fusion protein in a host cell" is intended to mean that the recombinant expression vector includes one or more regulatory sequences operatively linked to the nucleic acid encoding the fusion protein in a manner which allows for transcription of the nucleic acid into mRNA and translation of the mRNA into the fusion protein.

The term "host cell" includes eukaryotic or prokaryotic cells or cell lines. Non-limiting examples of mammalian cell lines which can be used include CHO dhfr.sup.-cells (Urlaub and Chasin (1980) Proc. Natl. Acad. Sci. USA 77:4216-4220), 293 cells (Graham et al. (1977) J. Gen. Virol..36: pp59) or myeloma cells like SP2 or NS0 (Galfre and Milstein (1981) Meth. Enzymol. 73(B):3-46).

The term "subject" includes humans and other non-human mammals including monkeys, cows, goats, sheep, dogs, cats, rabbits, rats, mice, and.transgenic and homologous recombinant species thereof. Furthermore, the term "subject" includes non-mammalian animals such as insects, amphibians, unicellular organisms, parasites, plants, such as transgenic plants, etc.

The terms "transformation" and "transfection" refer to a variety of art-recognized techniques for introducing foreign nucleic acid (e*.g*., DNA) into a host cell, including calcium phosphate or calcium chloride co-precipitation, DEAE-dextran-mediated transfection, lipofection, or electroporation. Suitable methods for transforming or transfecting host cells can be found in Sambrook, et al. (Molecular Cloning: A Laboratory Manual. 2nd, ed, Cold Spring Harbor Laboratory, Cold Spring Harbor Laboratory Press, Cold Spring Harbor, NY, 1989), and other laboratory manuals.

The term "homologous recombinant organism" as used herein describes an organism, e.g. animal, plant, or unicellular organism containing a gene which has been modified by homologous recombination between the gene and a DNA molecule introduced into a cell of the animal, e.g., an embryonic cell of the animal. In one embodiment, the non-human animal is a mouse, although the invention is not limited thereto. An animal can be created in which nucleic acid encoding the fusion protein has been introduced into a specific site of the genome, i.e., the nucleic acid has homologously recombined with an endogenous gene.

### L Tetracycline Compounds

In one embodiment, the substituted tetracycline compound for use in the methods of the invention is of formula (I): 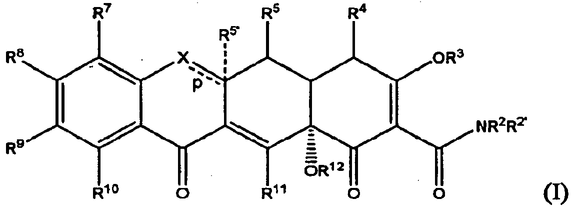 wherein
p is a single or double bond;
X is CHC(R¹³Y'Y) CR^{6'}R⁶, C=CR^{6'}R⁶, S, NR⁶, or O; and R^{5'} is hydrogen when p is a single bond;
X is CR^{6"} and R^{5'} is absent when p is a double bond;
R², R^{2'}, R^{4'}, and R^{4"} are each independently hydrogen, alkyl, alkenyl, alkynyl, alkoxy, alkylthio, alkylsulfinyl, alkylsulfonyl, alkylamino, arylalkyl, aryl, heterocyclic, heteroaromatic or a prodrug moiety;
R³ and R¹² are each hydrogen or a pro-drug moiety;
R⁴ is NR^{4'}R^{4"}, alkyl, alkenyl, alkynyl, hydroxyl, halogen, or hydrogen;
R⁵ is hydroxyl, hydrogen, thiol, alkanoyl, aroyl, alkaroyl, aryl, heteroaromatic, alkyl, alkenyl, alkynyl, alkoxy, alkylthio, alkylsulfinyl alkylsulfonyl, alkylamino, arylalkyl, alkyl carbonyloxy, or aryl carbonyloxy;
R⁶ and R^{6'} are each independently hydrogen, methylene, absent, hydroxyl, halogen, thiol, alkyl, alkenyl, alkynyl, aryl, alkoxy, alkylthio, alkylsulfinyl, alkylsulfonyl, alkylamino, or an arylalkyl;
R⁷ is hydrogen, hydroxyl, halogen, cyano, oximyl, alkoxycarbonyl, alkylcarbonyl, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃(NR^{7c})₀₋₁C(=W')WR^{7a};
R⁸ is hydrogen, hydroxyl, halogen, cyano, oximyl, alkoxycarbonyl, alkylcarbonyl, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃(NR^{8c})₀₋₁C(=E')ER^{8a};
R⁹ is hydrogen, hydroxyl, halogen, cyano, oximyl, alkoxycarbonyl, alkylcarbonyl, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃(NR^{9c})₀₋₁C(=Z')ZR^{9a};
R¹⁰ is hydrogen, hydroxyl, halogen, thiol, nitro, alkyl, alkenyl, alkynyl, aryl, alkoxy, alkylthio, alkylsulfinyl, alkylsulfonyl, arylalkyl, amino, arylalkeny), arylalkynyl, acyl, aminoalkyl, heterocyclic or thionitroso;
R¹¹ is hydrogen, hydroxyl or alkoxyl;
R^{7a}, R^{7b}, R^{7c}, R^{7d}, R^{7e}, R^{7f}, R^{7a}, R^{8b}, R^{8c}, R^{8d}, R^{8e}, R^{8c}, R^{9a}, R^{9b}, R^{9c}, R^{9d}, R^{9e}, and R^{9f} are each independently hydrogen, acyl, alkyl, alkenyl, alkynyl, alkoxy, alkylthio, alkylsulfinyl, alkylsulfonyl, alkylamino, arylalkyl, aryl, heterocyclic, heteroaromatic or a prodrug moiety;
R¹³ is hydrogen, hydroxy, alkyl, alkenyl, alkynyl, alkoxy, alkylthio, aryl, alkylsulfinyl, alkylsulfonyl, alkylamino, or an arylalkyl;
E is CR^{8d}R^{8e}, S, NR^{8b} or O;
E' is O, NR^{8f} ,or S;
W is CR^{7d}R^{7e}, S, NR^{7b} or O;
W' is O, NR^{7f}, or S;
Z is CR^{9d}R^{9e}, S, NR^{9b} or O;
Z' is O, S, or NR^{9f};
Y' and Y are each independently hydrogen, halogen, hydroxyl, cyano, sulfhydryl, amino, alkyl, alkenyl, alkynyl, alkoxy, alkylthio, alkylsulfinyl, alkylsulfonyl, alkylamino, or an arylalkyl; and pharmaceutically acceptable salts, esters and enantiomers thereof.

In an embodiment, the substituted tetracycline compounds used in the methods and compositions of the invention are substituted sancycline compounds, *e*.*g*., with substitution at the, for example, 2, 5, 6, 7, 8, 9, 10, 11, 11a, 12, 12a position and/or, in the case of methacycline, the 13 position. In substituted sancycline compounds of the invention, R⁴' and R^{4"} are each alkyl (*e.g.,* lower alkyl, *e.g.,* methyl); X is CR⁶R^{6'}; and R⁶, R^{6'} and R⁵ are each, generally, hydrogen. In an embodiment, the substituted tetracycline compound is a substituted tetracycline (*e*.*g*., generally, wherein R⁴ is NR^{4'}R^{4"}, R^{4'} and R^{4"} are methyl, R⁵ is hydrogen and X is CR⁶R^{6'}, wherein R⁶ is methyl and R^{6'} is hydroxy); substituted doxycycline (*e*.*g*., wherein R⁴ is NR^{4'}R^{4"}, R^{4'} and R^{4"} are methyl, R⁵ is hydroxyl and X is CR⁶R^{6'}, wherein R⁶ is methyl and R^{6'} is hydrogen); substituted minocycline (*e*.*g*., wherein R⁴ is NR^{4'}R^{4"}, R^{4'} and R^{4"} are methyl; R⁵ is hydrogen and X is CR⁶R^{6'} wherein R⁶ and R^{6'} are hydrogen atoms and R⁷ is dimethylamino) or substituted sancycline (wherein R⁴ is NR^{4'}R^{4"}, R^{4'} and R^{4"} are methyl; R⁵ is hydrogen and X is CR⁶R^{6'} wherein R⁶ and R^{6'} are hydrogen atoms). In one embodiment, the substituted tetracycline compound is non-antibiotic.

In one embodiment, R², R^{2'}, R³ and R¹² are each hydrogen; R⁴ is NR⁴R^{4'}; R⁴ and R^{4'} are each alkyl; and R¹¹ is hydroxyl. In a further embodiment, p is a single bond; X is CR⁶R^{6'}; R⁶ is alkyl; R^{6'} is hydrogen; R⁵ is hydroxyl; R⁷ and R⁸ are each hydrogen and R¹⁰ is hydroxyl. In yet another embodiment, R⁹ is aminoalkyl (*e*.*g*., aminomethyl), alkenyl (*e.g.,* aminocarbonylalkyl), alkoxycarbonyl (e.g., methoxycarbonyl) or a heterocyclic moiety (*e,g.,* morpholine).

In yet another embodiment, R², R^{2'}, R³ and R¹² are each hydrogen; R⁴ is NR⁴R^{4'}; R⁴ and R^{4'} are each alkyl; and R¹¹ is hydroxyl. In a further embodiment, p is a single bond; X is CR⁶R^{6'}; R⁶ is alkyl; R^{6'} is hydrogen; R⁵ is hydroxyl; R⁷ and R⁸ are each hydrogen; R⁹ is hydrogen and R¹⁰ is alkoxy (*e*.*g*., butoxy)

In another embodiment, R², R^{2'}, R³ and R¹² are each hydrogen; R⁴ is NR⁴R^{4'}; R⁴ and R^{4'} are each alkyl; and R¹¹ is hydroxyl; p is a single bond; X is CR⁶R^{6'}; R⁵, R⁶, R^{6'} and R⁸ are each hydrogen; R⁷ is alkylamino *(e.g.,* dialkylamino such as, for example, dimethylamino); R¹⁰ is hydroxyl and R⁹ is halogen (*e.g.,* fluorine), alkyl (*e.g.,* propyl), aminoalkyl (*e.g.,* alkylaminomehtyl or arylaminomethyl), alkoxycarbonyl (*e.g.,* methoxycarbonyl), alkylcarbonyl (*e.g.,* acyl), alkenyl (*e.g.,* aminoalkenyl such as alkylaminoalkenyl, or alkoxycarbonylalkenyl, such as ethoxycarbonuylalkenyl), alkynyl, aryl (*e.g.,* heteroaryl, such as, for example, substituted or unsubstituted furanyl (*e.g.,* alkyl substituted furanyl)) or heterocyclic moiety *(e.g.,* morpholine).

In a further embodiment, R², R^{2'}, R³ and R¹² are each hydrogen; R⁴ is NR⁴R^{4'}; R⁴ and R^{4'} are each alkyl; and R¹¹ is hydroxyl; p is a single bond; X is CR⁶R^{6'}; R⁵, R⁶, R^{6'} and R⁸ are each hydrogen; R⁷ is alkylamino (*e.g.,* dialkylamino such as, for example, dimethylamino); R⁹ is hydrogen and R¹⁰ is alkoxy (*e.g.,* butoxy)

In yet another embodiment, R², R^{2'}, R³ and R¹² are each hydrogen; R⁴ is NR⁴R^{4'}; R⁴ and R^{4'} are each alkyl; and R¹¹ is hydroxyl; p is a single bond; X is CR⁶R^{6'}; R⁵, R⁶, R^{6'} and R⁸ are each hydrogen; R¹⁰ is hydroxyl; R⁷ is halogen (*e.g.,* iodine) and R⁹ is thioalkyl, sulfonylalkyl or sulfinylalkyl.

In one embodiment, R², R^{2'}, R³ and R¹² are each hydrogen; R⁴ is NR⁴R^{4'}; R⁴ and R^{4'} are each alkyl; and R¹¹ is hydroxyl; p is a single bond; X is CR⁶R^{6'}; R⁵,R⁶, R^{6'} and R⁸ are each hydrogen; R¹⁰ is hydroxyl; R⁷ is aryl (*e.g.,* heteroaryl such as pyridinyl) and R⁹ is alkyl (*e.g.,* methyl).

In another embodiment, R², R^{2'}, R³ and R¹² are each hydrogen; R⁴ is NR⁴R^{4'}; R⁴ and R^{4'} are each alkyl; and R¹¹ is hydroxyl; p is a single bond; X is CR⁶R^{6'}; R⁵, R⁶, R^{6'} and R⁸ are each hydrogen; R¹⁰ is hydroxyl; R⁷ is alkenyl (*e.g.,* alkenyl substituted with one or more halogens, such as fluorine) and R⁹ is amino or nitro.

In a further embodiment, R², R^{2'}, R³ and R¹² are each hydrogen; R⁴ is NR⁴R^{4'}; R⁴ and R^{4'} are each alkyl; and R¹¹ is hydroxyl; p is a single bond; X is CR⁶R^{6'}; R⁵, R⁶, R^{6'} and R⁸ are each hydrogen; R¹⁰ is hydroxyl; R⁷ is alkyl ***(**e.g.,* ethyl) and R⁹ is aminoalkyl *(e.g.,* alkylaminoalkyl).

In yet another embodiment, R², R^{2'}, R³ and R¹² are each hydrogen; R⁴ is NR⁴R^{4'}; R⁴ and R^{4'} are each alkyl; and R¹¹ is hydroxyl; p is a single bond; X is CR⁶R^{6'}; R⁵, R⁶, R^{6'}, R⁸ and R⁹ are each hydrogen; R¹⁰ is hydroxyl and R⁷ is amino (*e.g.,* alkylamino or alkylcarbonylamino), aryl (*e.g.,* phenyl or heteroaryl such as, for example, pyridinyl, which may be substituted with one or more halogens (*e.g.,* chlorine or fluorine), furanyl, isoxazolyl (*e.g.,* alkyl substituted isoxazolyl such as methyl, isobutyl, isopropyl, isopropenyl or alkoxy substituted alkyl), pyrazolyl), alkyl, alkenyl (*e.g.,* alkenyl substituted with one or more halogens, such as fluorine, cyano, alkoxycarbonyl, such as methoxycarbonyl, or alkylcarbonyl, such as acyl), amino (*e.g.,* dialkylamino such as diethylamino), aminoalkyl (*e*.*g*., aminomethyl substituted with a heterocyclic moiety such as piperidine) cyano, a heterocyclic moiety (*e.g.,* morpholine) or oximyl.

In another embodiment, R², R^{2'}, R³ and R¹² are each hydrogen; R⁴ is NR⁴R^{4'}; R⁴ and R^{4'} are each alkyl; and R¹¹ is hydroxyl; p is a single bond; X is CR⁶R^{6'}; R⁵, R⁶, R^{6'}, R⁷ and R⁹ are each hydrogen; R¹⁰ is hydroxyl; and R⁸ is halogen (*e*:*g*., chlorine or bromine) or aryl (*e.g.,* phenyl or pyridinyl, such as halogen substituted pyridinyl).

In a further embodiment, R², R^{2'}, R³ and R¹² are each hydrogen; R⁴ is NR⁴R^{4'}; R⁴ and R^{4'} are each alkyl; and R¹¹ is hydroxyl; p is a single bond; X is CR⁶R^{6'}; R⁵, R⁶, R^{6'} and R⁷ are each hydrogen; and R⁸ is halogen (*e.g.,* chlorine or bromine) or aryl (*e.g.,* phenyl or pyridinyl, such as halogen substituted pyridinyl); R¹⁰ is hydroxyl; and R⁹ is hydrogen or amino (*e*.*g*., amino substituted with alkylcarbonyl, such as acyl).

In one embodiment, R², R^{2'}, R³ and R¹² are each hydrogen; R⁴ is NR⁴R^{4'}; R⁴ and R^{4'} are each alkyl; and R¹¹ is hydroxyl; p is a single bond; X is CR⁶R^{6'}; R⁵, R⁶, R^{6'} and R⁷ are each hydrogen; and R⁸ is halogen (*e.g.,* chlorine or bromine) or aryl (*e.g.,* phenyl or pyridinyl, such as halogen substituted pyridinyl); R¹⁰ is hydroxyl; and R⁹ is hydrogen or amino (*e.g.,* amino substituted with alkylcarbonyl, such as acyl).

In another embodiment, R², R^{2'}, R³ and R¹² are each hydrogen; R⁴ is NR⁴R^{4'}; R⁴ and R^{4'} are each alkyl; and R¹¹ is hydroxyl; p is a single bond; X is CR⁶R^{6'}; R⁵, R⁶, R^{6'} and R⁹ are each hydrogen; R¹⁰ is hydroxyl; R⁷ is amino; and R⁸ is halogen (*e*.*g*., halogen).

In one embodiment, R², R^{2'}, R³ and R¹² are each hydrogen; R⁴ is NR⁴R^{4'}; R⁴ and R^{4'} are each alkyl; and R¹¹ is hydroxyl; p is a single bond; X is CR⁶R^{6'}; R⁵,R⁶, R^{6'}, R⁷ and R⁸ are each hydrogen; R¹⁰ is hydroxyl; and R⁹ is a heterocyclic moiety (*e.g.,* piperidine or morpholine) or aminoalkyl (*e.g.,* aminomethyl such as aminomethyl substituted with trifluoroalkyl).

In another embodiment, R², R^{2'}, R³ and R¹² are each hydrogen; R⁴ is NR⁴R^{4'}; R⁴ and R^{4'} are each alkyl; and R¹¹ is hydroxyl; p is a single bond; X is CR⁶R^{6'}; R⁵, R⁶, R^{6'}, R⁷, R⁸ and R⁹ are each hydrogen; and R¹⁰ is alkoxy (*e.g.,* propoxy or butoxy which may be substituted with hydroxyl).

In yet another embodiment, R², R^{2'}, R³ and R¹² are each hydrogen; R⁴ is NR⁴R^{4'}; R⁴ and R^{4'} are each alkyl; and R¹¹ is hydroxyl; p is a double bond; X is CR^{6"}; and R^{6"}, R⁸ and R⁹ are each hydrogen; R¹⁰ is hydroxyl and R⁷ is halogen (*e.g.,* chlorine).

In one embodiment, the substituted tetracycline compound for use in the methods of the invention is of formula (II): 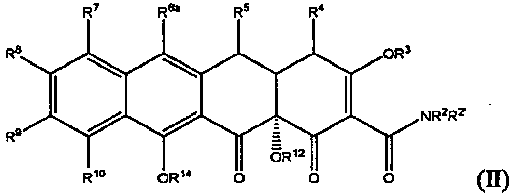 wherein
R², R^{2'}, R^{4'}, and R^{4"} are each independently hydrogen, alkyl, alkenyl, alkynyl, alkoxy, alkylthio, alkylsulfinyl, alkylsulfonyl, alkylamino, arylalkyl, aryl, heterocyclic, heteroaromatic or a prodrug moiety;
R³, R¹² and R¹⁴ are each hydrogen or a pro-drug moiety;
R⁴ is NR^{4'}R^{4"}, alkyl, alkenyl, alkynyl, hydroxyl, halogen, or hydrogen;
R⁵ is hydroxyl, hydrogen, thiol, alkanoyl, aroyl, alkaroyl, aryl, heteroaromatic, alkyl, alkenyl, alkynyl, alkoxy, alkylthio, alkylsulfinyl, alkylsulfonyl, alkylamino, arylalkyl, alkyl carbonyloxy, or aryl carbonyloxy;
R^{6a} is hydrogen, hydroxyl, halogen, thiol, alkyl, alkenyl, alkynyl, aryl, alkoxy, alkylthio, alkylsulfinyl, alkylsulfonyl, alkylamino, or an arylalkyl;
R⁷ is hydrogen, hydroxyl, halogen, cyano, oximyl, alkoxycarbonyl, alkylcarbonyl, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃(NR^{7c})₀₋₁C(=W')WR^{7a};
R⁸ is hydrogen, hydroxyl, halogen, cyano, oximyl, alkoxycarbonyl, alkylcarbonyl, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃(NR^{8c})₀₋₁C(=E')ER^{8a};
R⁹ is hydrogen, hydroxyl, halogen, cyano, oximyl, alkoxycarbonyl, alkylcarbonyl, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃(NR^{9c})₀₋₁C(=Z')ZR^{9a};
R¹⁰ is hydrogen, hydroxyl, halogen, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic or thionitroso;
R^{7a}, R^{7b}, R^{7c}, R^{7d}, R^{7e}, R^{7f}, R^{8a}, R^{8b}, R^{8c}, R^{8d}, R^{8e}, R^{8f}, R^{9a}, R^{9b}, R^{9c}, R^{9d}, R^{9e}, and R^{9f} are each independently hydrogen, acyl, alkyl, alkenyl, alkynyl, alkoxy, alkylthio, alkylsulfinyl, alkylsulfonyl, alkylamino, arylalkyl, aryl, heterocyclic, heteroaromatic or a prodrug moiety;
W is CR^{7d}R^{7e}, S, NR^{7b} or O;
W' is O, NR^{7f}, or S;
E is CR^{8d}R^{8e}, S, NR^{8b} or O;
E' is O, NR^{8f}, or S;
Z is CR^{9d}R^{9e}, S, NR^{9b} or O;
Z' is O, S, or NR^{9f}; and pharmaceutically acceptable salts, esters and enantiomers thereof.

In another embodiment, R², R^{2'}, R³, R⁵, R⁸, R⁹, R¹¹, R¹², and R¹⁴ are each hydrogen; R⁴ is NR⁴R^{4'}; R⁴, R^{4'} are R^{6a} are each alkyl; R¹⁰ is hydroxyl; and R⁷ is hydrogen or halogen (e.g., chlorine).

In yet another embodiment, the substituted tetracycline compound is a compound of Table 2.

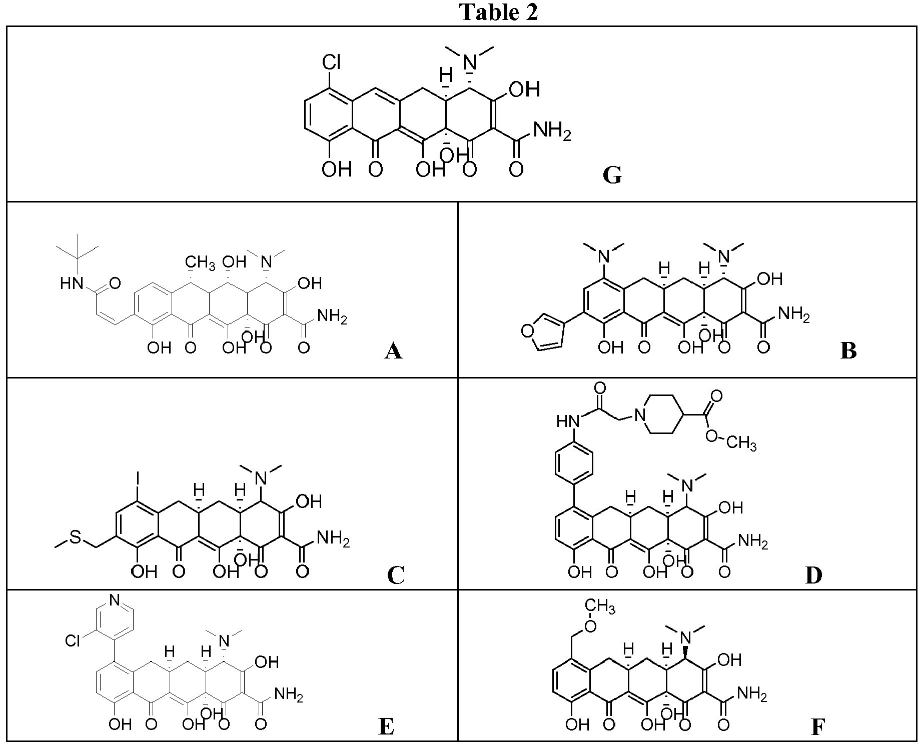

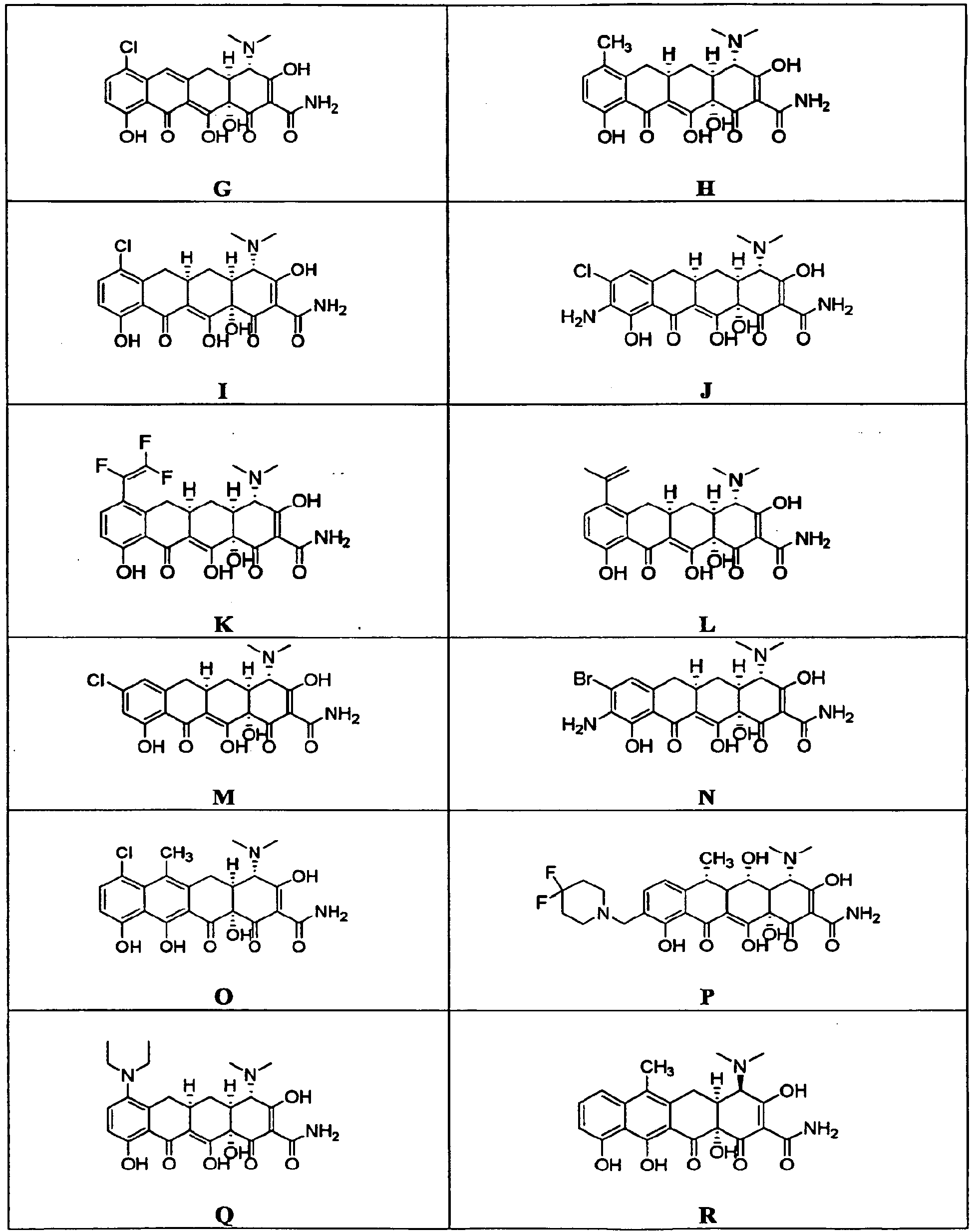

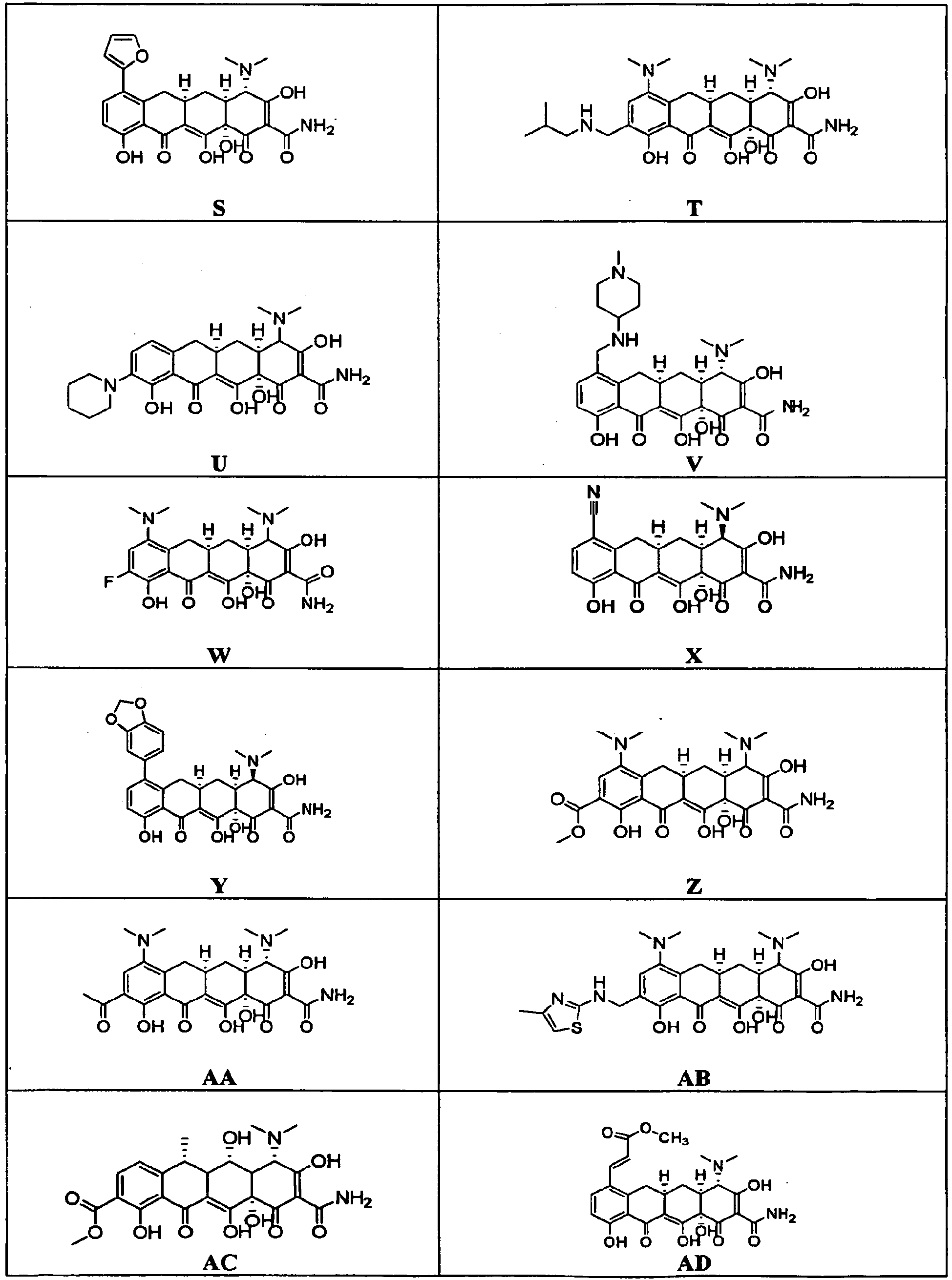

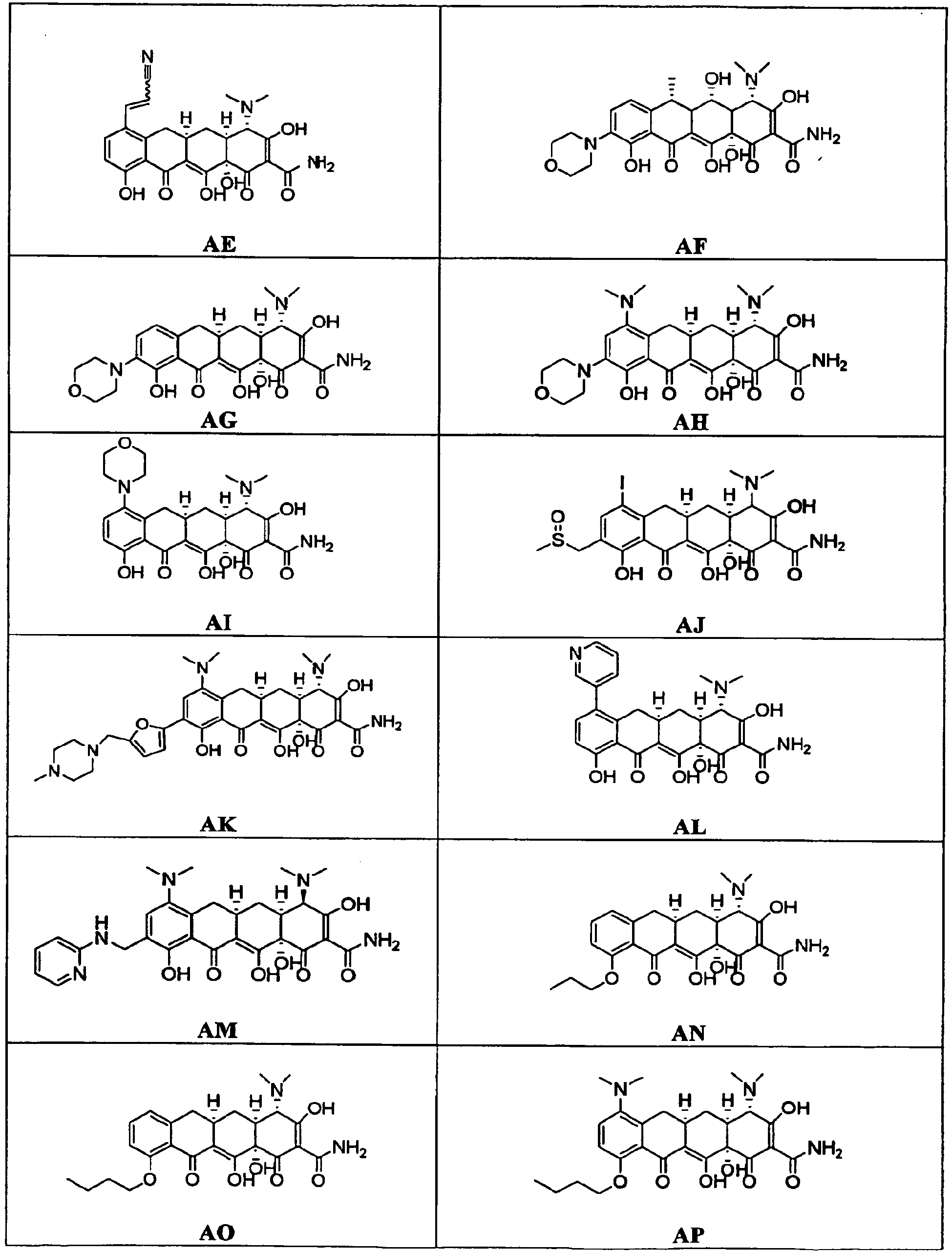

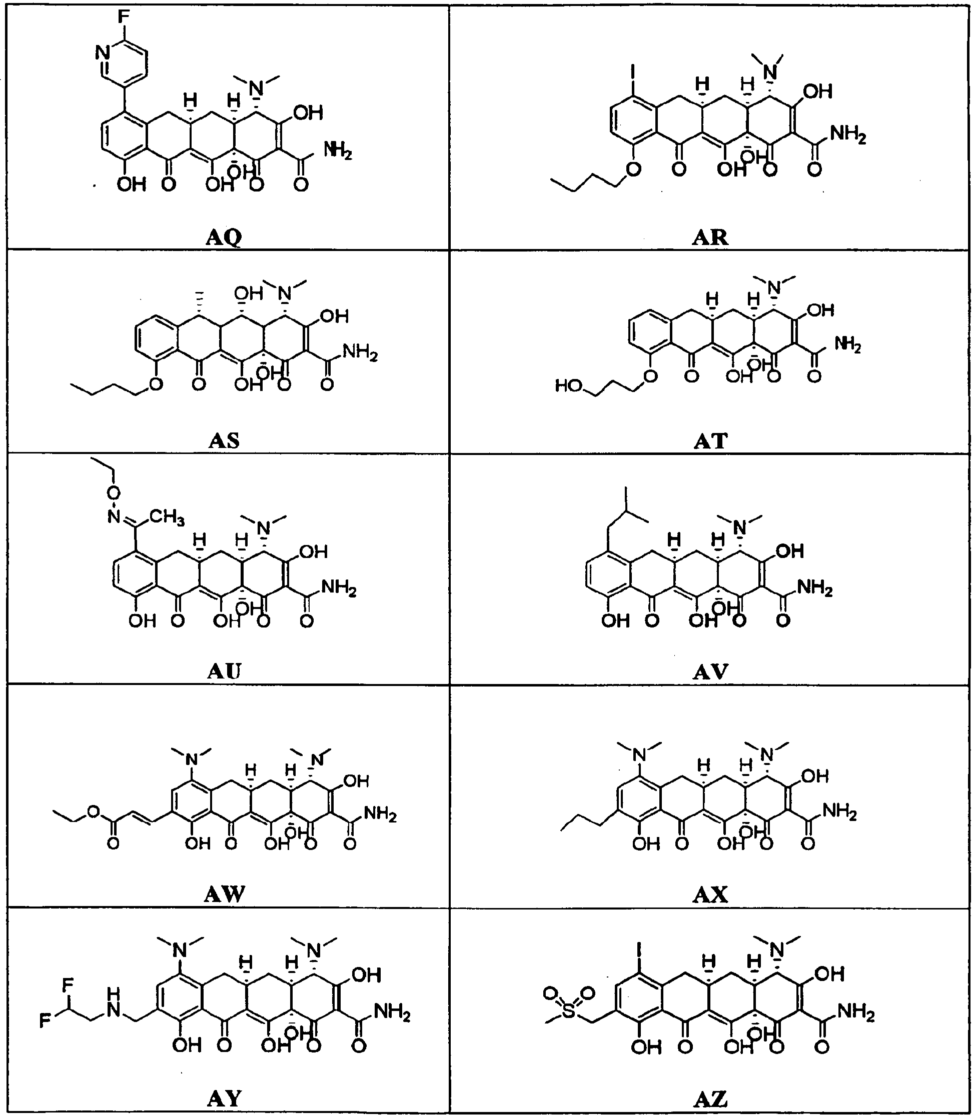

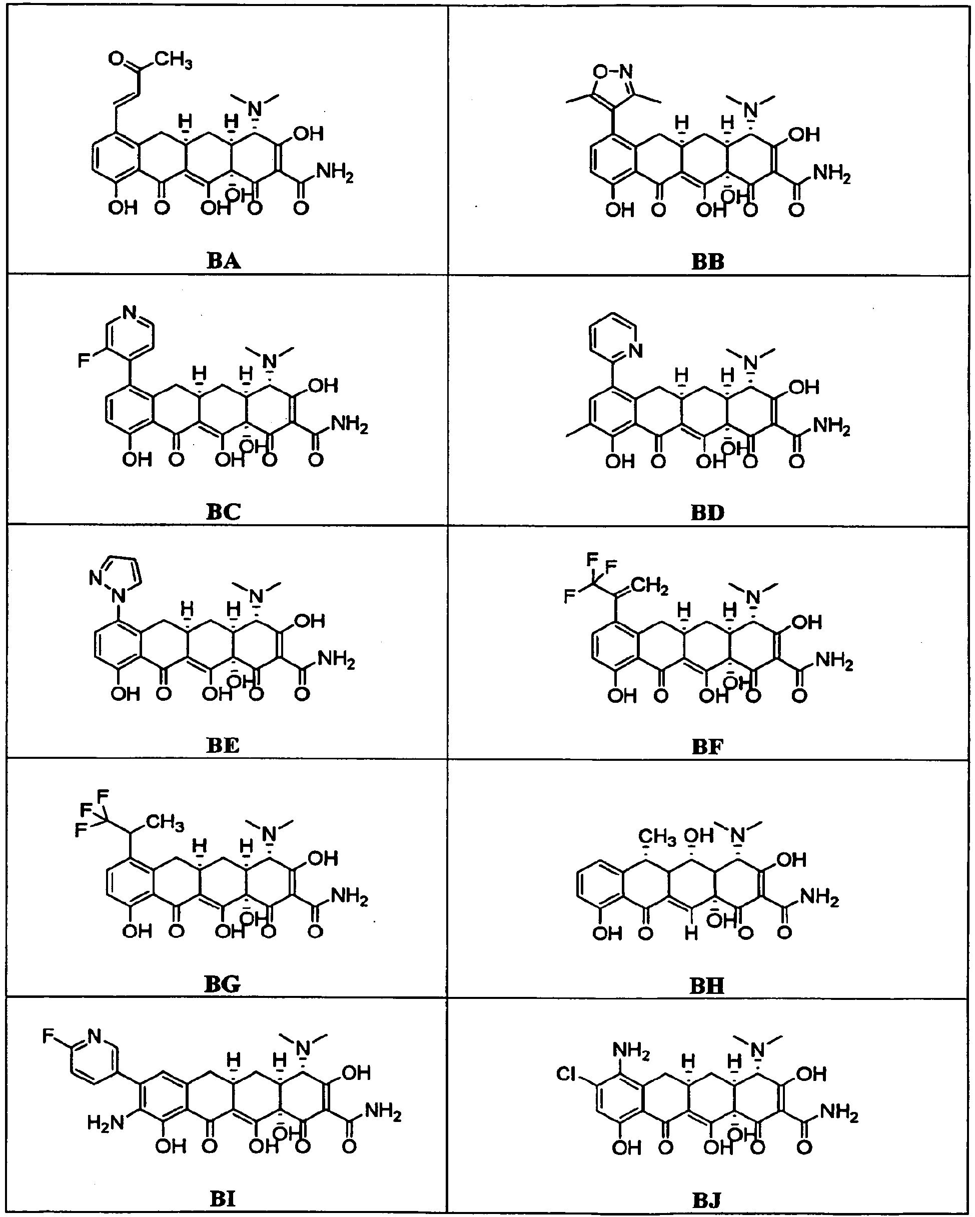

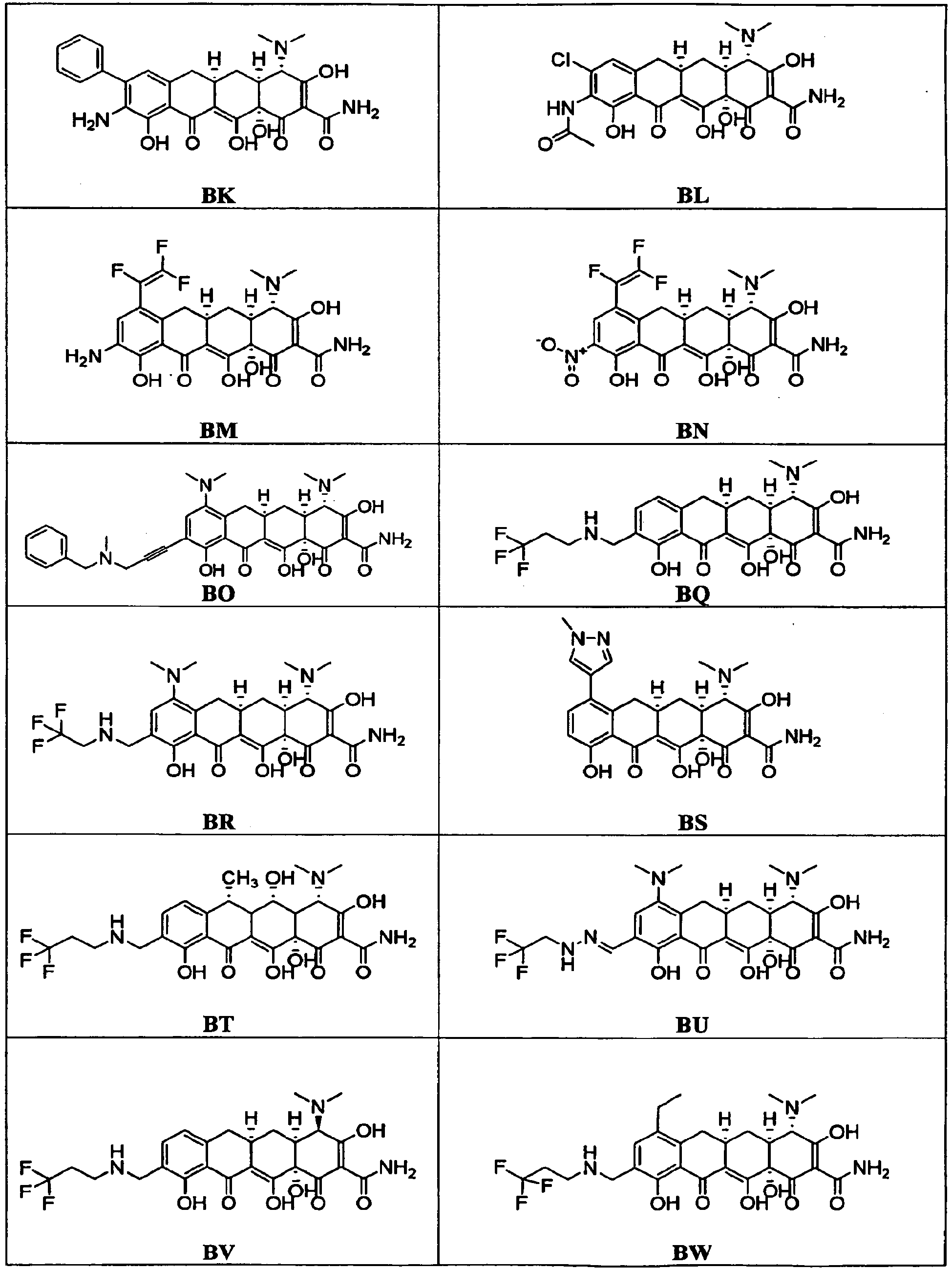

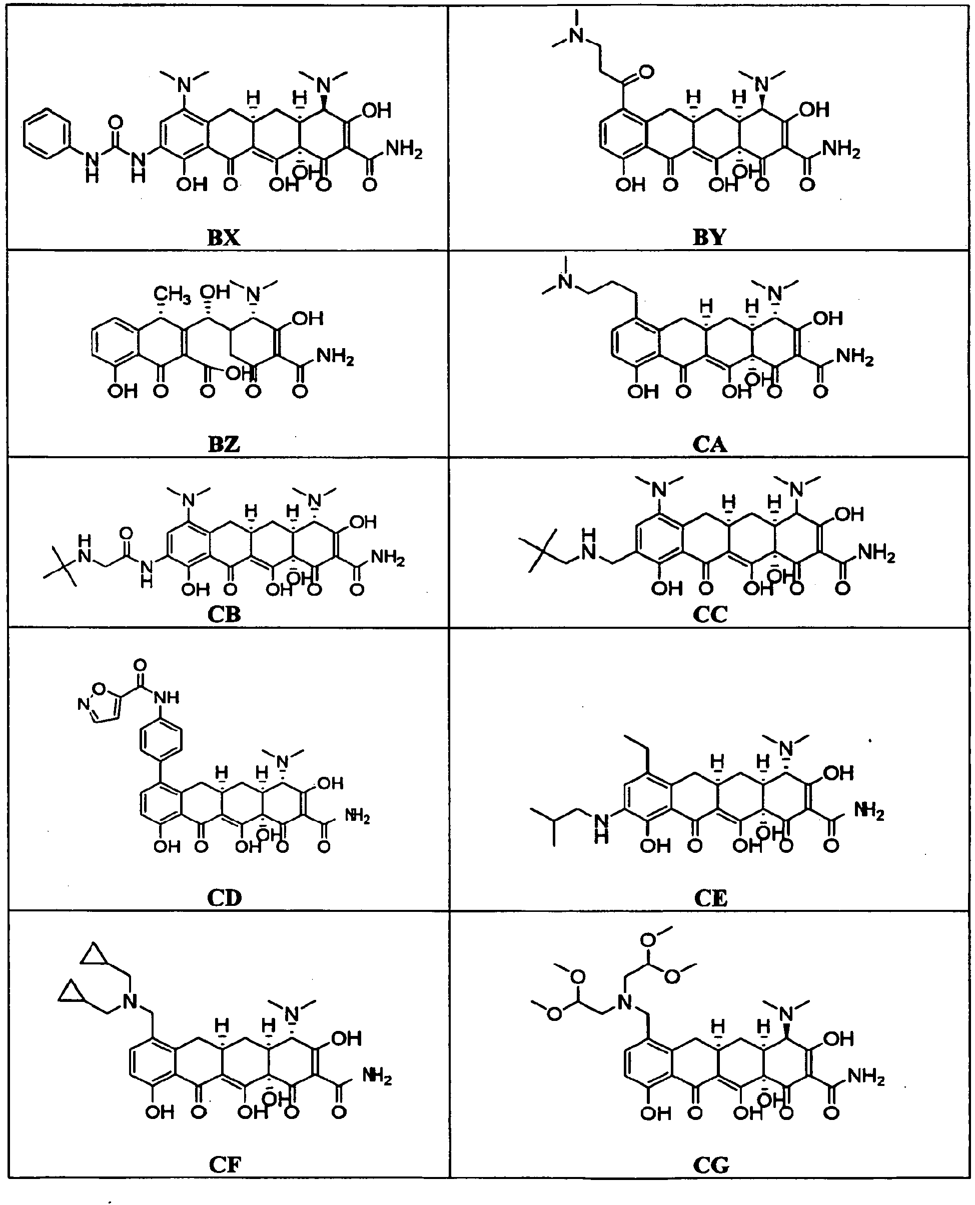

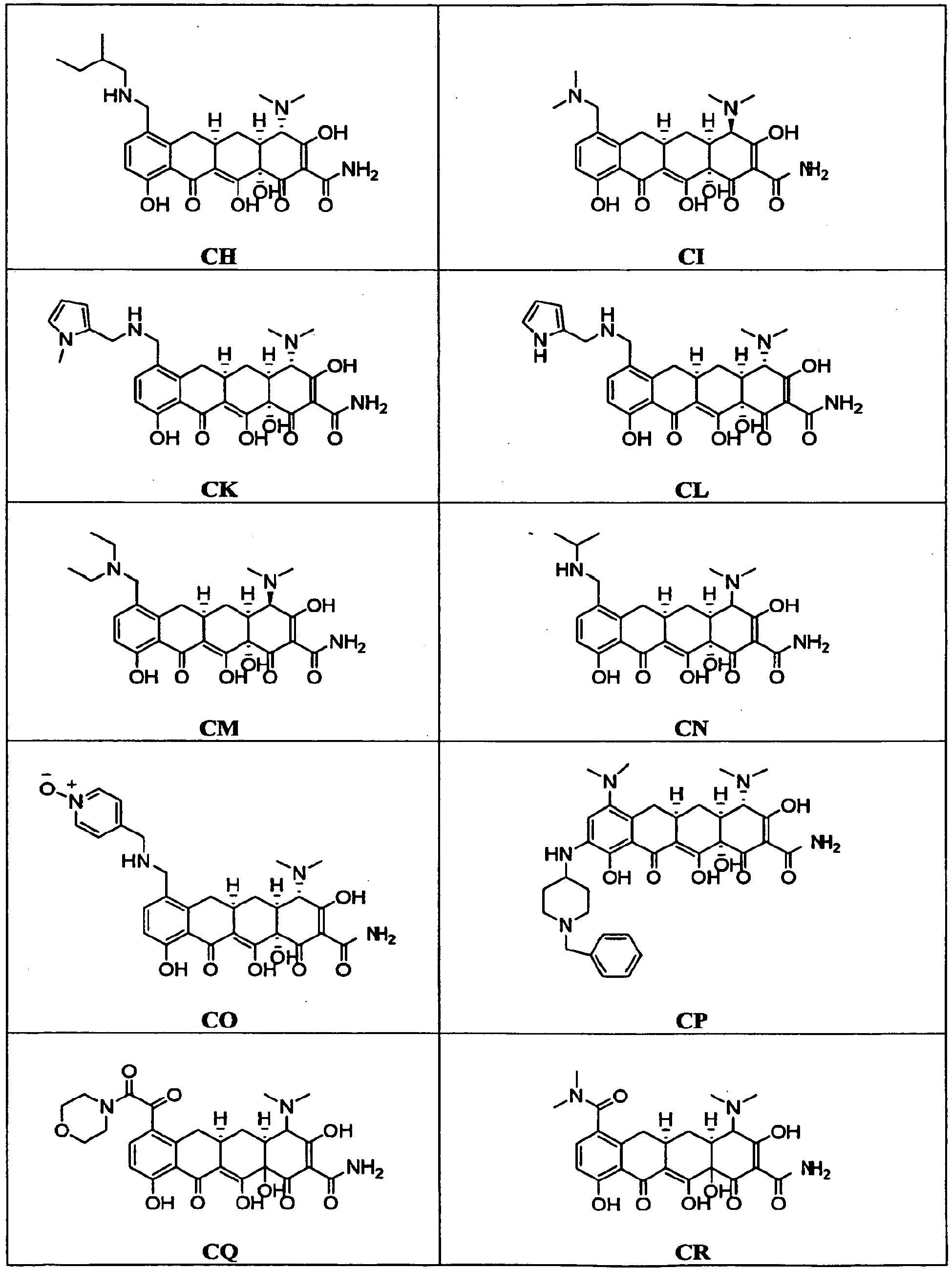

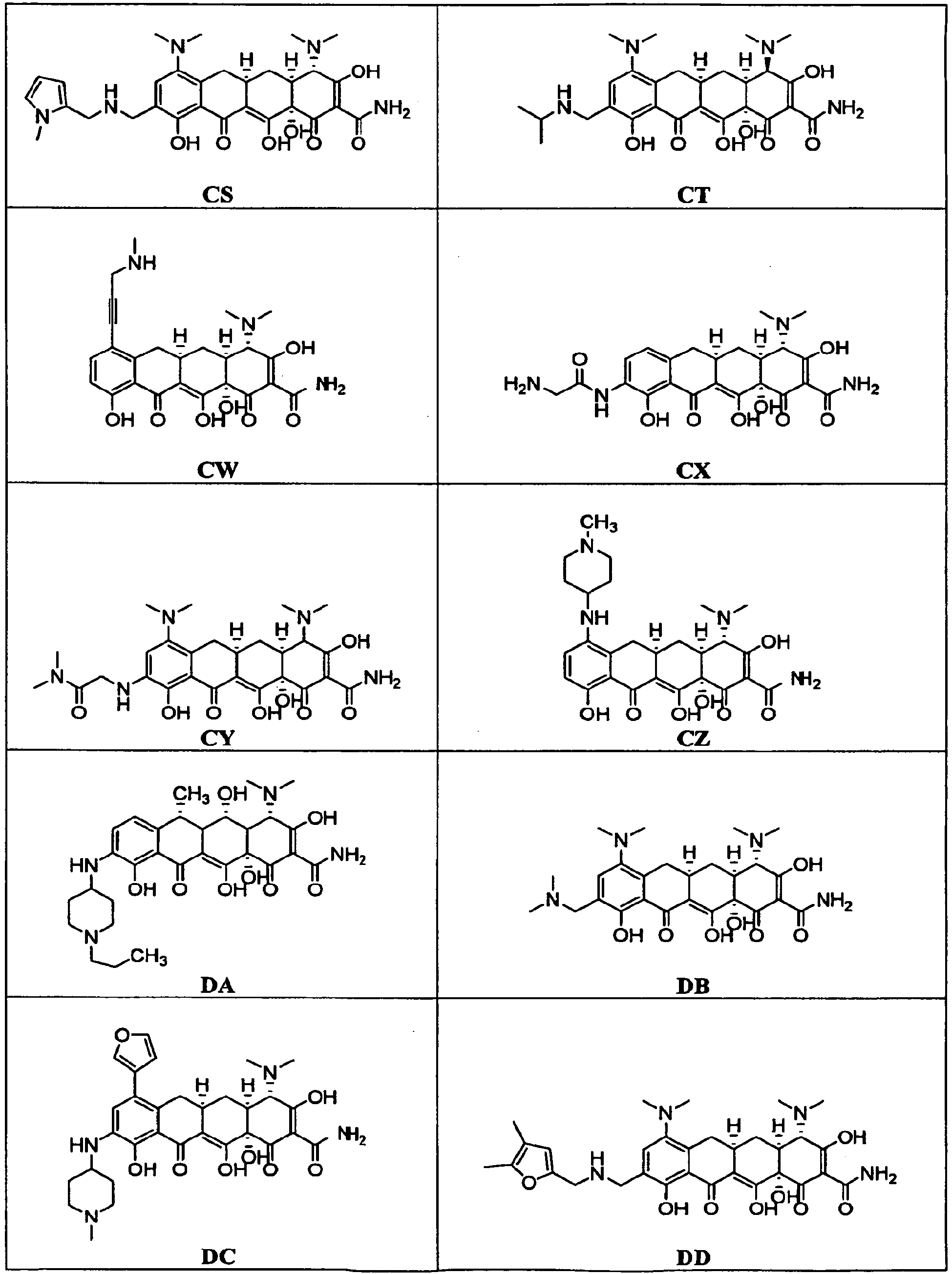

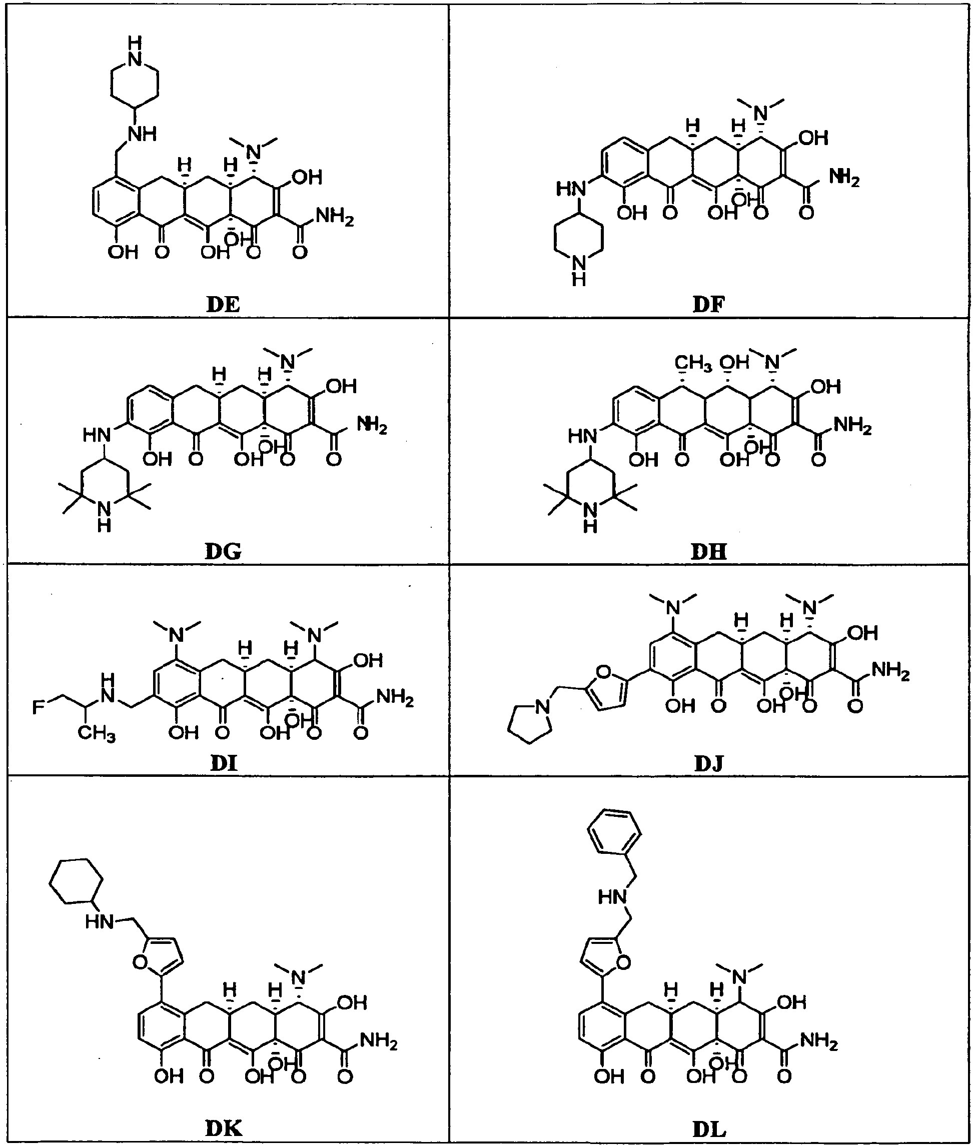

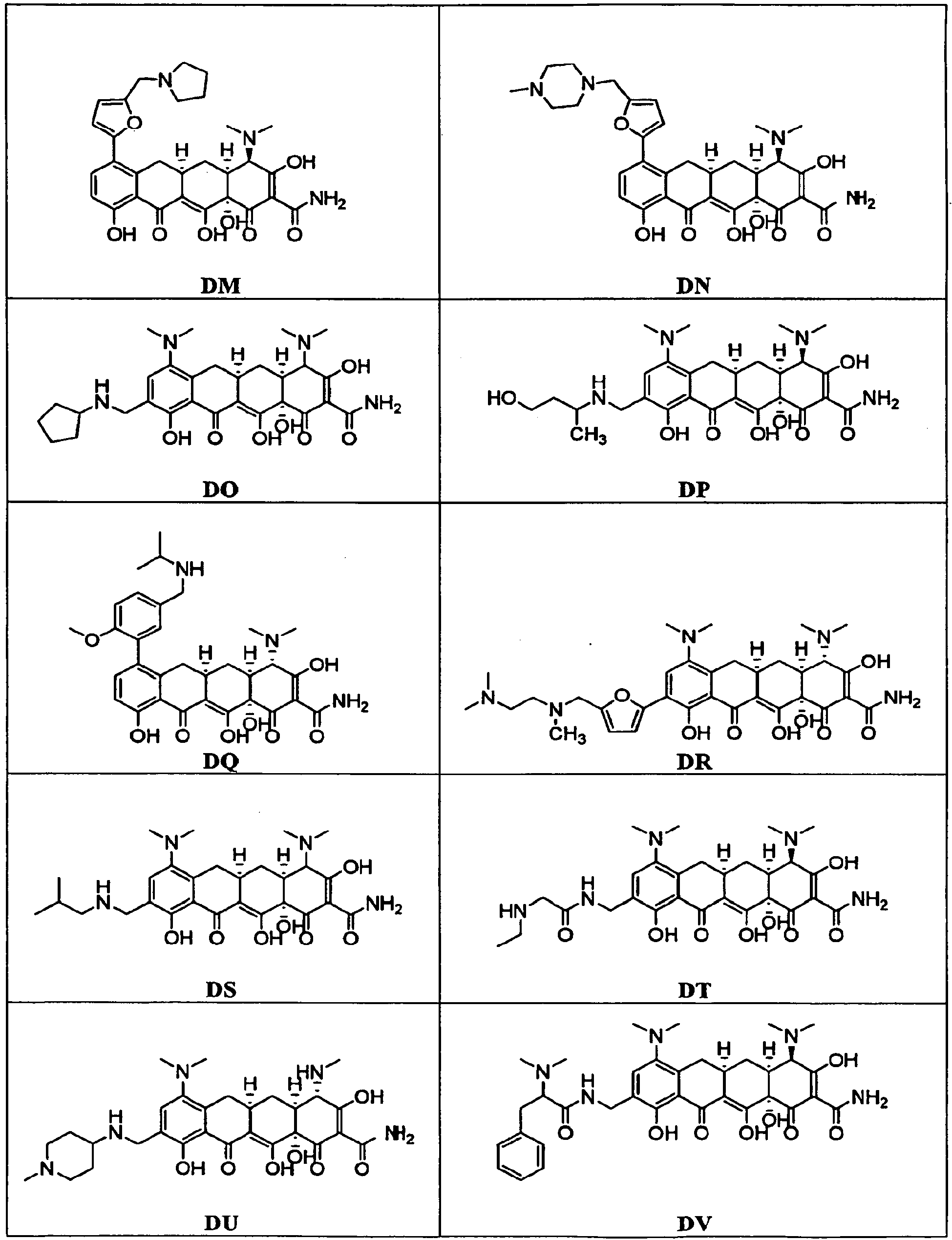

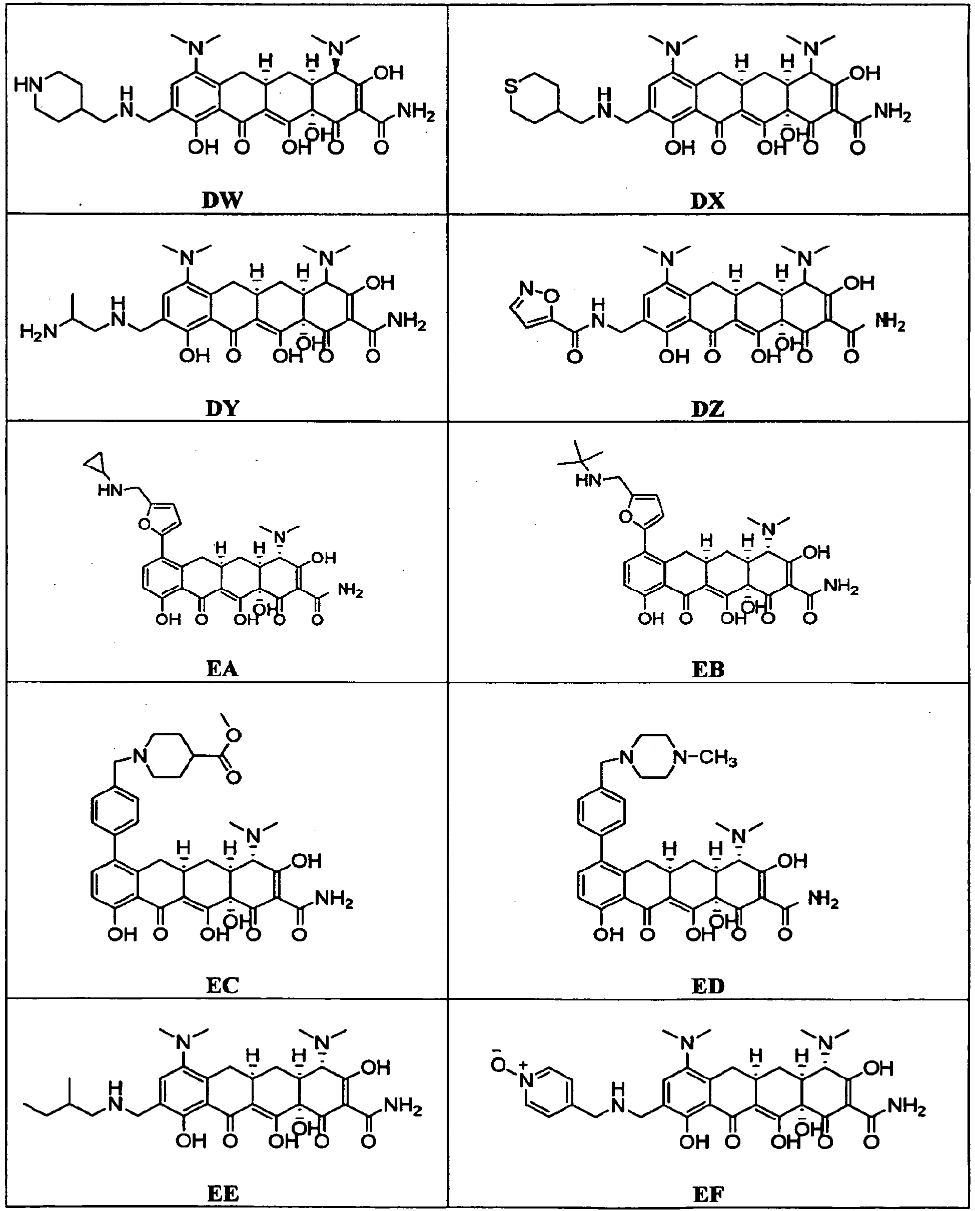

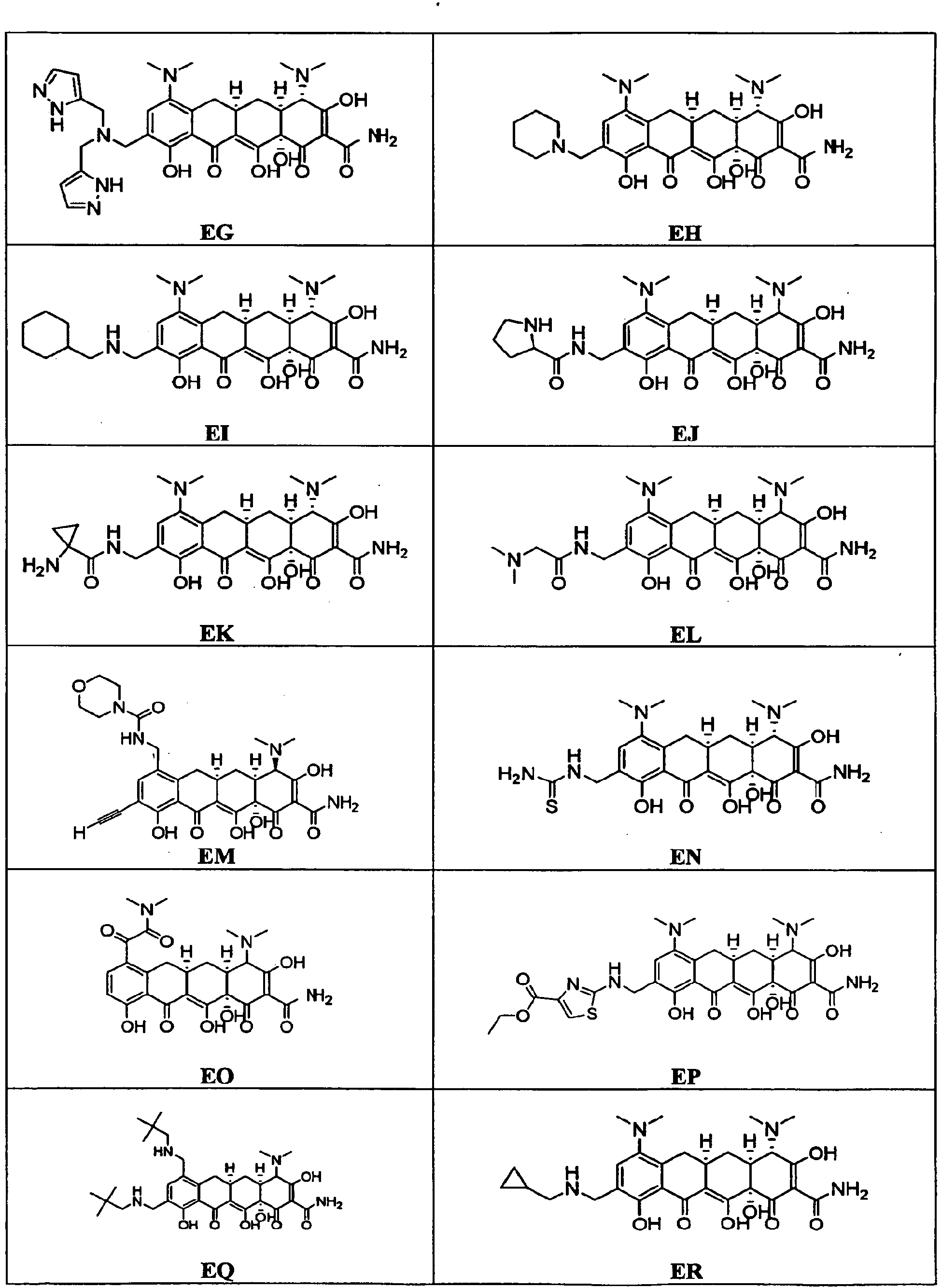

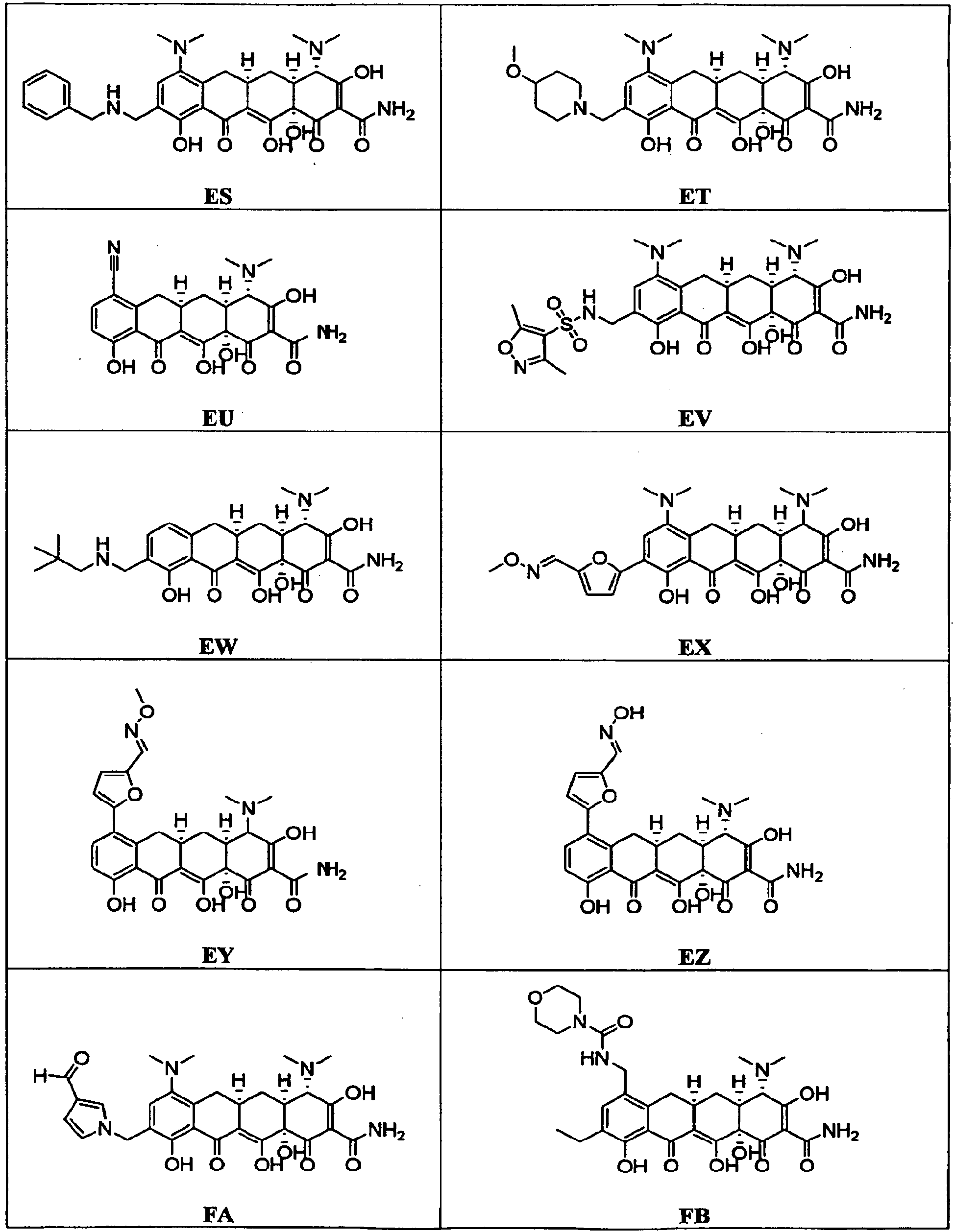

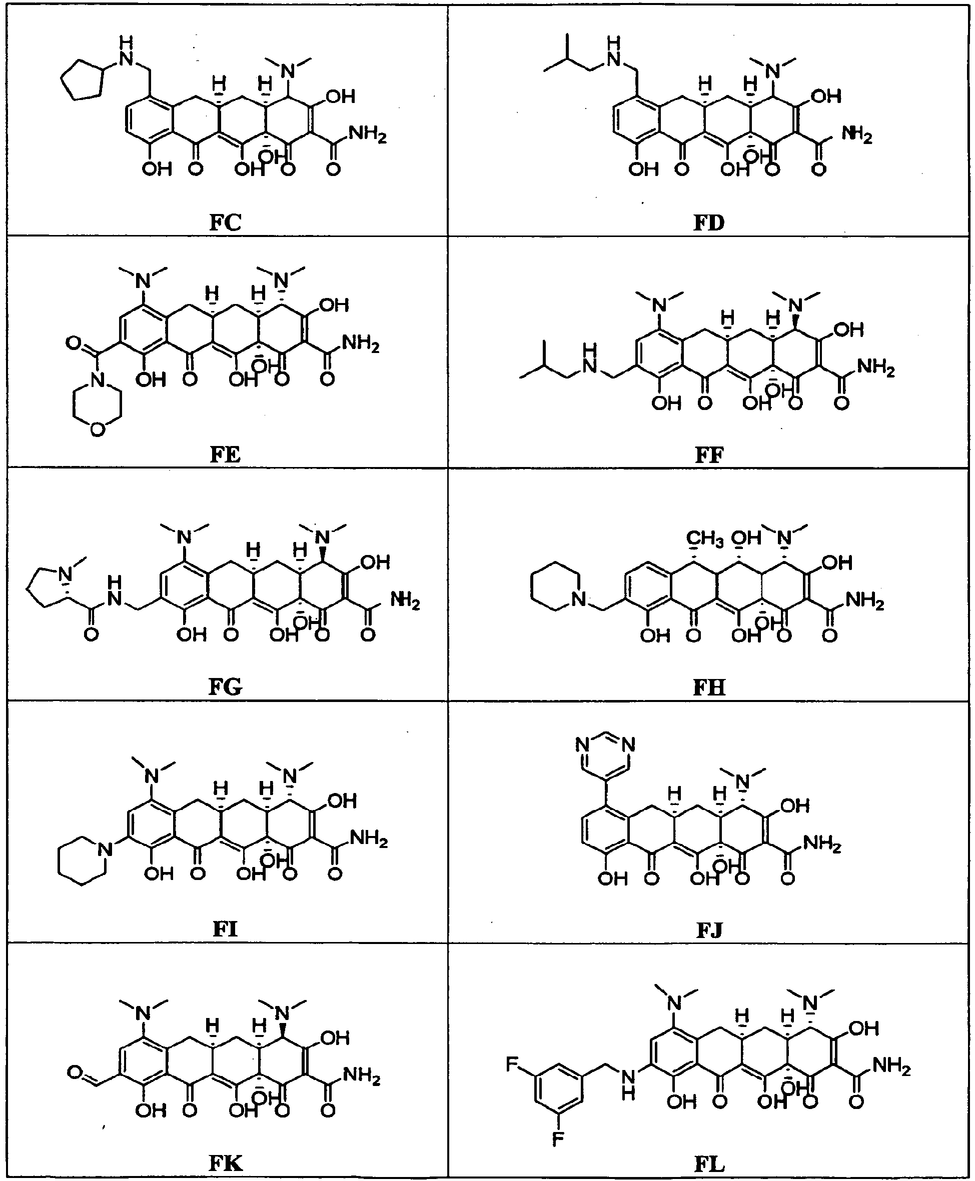

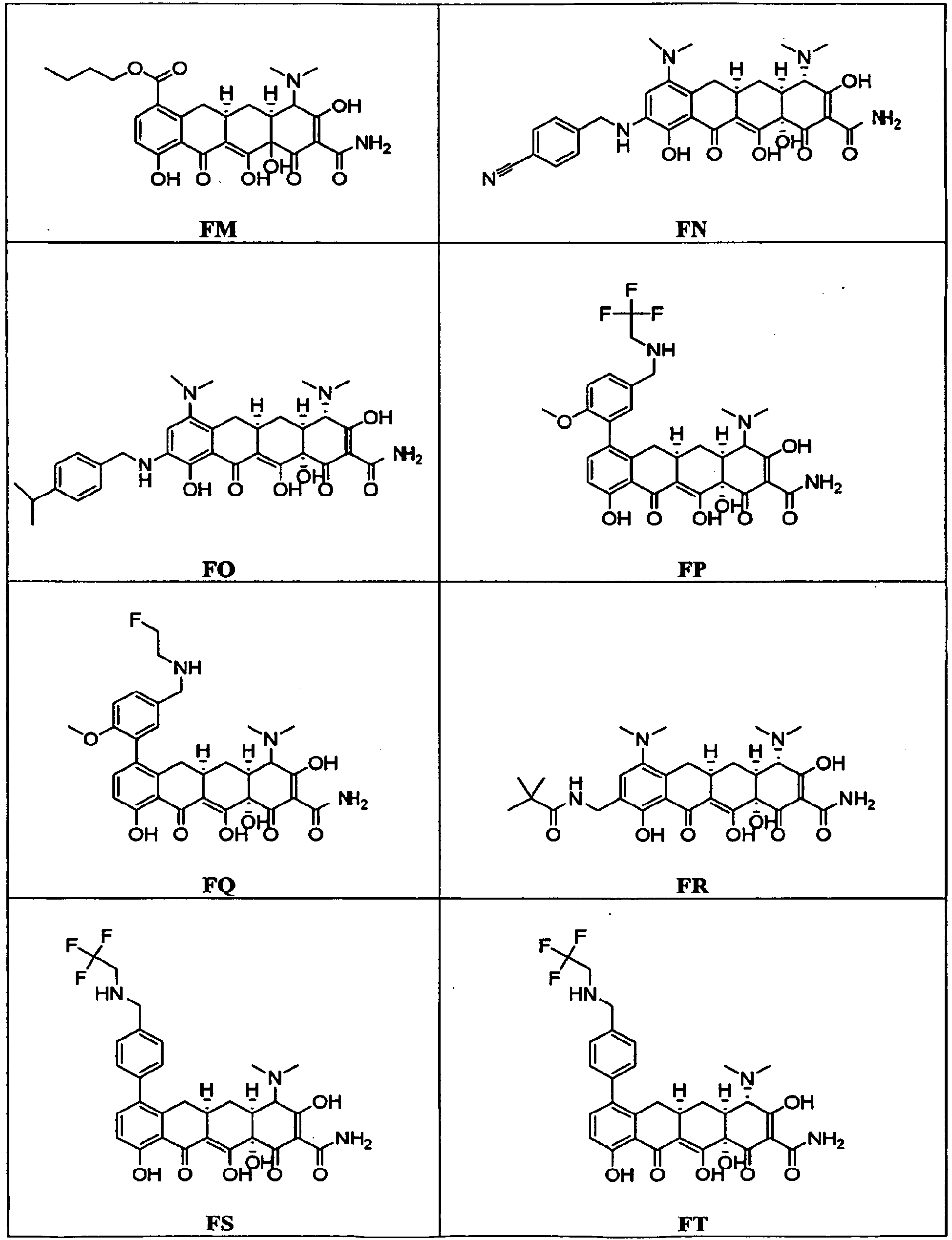

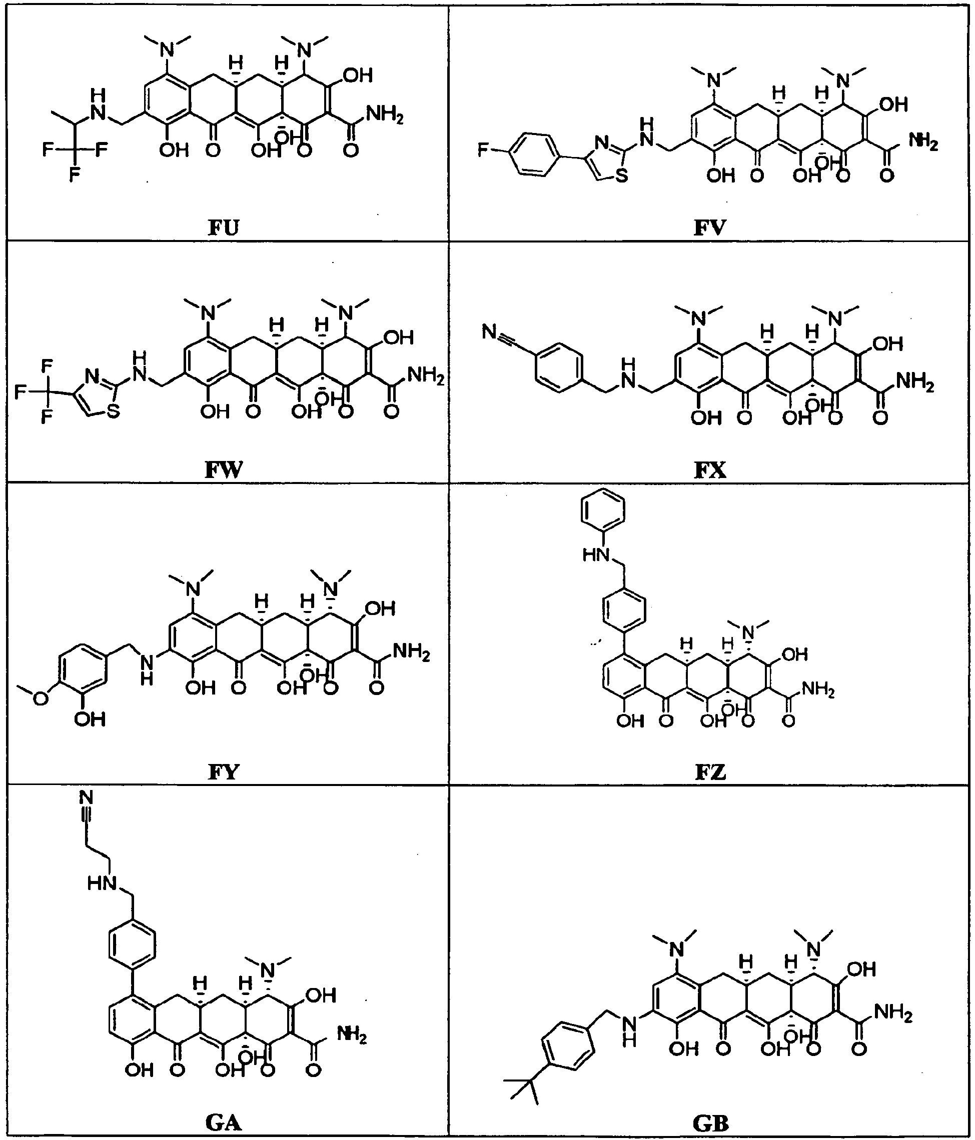

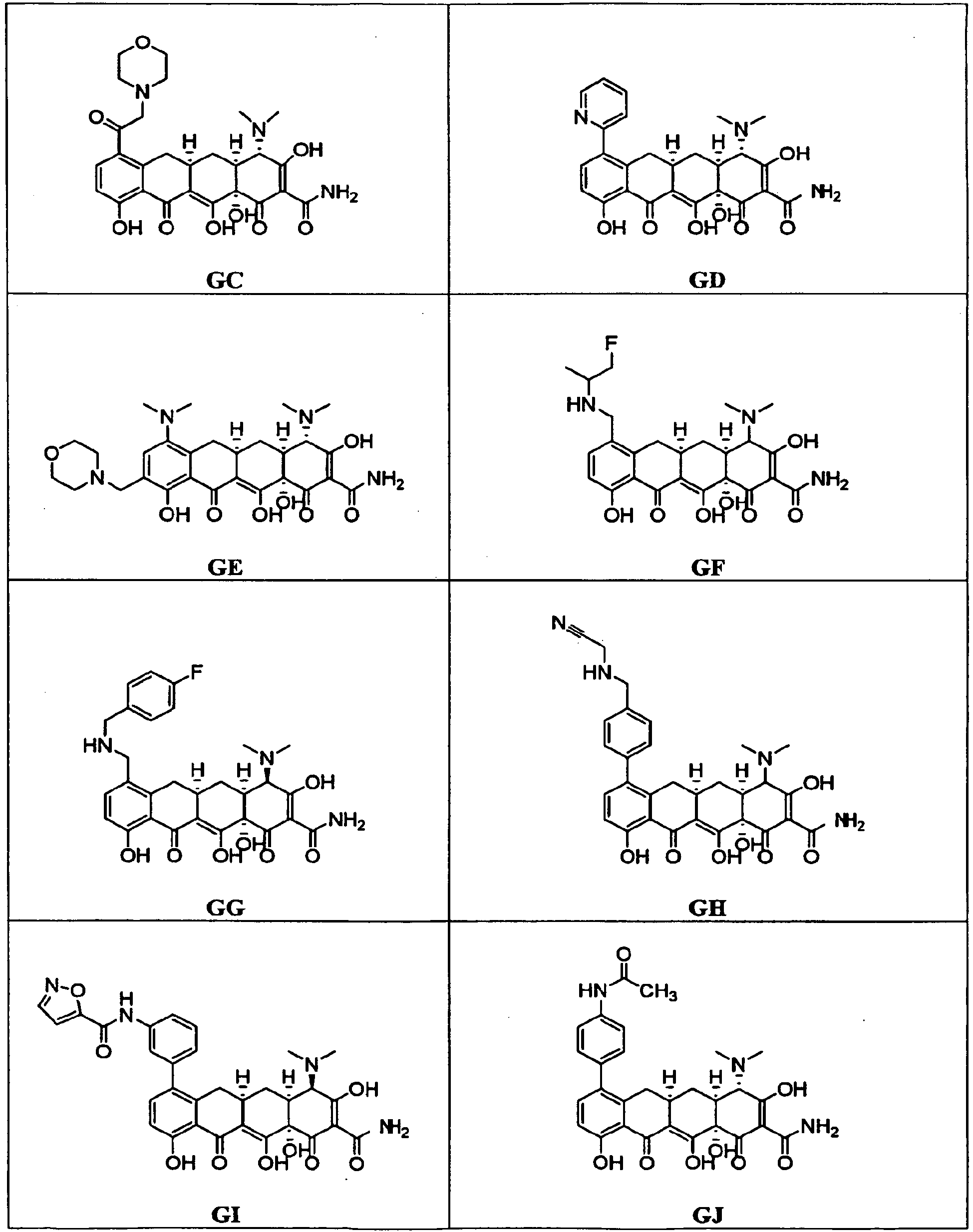

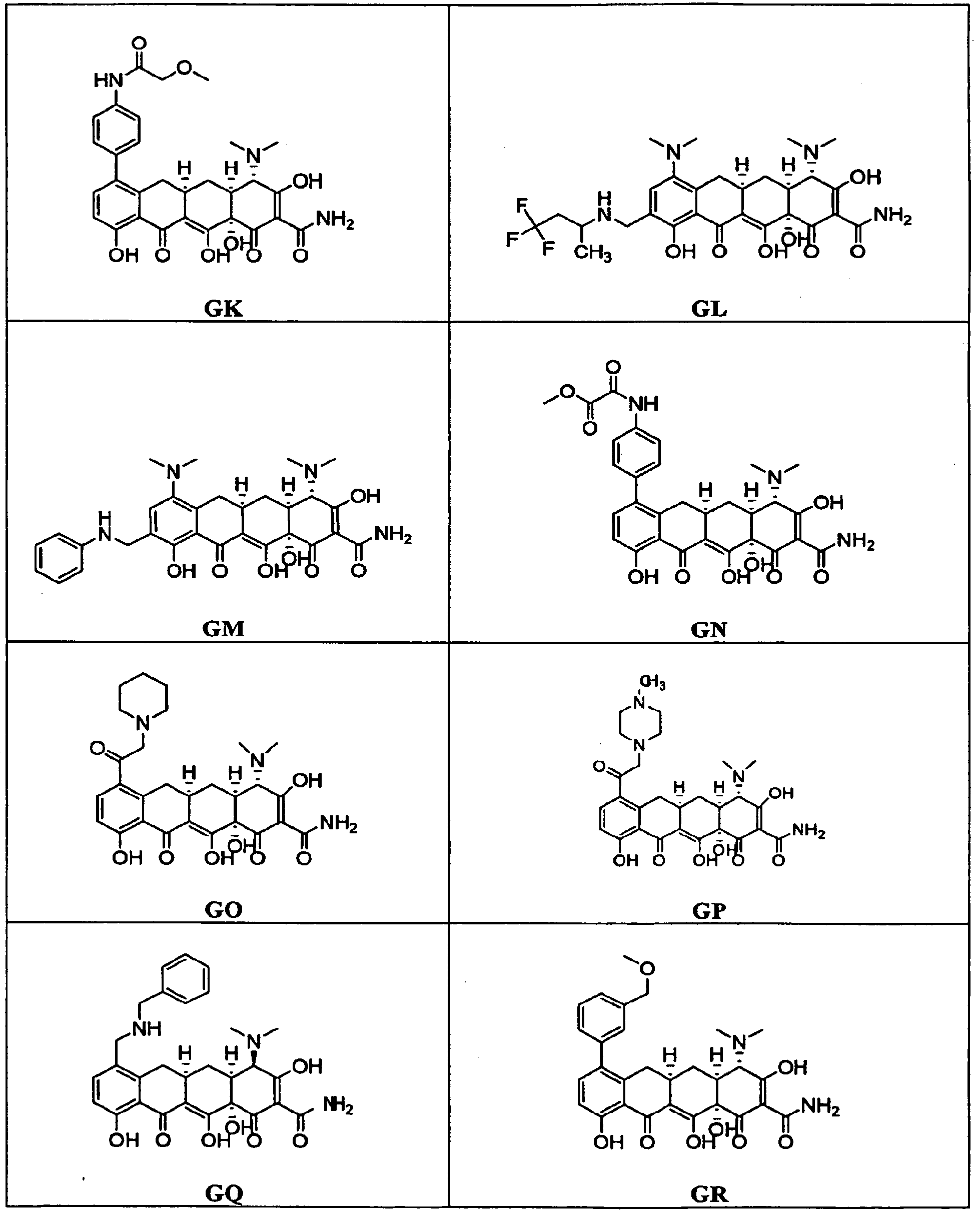

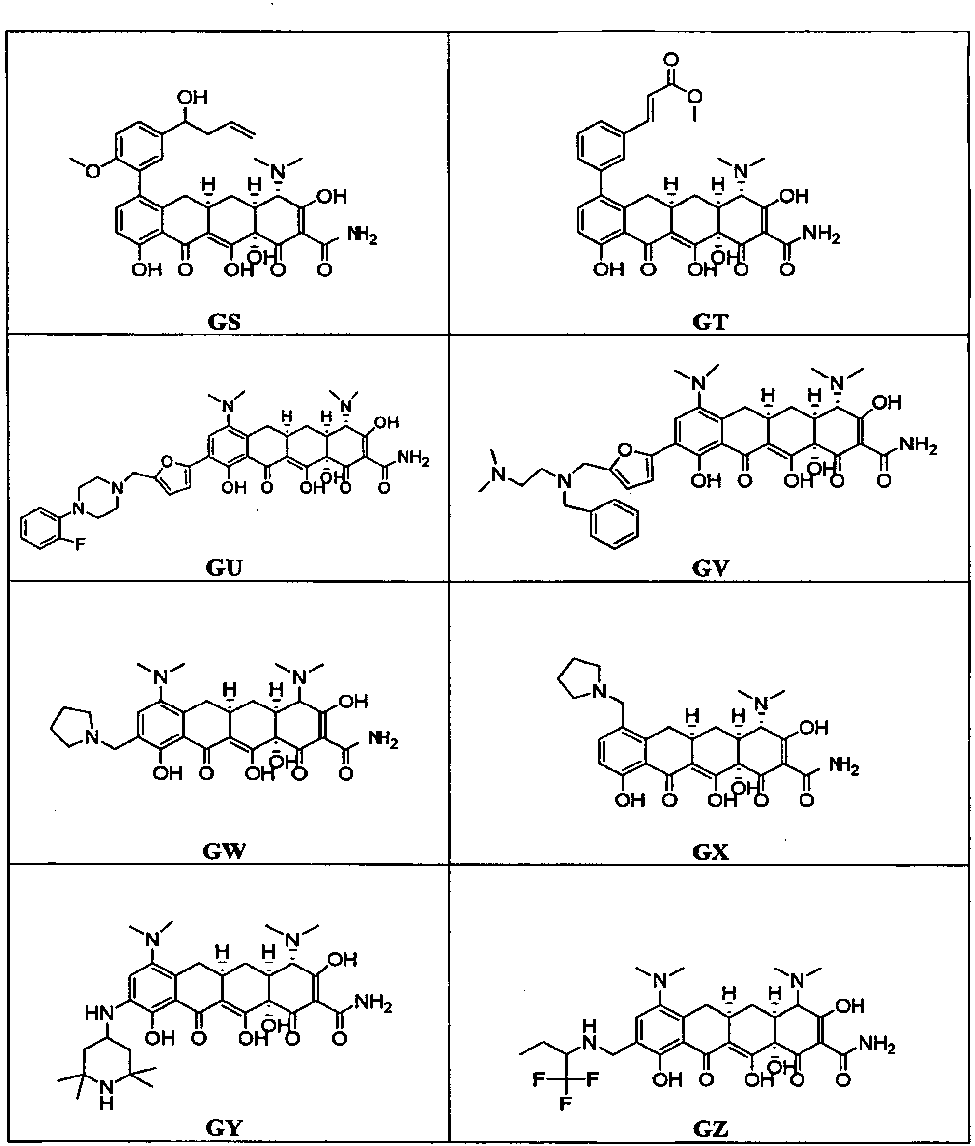

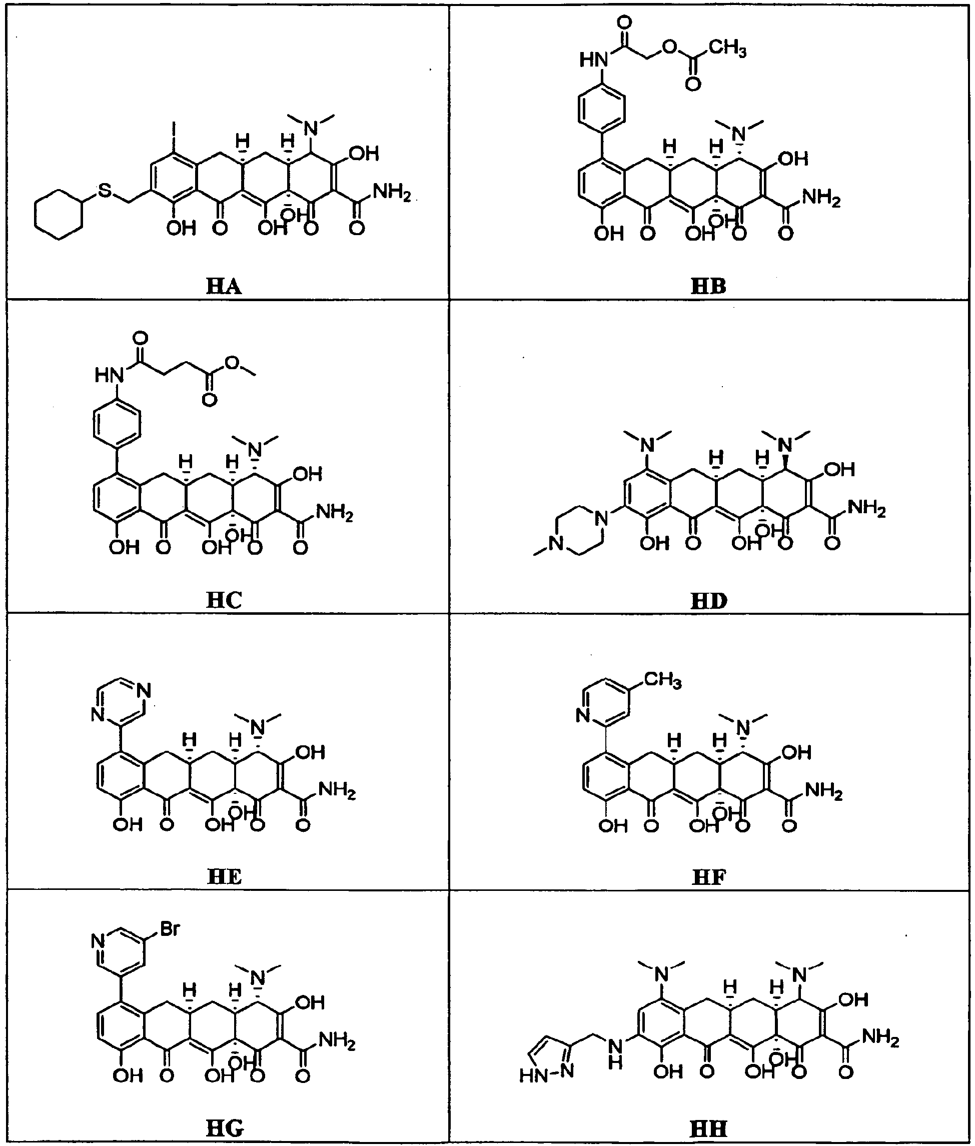

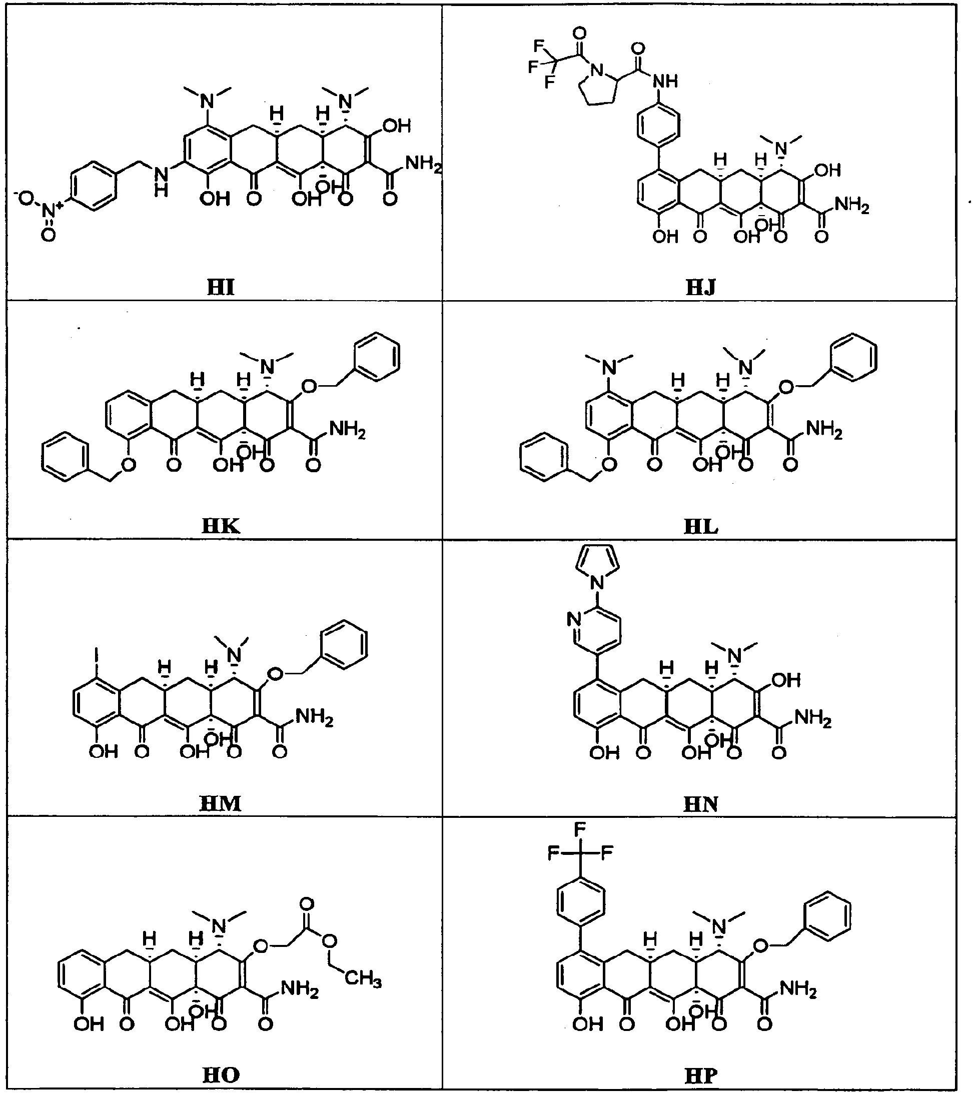

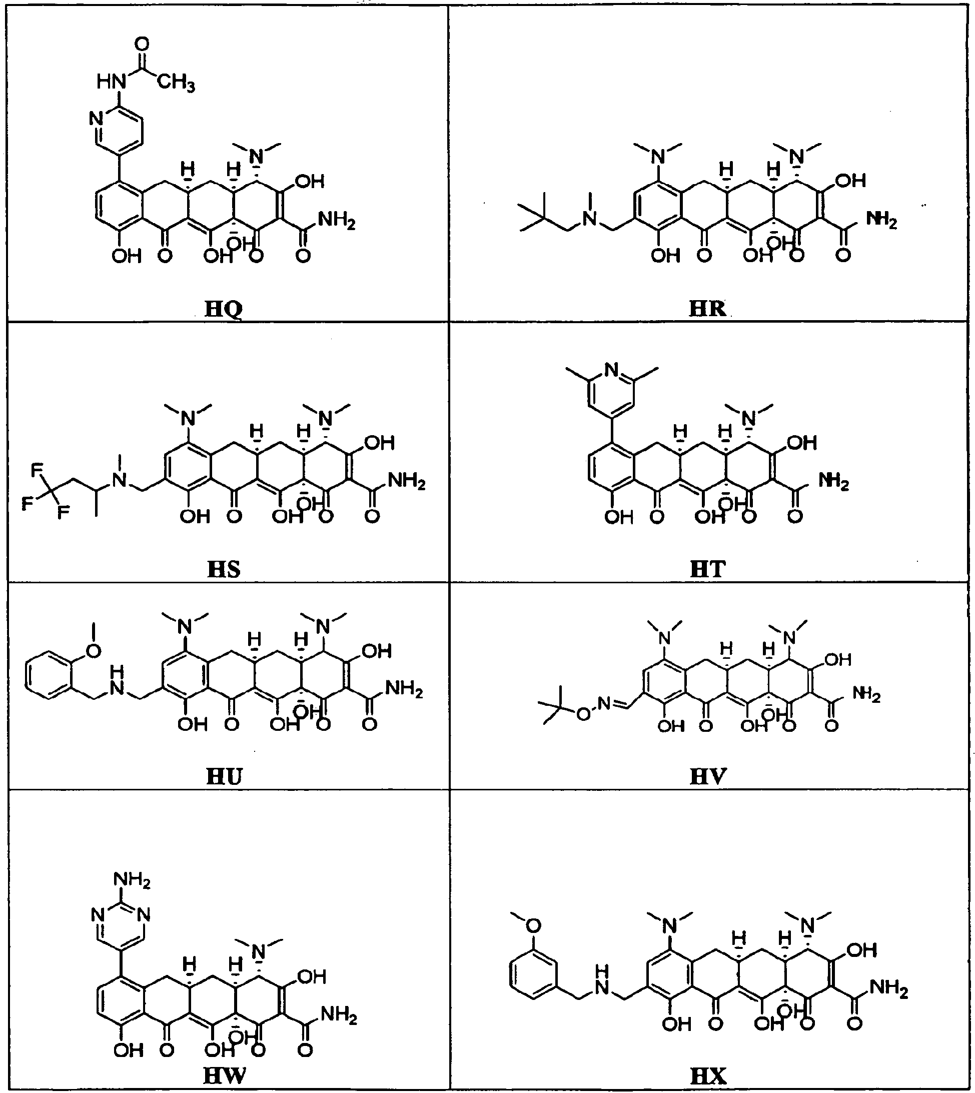

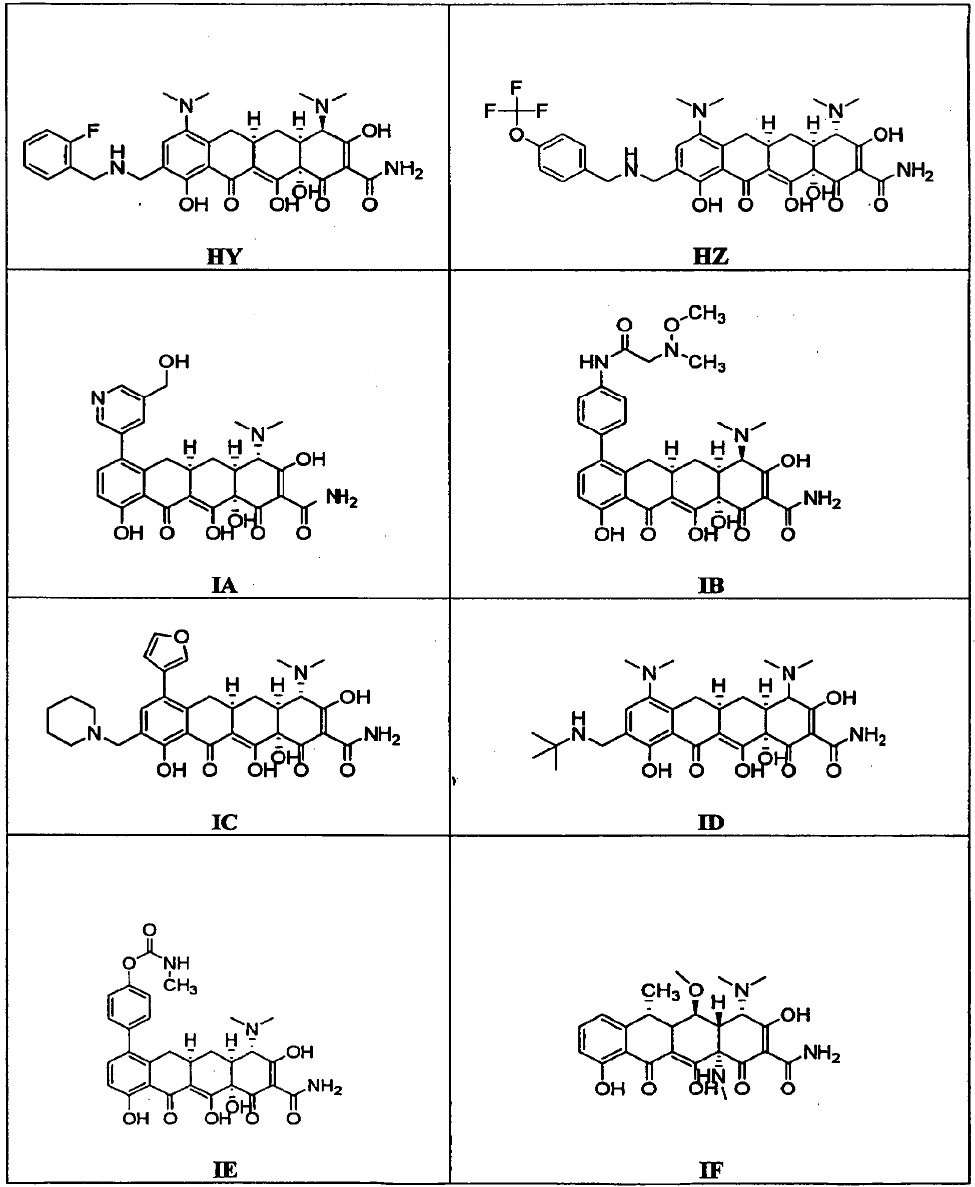

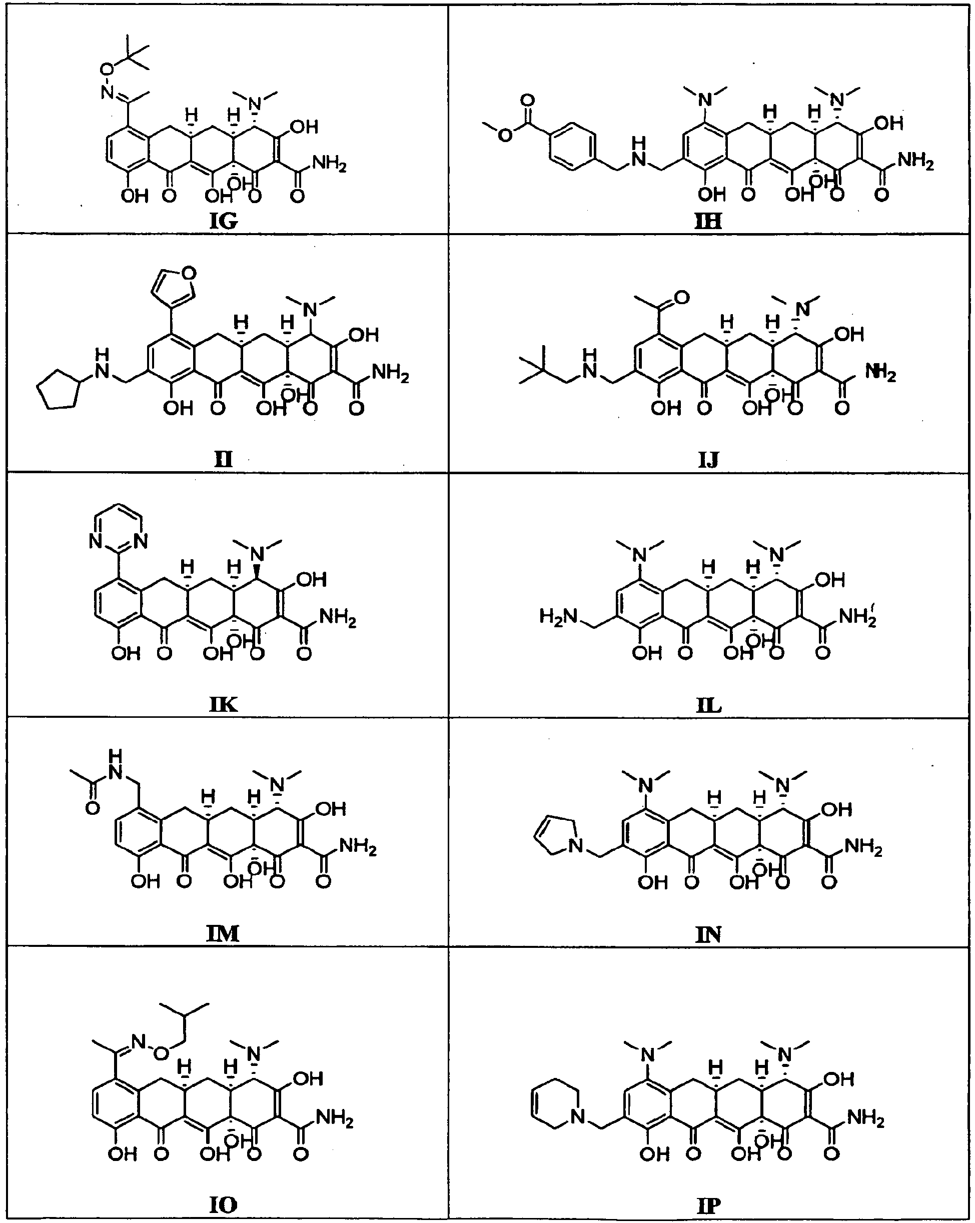

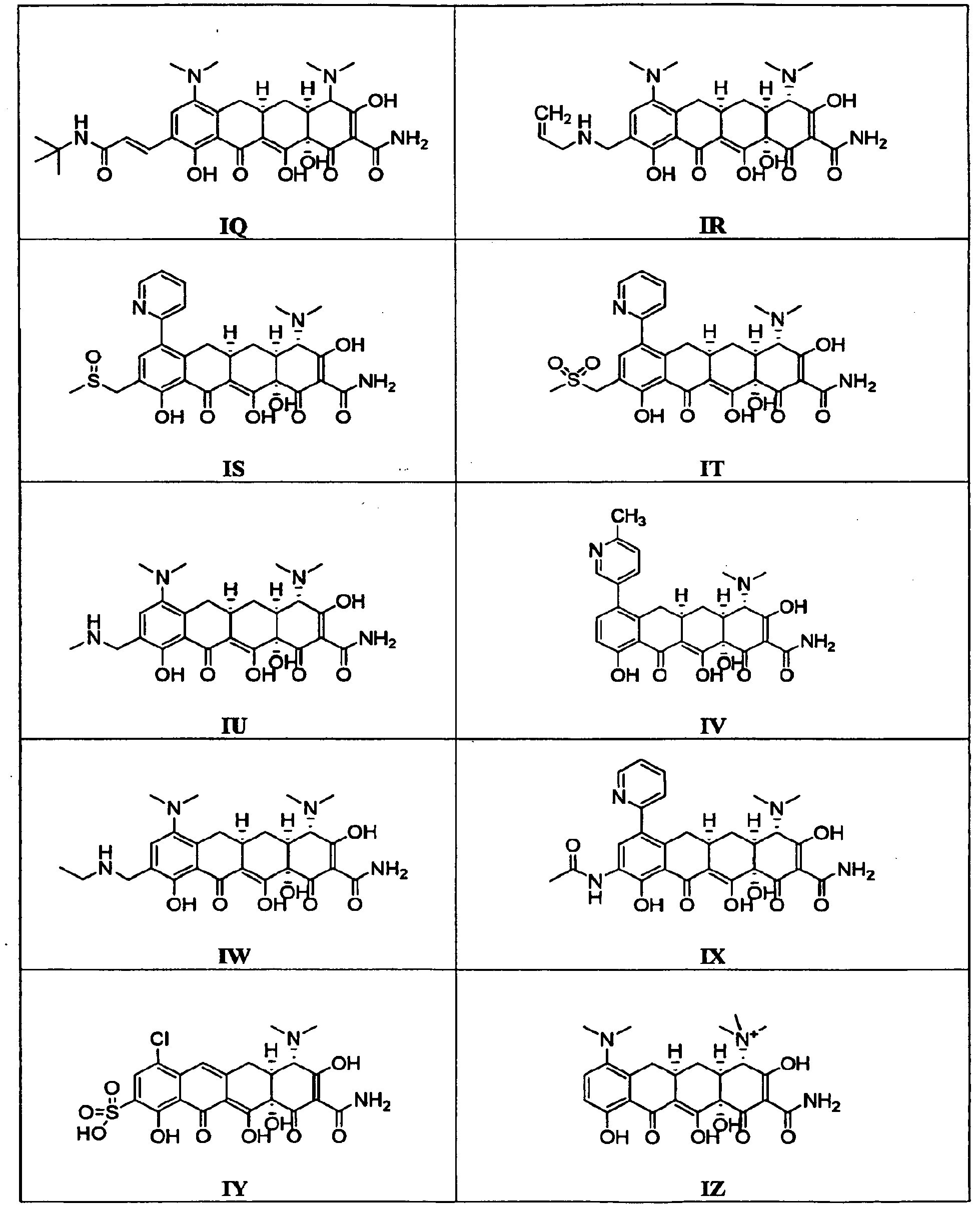

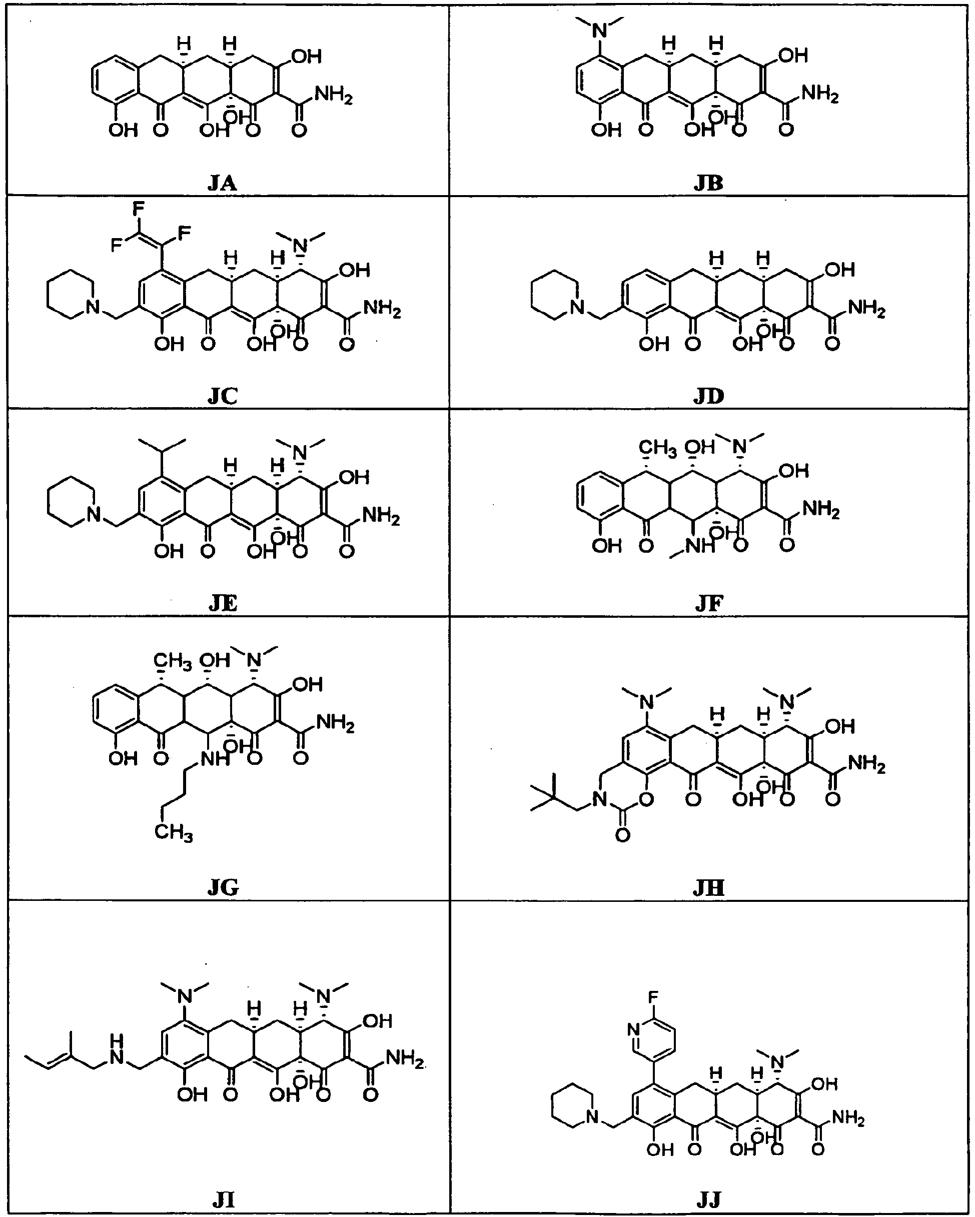

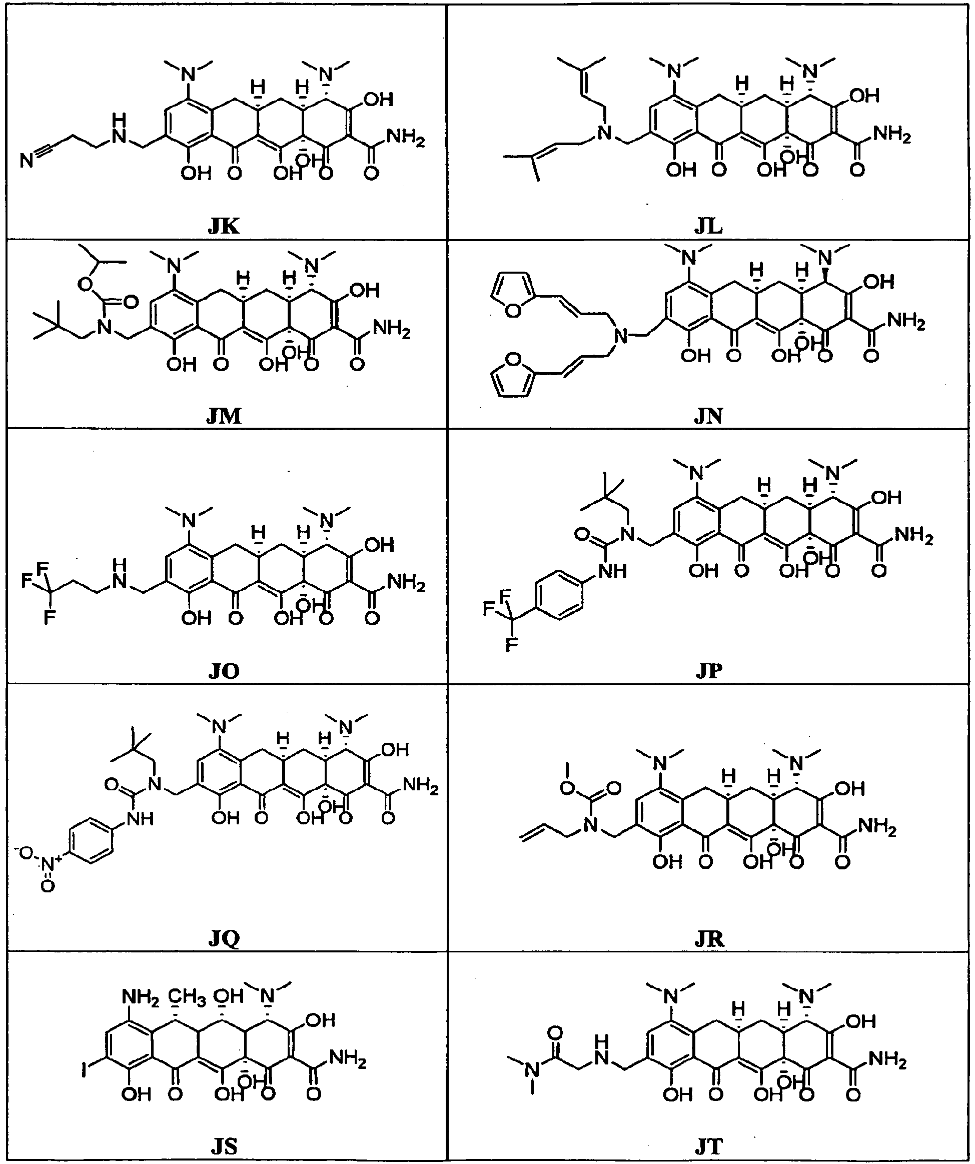

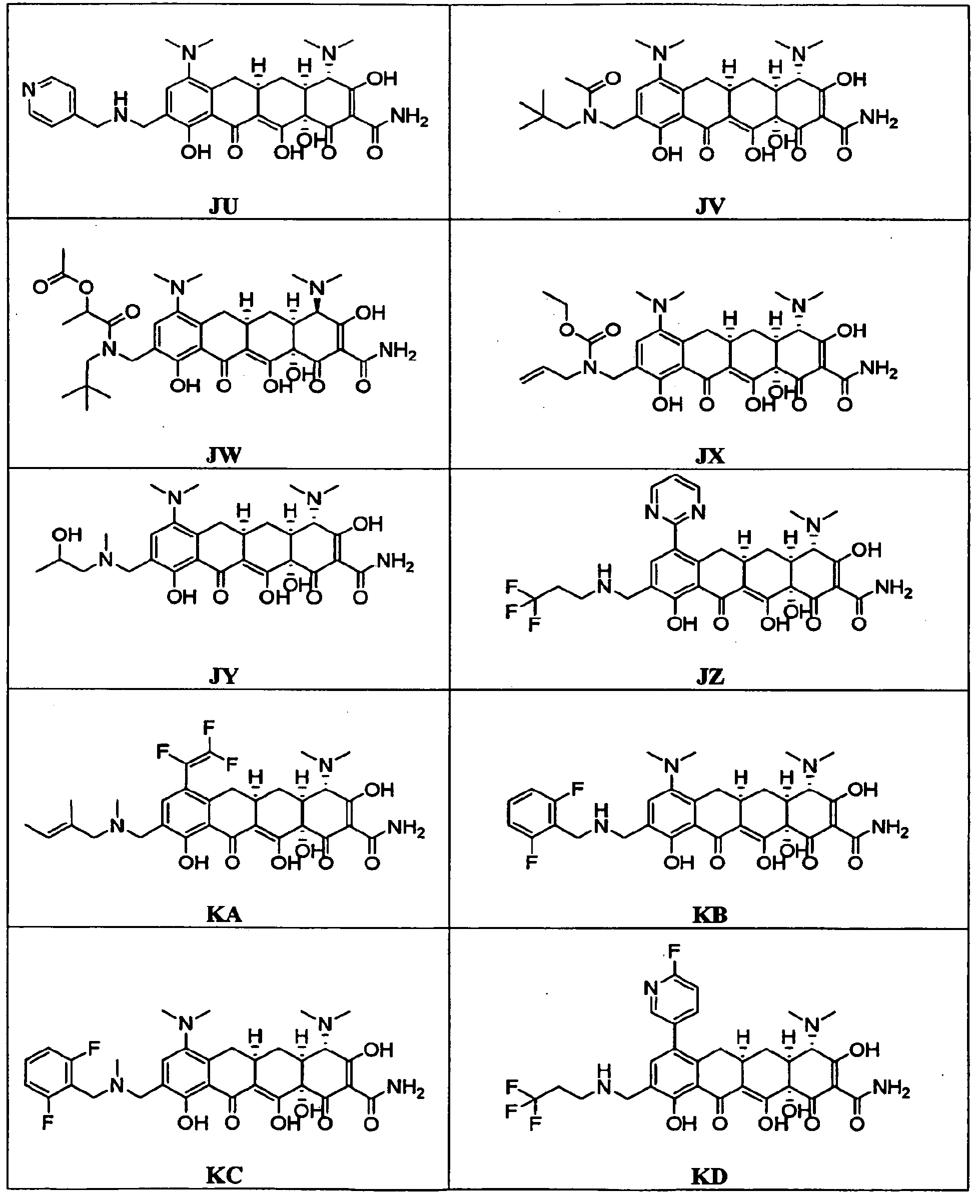

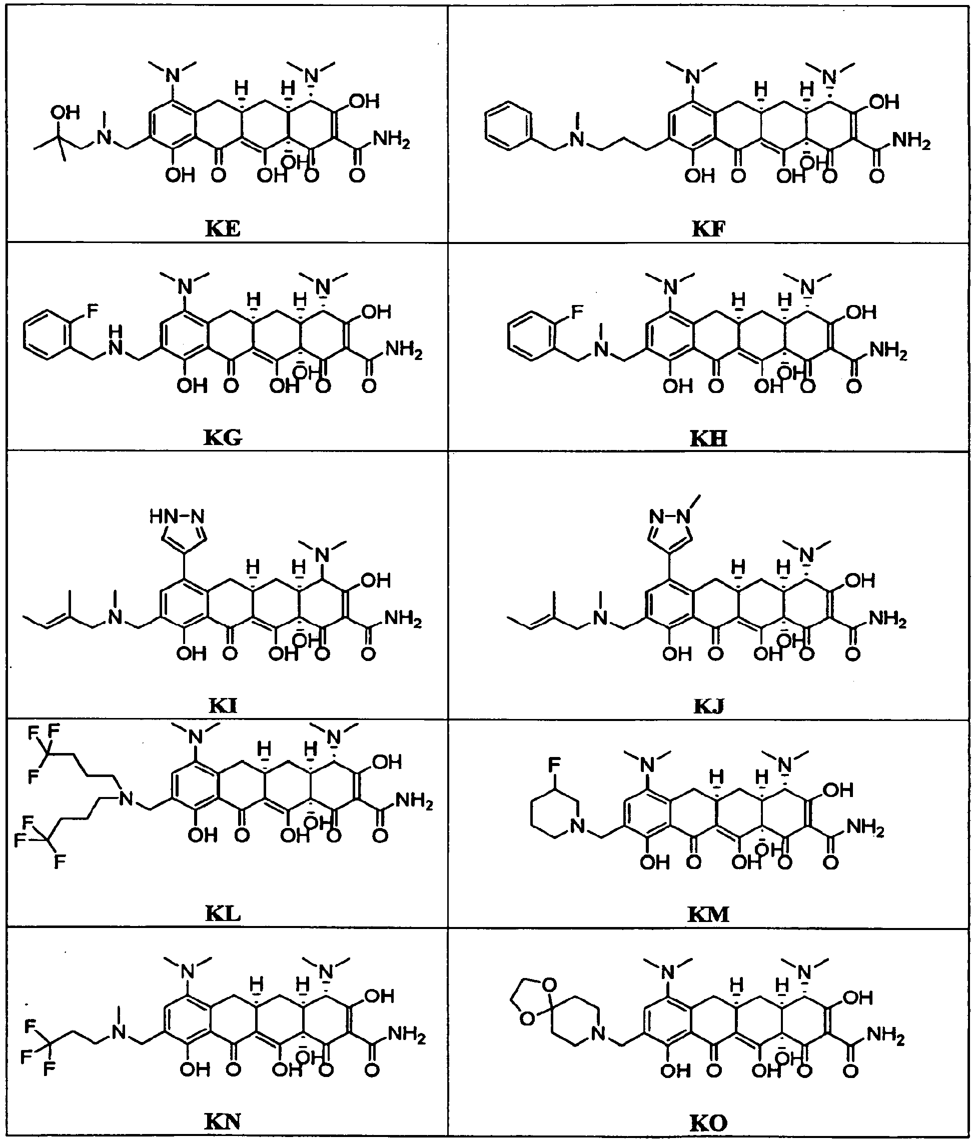

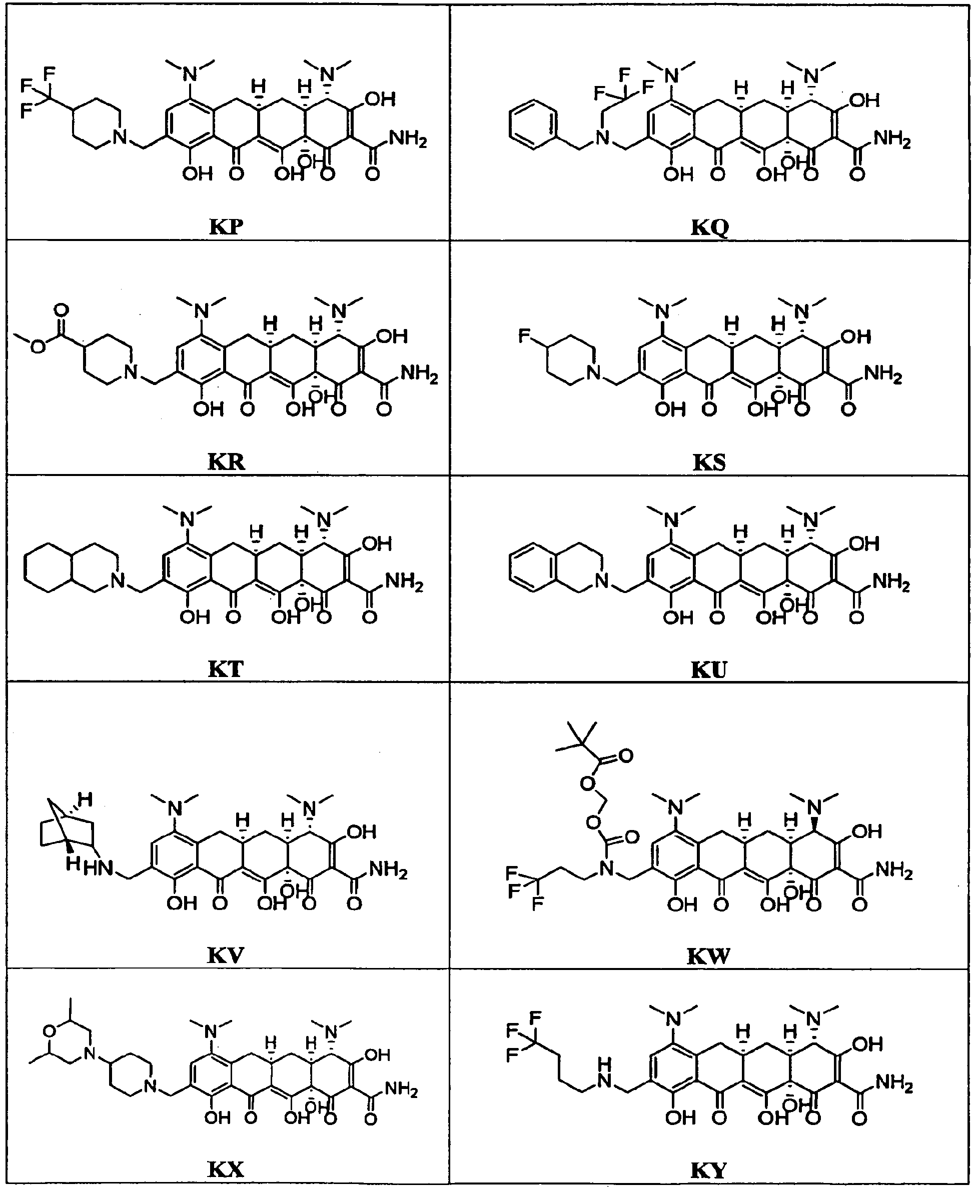

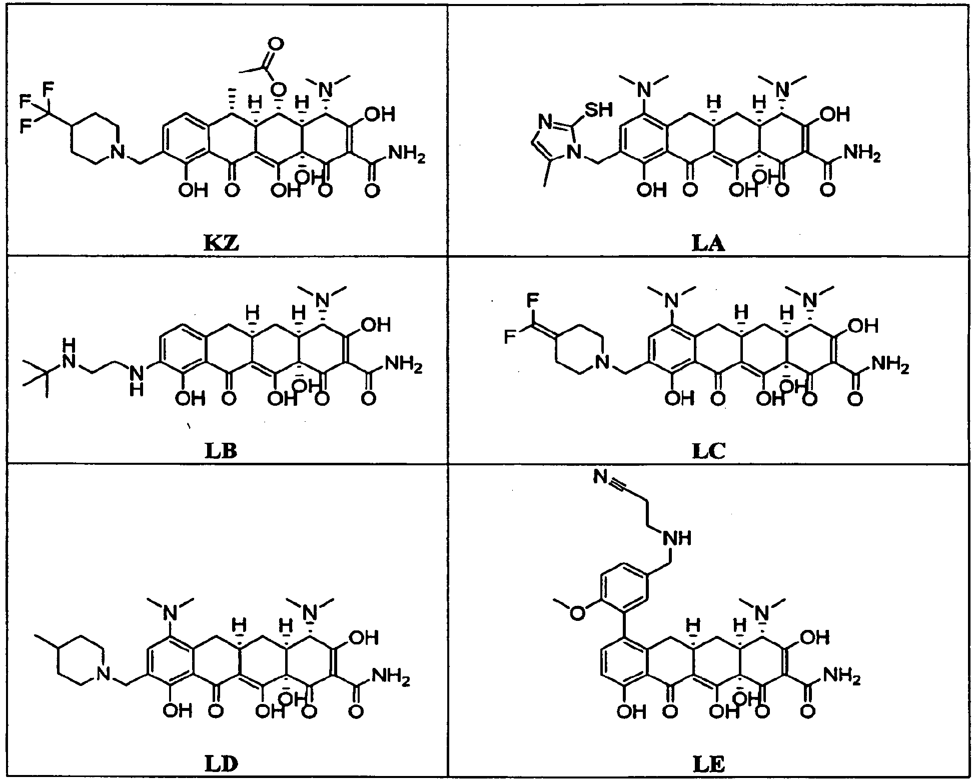

and pharmaceutically acceptable salts thereof.

In one embodiment, the substituted tetracycline compound exhibits a Klux of greater than 70 at a concentration of at least about 13 µg/mL when the substituted tetracycline is screened with the the tetA promoter/operator, as illustrated by Example 5. In another embodiment, the substituted tetracycline compound exhibits a Klux of between about 70 and about 51 at a concentration of at least about 13 µg/mL when the substituted tetracycline is screened with the tetA promoter/operator.

The substituted tetracycline compounds of the invention can be synthesized using the methods described in the following schemes and/or by using art recognized techniques. All novel substituted tetracycline compounds described herein are included in the invention as compounds.

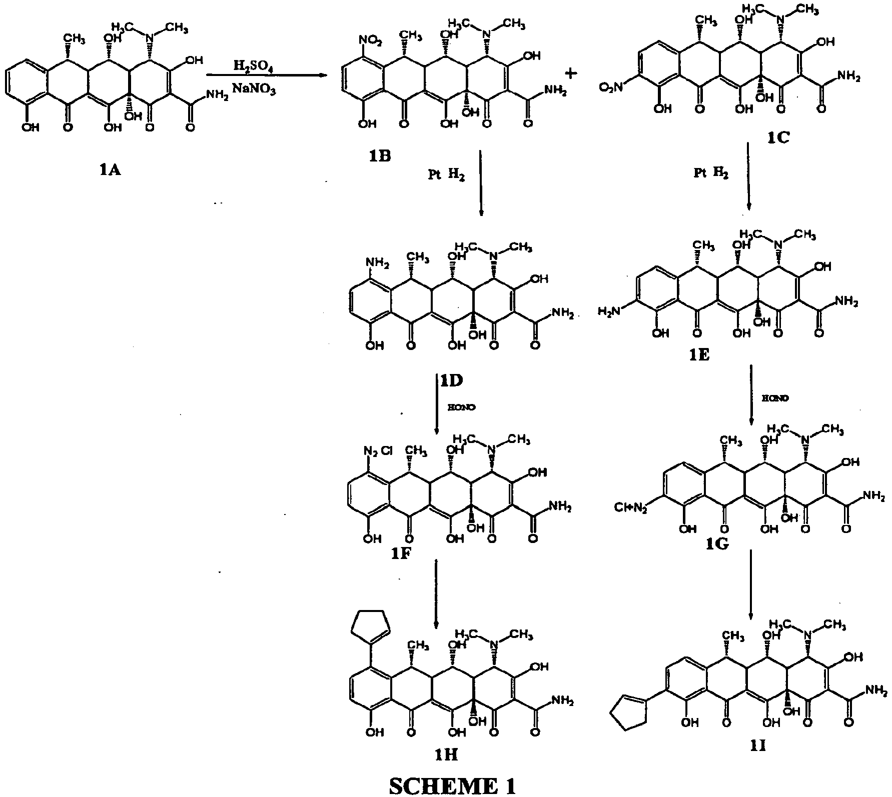

9- and 7- substituted tetracyclines can be synthesized by the method shown in Scheme 1. As shown in Scheme 1, 9- and 7-substituted tetracycline compounds can be synthesized by treating a tetracycline compound (e.g., doxycycline, 1A), with sulfuric acid and sodium nitrate. The resulting product is a mixture of the 7-nitro and 9-nitro isomers (1B and 1C, respectively). The 7-nitro (1B) and 9- nitro (1C) derivatives are treated by hydrogenation using hydrogen gas and a platinum catalyst to yield amines 1D and 1E. The isomers are separated at this time by conventional methods. To synthesize 7- or 9-substituted alkenyl derivatives, the 7- or 9-amino tetracycline compound (1E and 1F, respectively) is treated with HONO, to yield the diazonium salt (1G and 1H). The salt (1G and 1H) is treated with an appropriate reactive reagent to yield the desired compound(e.g., in Scheme 1, 7-cyclopent-1-enyl doxycycline (1H) and 9-cyclopent-1-enyl doxycycline (1I)).

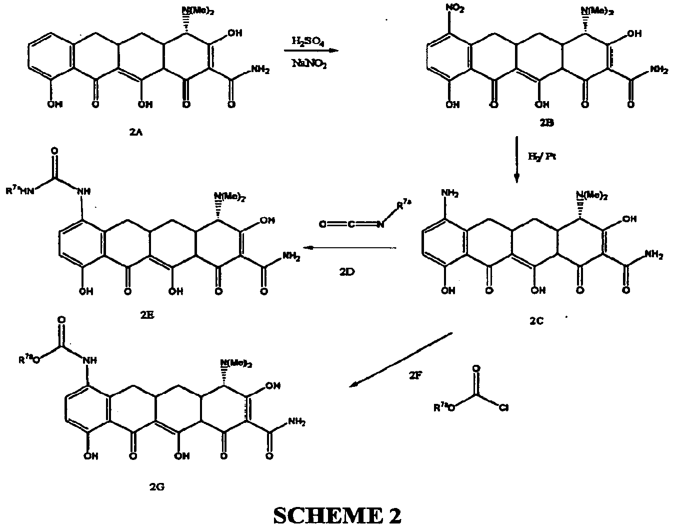

As shown in Scheme 2, tetracycline compounds of the invention wherein R⁷ is a carbamate or a urea derivative can be synthesized using the following protocol. Sancycline (2A) is treated with NaNO₂ under acidic conditions forming 7-nitro sancycline (2B) in a mixture of positional isomers. 7-nitrosancycline (2B) is then treated with H₂ gas and a platinum catalyst to form the 7-amino sancycline derivative (2C). To form the urea derivative (2E), isocyanate (2D) is reacted with the 7-amino sancycline derivative (2C). To form the carbamate (2G), the appropriate acid chloride ester (2F) is reacted with 2C.

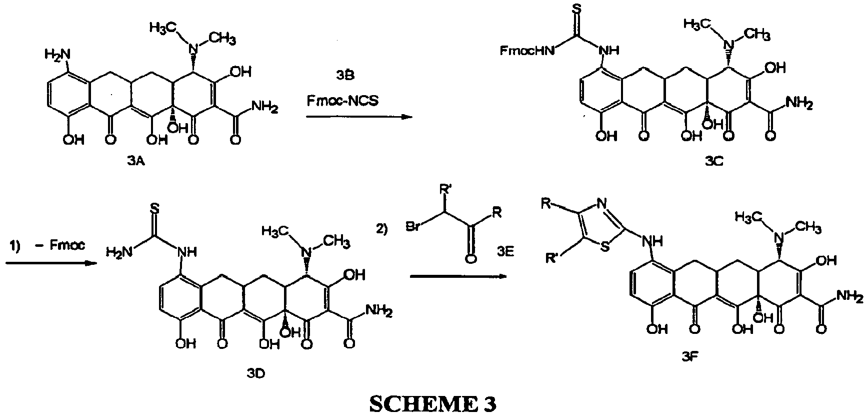

As shown in Scheme 3, tetracycline compounds of the invention, wherein R⁷ is a heterocyclic (i.e. thiazole) substituted amino group can be synthesized using the above protocol. 7-amino sancycline (3A) is reacted with Fmoc-isothiocyanate (3B) to produce the protected thiourea (3C). The protected thiourea (3C) is then deprotected yielding the active sancycline thiourea (3D) compound. The sancycline thiourea (3D) is reacted with an α-haloketone (3E) to produce a thiazole substituted 7-amino sancycline (3F).

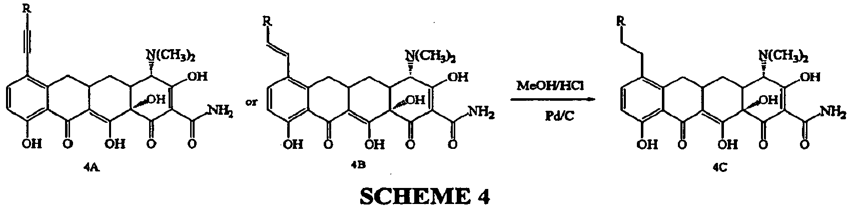

7- alkenyl tetracycline compounds, such as 7-alkynyl sancycline (4A) and 7-alkenyl sancycline (4B), can be hydrogenated to form 7-alkyl substituted tetracycline compounds (e.g., 7-alkyl sancycline, 4C). Scheme 4 depicts the selective hydrogenation of the 7- position double or triple bond, in saturated methanol and hydrochloric acid solution with a palladium/carbon catalyst under pressure, to yield the product.

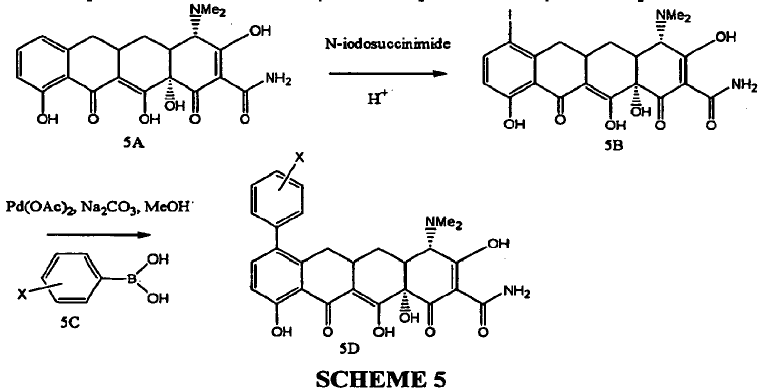

In Scheme 5, a general synthetic scheme for synthesizing 7-position aryl derivatives is shown. A Suzuki coupling of an aryl boronic acid with an iodosancycline compound is shown. An iodo sancycline compound (5B) can be synthesized from sancycline by treating sancycline (5A) with at least one equivalent N-iodosuccinimide (NIS) under acidic conditions. The reaction is quenched, and the resulting 7-iodo sancycline (5B) can then be purified using standard techniques known in the art. To form the aryl derivative, 7-iodo sancycline (5B) is treated with an aqueous base (e.g., Na₂CO₃) and an appropriate boronic acid (5C) and under an inert atmosphere. The reaction is catalyzed with a palladium catalyst (e.g., Pd(OAc)₂). The product (5D) can be purified by methods known in the art (such as HPLC). Other 7-aryl, alkenyl, and alkynyl tetracycline compounds can be synthesized using similar protocols.

The 7-substituted tetracycline compounds of the invention can also be synthesized using Stille cross couplings. Stille cross couplings can be performed using an appropriate tin reagent (e.g., R-SnBu₃) and a halogenated tetracycline compound, (e.g., 7-iodosancycline). The tin reagent and the iodosancycline compound can be treated with a palladium catalyst (e.g., Pd(PPh₃)₂Cl₂ or Pd(AsPh₃)₂Cl₂) and, optionally, with an additional copper salt, e.g., CuI. The resulting compound can then be purified using techniques known in the art.

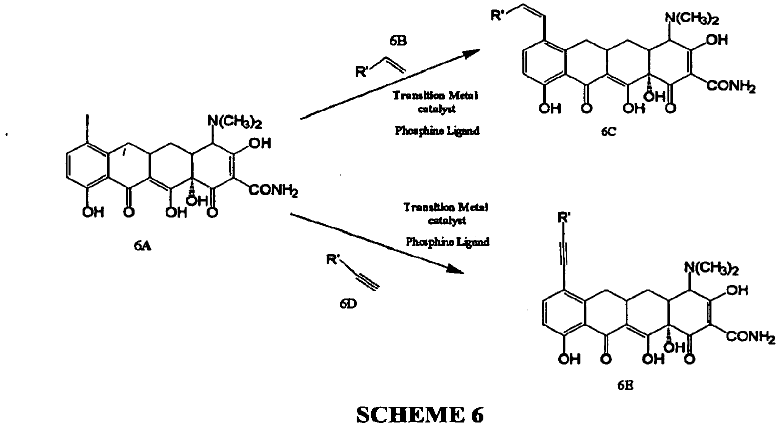

The compounds of the invention can also be synthesized using Heck-type cross coupling reactions. As shown in Scheme 6, Heck-type cross-couplings can be performed by suspending a halogenated tetracycline compound (e.g., 7-iodosancycline, 6A) and an appropriate palladium or other transition metal catalyst (e.g., Pd(OAc)₂ and CuI) in an appropriate solvent (e.g., degassed acetonitrile). The substrate, a reactive alkene (6B) or alkyne (6D), and triethylamine are then added and the mixture is heated for several hours, before being cooled to room temperature. The resulting 7-substituted alkenyl (6C) or 7-substituted alkynyl (6E) tetracycline compound can then be purified using techniques known in the art.

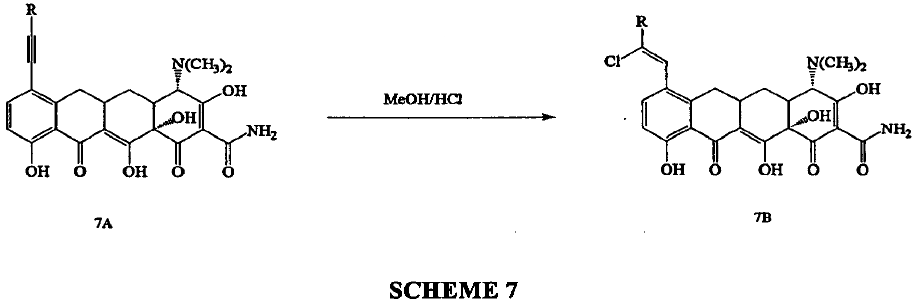

To prepare 7-(2'-Chloro-alkenyl)-tetracycline compounds, the appropriate 7-(alkynyl)-sancycline (7A) is dissolved in saturated methanol and hydrochloric acid and stirred. The solvent is then removed to yield the product (7B).

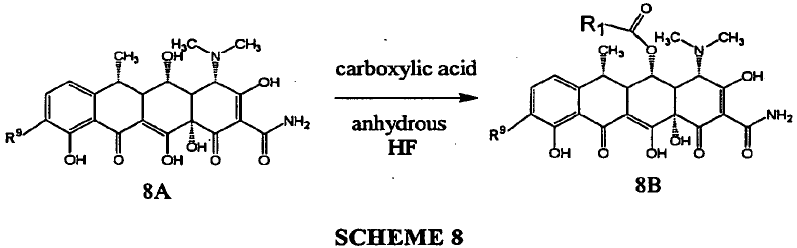

As depicted in Scheme 8, 5-esters of 9- substituted tetracycline compounds can be formed by dissolving the 9- substituted compounds (8A) in strong acid (e.g. HF, methanesulphonic acid, and trifluoromethanesulfonic acid) and adding the appropriate carboxylic acid to yield the corresponding esters (8B).

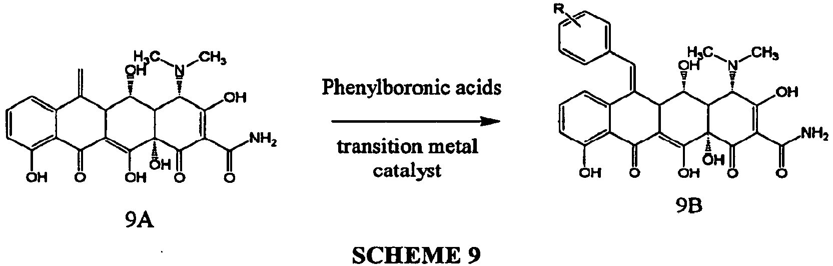

As shown in Scheme 9, methacycline (9A) can be reacted with a phenylboronic acid in the presence of a palladium catalyst such as Pd(OAc)₂ to form a 13 aryl substituted methacycline compound. The resulting compound can then be purified using techniques known in the art such as preparative HPLC and characterized.

As shown in Scheme 10 below, 7 and 9 aminomethyl tetracyclines may be synthesized using reagents such as hydroxymethyl-carbamic acid benzyl ester.

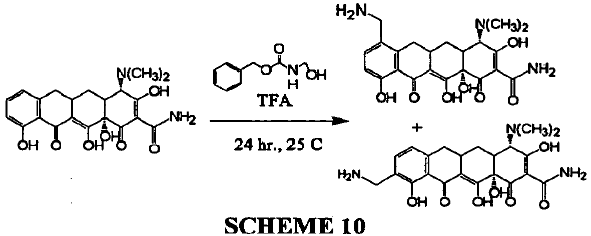

Substituted tetracycline compounds substituted at the 3, 10 or 12a position can be synthesized by contacting the tetracycline compound with a base to deprotonate the hydroxyl group. Examples of bases that can be used include potassium hydride and sodium hydroxide. The tetracyclines can then be further derivatized by using halides and other reactive species known in the art.

Other examples of chemical syntheses are described in WO 03/079984, WO 03/075857, WO 03/057169, and U.S.S.N. 101619,653; the entire contents of each of which are hereby incorporated herein by reference.

The term "alkyl" includes saturated aliphatic groups, including straight-chain alkyl groups (*e.g.,* methyl, ethyl, propyl, butyl, pentyl, hexyl, heptyl, octyl, nonyl, decyl, *etc.*)*,* branched-chain alkyl groups (*e.g.,* isopropyl, tert-butyl, isobutyl, *etc.*)*,* cycloalkyl (alicyclic) groups (*e.g.,* cyclopropyl, cyclopentyl, cyclohexyl, cycloheptyl, cyclooctyl), alkyl substituted cycloalkyl groups, and cycloalkyl substituted alkyl groups. The term alkyl further includes alkyl groups, which can further include oxygen, nitrogen, sulfur or phosphorous atoms replacing one or more carbons of the hydrocarbon backbone. In certain embodiments, a straight chain or branched chain alkyl has 20 or fewer carbon atoms in its backbone (*e.g.,* C₁-C₂₀ for straight chain, C₃-C₂₀ for branched chain), and more preferably 4 or fewer. Cycloalkyls may have from 3-8 carbon atoms in their ring structure, and more preferably have 5 or 6 carbons in the ring structure. The term C₁-C₆ includes alkyl groups containing 1 to 6 carbon atoms.

Moreover, the term alkyl includes both "unsubstituted alkyls" and "substituted alkyls," the latter of which refers to alkyl moieties having substituents replacing a hydrogen on one or more carbons of the hydrocarbon backbone. Such substituents can include, for example, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety. Cycloalkyls can be further substituted, *e.g.,* with the substituents described above. An "alkylaryl" or an "arylalkyl," moiety is an alkyl substituted with an aryl (*e.g.,* phenylmethyl (benzyl)). The term "alkyl" also includes the side chains of natural and unnatural amino acids.

The term "aryl" includes groups, including 5- and 6-membered single-ring aromatic groups that may include from zero to four heteroatoms, for example, benzene, phenyl, pyrrole, furan, thiophene, thiazole, isothiazole, imidazole, triazole, tetrazole, pyrazole, oxazole, isooxazole, pyridine, pyrazine, pyridazine, and pyrimidine, and the like. Furthermore, the term "aryl" includes multicyclic aryl groups, *e*.*g.*, tricyclic, bicyclic, *e.g.,* naphthalene, benzoxazole, benzodioxazole, benzothiazole, benzoimidazole, benzothiophene, methylenedioxophenyl, quinoline, isoquinoline, naphthridine, indole, benzofuran, purine, benzofuran, deazapurine, or indolizine. Those aryl groups having heteroatoms in the ring structure may also be referred to as "aryl heterocycles", "heterocycles," "heteroaryls" or "heteroaromatics". The aromatic ring can be substituted at one or more ring positions with such substituents as described above, as for example, halogen, hydroxyl, alkoxy, alkylcarbonyloxy, arylcarbonyloxy, alkoxycarbonyloxy, aryloxycarbonyloxy, carboxylate, alkylcarbonyl, alkylaminocarbonyl, arylalkyl aminocarbonyl, alkenylaminocarbonyl, alkylcarbonyl, arylcarbonyl, arylalkylcarbonyl, alkenylcarbonyl, alkoxycarbonyl, aminocarbonyl, alkylthiocarbonyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, allcylaryl, or an aromatic or heteroaromatic moiety. Aryl groups can also be fused or bridged with alicyclic or heterocyclic rings which are not aromatic so as to form a polycycle (e.g., tetralin).

The term "alkenyl" includes unsaturated aliphatic groups analogous in length and possible substitution to the alkyls described above, but that contain at least one double bond.

For example, the term "alkenyl" includes straight-chain alkenyl groups (*e.g.,* ethylenyl, propenyl, butenyl, pentenyl, hexenyl, heptenyl, octenyl, nonenyl, decenyl, *etc.*)*,* branched-chain alkenyl groups, cycloalkenyl (alicyclic) groups (cyclopropenyl, cyclopentenyl, cyclohexenyl, cycloheptenyl, cyclooctenyl), alkyl or alkenyl substituted cycloalkenyl groups, and cycloalkyl or cycloalkenyl substituted alkenyl groups. The term alkenyl further includes alkenyl groups which include oxygen, nitrogen, sulfur or phosphorous atoms replacing one or more carbons of the hydrocarbon backbone. In certain embodiments, a straight chain or branched chain alkenyl group has 20 or fewer carbon atoms in its backbone (*e.g*., C₂-C₂₀ for straight chain, C₃-C₂₀ for branched chain). Likewise, cycloalkenyl groups may have from 3-8 carbon atoms in their ring structure, and more preferably have 5 or 6 carbons in the ring structure. The term C₂-C₂₀ includes alkenyl groups containing 2 to 20 carbon atoms.

Moreover, the term alkenyl includes both "unsubstituted alkenyls" and "substituted alkenyls," the latter of which refers to alkenyl moieties having substituents replacing a hydrogen on one or more carbons of the hydrocarbon backbone. Such substituents can include, for example, alkyl groups, alkynyl groups, halogens, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfinyl, sulfonyl, thio, sulfonato, sulfamoyl, sulfonamido, nitro, trifluoromethyl, cyano, azido, heterocyclyl, alkylaryl, or an aromatic or heteroaromatic moiety.

The term "alkynyl" includes unsaturated aliphatic groups analogous in length and possible substitution to the alkyls described above, but which contain at least one triple bond.

For example, the term "alkynyl" includes straight-chain alkynyl groups (e.g., ethynyl, propynyl, butynyl, pentynyl, hexynyl, heptynyl, octynyl, nonynyl, decynyl, etc.), branched-chain alkynyl groups, and cycloalkyl or cycloalkenyl substituted alkynyl groups. The term alkynyl further includes alkynyl groups which include oxygen, nitrogen, sulfur or phosphorous atoms replacing one or more carbons of the hydrocarbon backbone. In certain embodiments, a straight chain or branched chain alkynyl group has 20 or fewer carbon atoms in its backbone (e.g., C₂-C₂₀ for straight chain, C₃-C₂₀ for branched chain). The term C₂-C₆ includes alkynyl groups containing 2 to 6 carbon atoms.

Moreover, the term alkynyl includes both "unsubstituted alkynyls" and "substituted alkynyls," the latter of which refers to alkynyl moieties having substituents replacing a hydrogen on one or more carbons of the hydrocarbon backbone. Such substituents can include, for example, alkyl groups, alkynyl groups, halogens, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including, e.g., alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

Unless the number of carbons is otherwise specified, "lower alkyl" as used herein means an alkyl group, as defined above, but having from one to five carbon atoms in its backbone structure. "Lower alkenyl" and "lower alkynyl" have chain lengths of, for example, 2-5 carbon atoms.

The term "acyl" includes compounds and moieties which contain the acyl radical (CH₃CO-) or a carbonyl group and includes both "unsubstituted acyl" groups and "substituted acyl" groups. The term "substituted acyl group" refers to where one or more of the hydrogen atoms are replaced by for example, alkyl groups, alkenyl, alkynyl groups, halogens, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

The term "acylamino" includes moieties wherein an acyl moiety is bonded to an amino group. For example, the term includes alkylcarbonylamino, arylcarbonylamino, carbamoyl and ureido groups.

The term "alkoxy" includes substituted and unsubstituted alkyl, alkenyl, and alkynyl groups covalently linked to an oxygen atom. Examples of alkoxy groups include methoxy, ethoxy, isopropyloxy, propoxy, butoxy, and pentoxy groups. Examples of substituted alkoxy groups include halogenated alkoxy groups. The alkoxy groups can be substituted with groups such as alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moieties. Examples of halogen substituted alkoxy groups include, but are not limited to, fluoromethoxy, difluoromethoxy, trifluoromethoxy, chloromethoxy, dichloromethoxy, trichloromethoxy, *etc*.

The terms "alkoxyalkyl," "alkylaminoalkyl" and "thioalkoxyalkyl" include alkyl groups, as described above, which further include oxygen, nitrogen or sulfur atoms replacing one or more carbons of the hydrocarbon backbone, *e.g.,* oxygen, nitrogen or sulfur atoms.

The term "amide" or "aminocarboxy" includes compounds or moieties which contain a nitrogen atom which is bound to the carbon of a carbonyl or a thiocarbonyl group. The term includes "alkaminocarboxy" groups which include alkyl, alkenyl, or alkynyl groups bound to an amino group bound to a carboxy group. It includes arylaminocarboxy groups which include aryl or heteroaryl moieties bound to an amino group which is bound to the carbon of a carbonyl or thiocarbonyl group. The terms "alkylaminocarboxy," "alkenylaminocarboxy," "alkynylaminocarboxy," and "arylaminocarboxy" include moieties wherein alkyl, alkenyl, alkynyl and aryl moieties, respectively, are bound to a nitrogen atom which is in turn bound to the carbon of a carbonyl group.

The term "amine" or "amino" includes compounds where a nitrogen atom is covalently bonded to at least one carbon or heteroatom. The term "alkyl amino" includes groups and compounds wherein the nitrogen is bound to at least one additional alkyl group. The term "dialkyl amino" includes groups wherein the nitrogen atom is bound to at least two additional alkyl groups. The term "arylamino" and "diarylamino" include groups wherein the nitrogen is bound to at least one or two aryl groups, respectively. The term "alkylarylamino," "alkylaminoaryl" or "arylaminoalkyl" refers to an amino group which is bound to at least one alkyl group and at least one aryl group. The term "alkaminoalkyl" refers to an alkyl, alkenyl, or alkynyl group bound to a nitrogen atom which is also bound to an alkyl group.

The term "aroyl" includes compounds and moieties with an aryl or heteroaromatic moiety bound to a carbonyl group. Examples of aroyl groups include phenylcarboxy, naphthyl carboxy, *etc.*

The term "carbonyl" or "carboxy" includes compounds and moieties which contain a carbon connected with a double bond to an oxygen atom. Examples of moieties which contain a carbonyl include aldehydes, ketones, carboxylic acids, amides, esters, anhydrides, *etc*. The carbonyl groups can be substituted with groups such as alkyl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moieties.

The term "ester" includes compounds and moieties which contain a carbon or a heteroatom bound to an oxygen atom which is bonded to the carbon of a carbonyl group. The term "ester" includes alkoxycarboxy groups such as methoxycarbonyl, ethoxycarbonyl, propoxycarbonyl, butoxycarbonyl, pentoxycarbonyl, *etc*. The alkyl, alkenyl, or alkynyl groups are as defined above.

The term "ether" includes compounds or moieties which contain an oxygen bonded to two different carbon atoms or heteroatoms. For example, the term includes "alkoxyalkyl" which refers to an alkyl, alkenyl, or alkynyl group covalently bonded to an oxygen atom which is covalently bonded to another alkyl group.

The term "halogen" includes fluorine, bromine, chlorine, iodine, *etc*. The term "perhalogenated" generally refers to a moiety wherein all hydrogens are replaced by halogen atoms.

The term "heteroatom" includes atoms of any element other than carbon or hydrogen. Preferred heteroatoms are nitrogen, oxygen, sulfur and phosphorus.

The term "hydroxy" or "hydroxyl" includes groups with an -OH or -O⁻ X⁺, where X⁺ is a counterion.

The terms "polycyclyl" or "polycyclic radical" refer to two or more cyclic rings (*e.g*., cycloalkyls, cycloalkenyls, cycloalkynyls, aryls and/or heterocyclyls) in which two or more carbons are common to two adjoining rings, *e.g*., the rings are "fused rings". Rings that are joined through non-adjacent atoms are termed "bridged" rings. Each of the rings of the polycycle can be substituted with such substituents as described above, as for example, halogen, hydroxyl, alkylcarbonyloxy, arylcarbonyloxy, alkoxycarbonyloxy, aryloxycarbonyloxy, carboxylate, alkylcarbonyl, alkoxycarbonyl, alkylaminoacarbonyl, arylalkylaminocarbonyl, alkenylaminocarbonyl, alkylcarbonyl, arylcarbonyl, arylalkyl carbonyl, alkenylcarbonyl, aminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkyl, alkylaryl, or an aromatic or heteroaromatic moiety.

The term "thiocarbonyl" or "thiocarboxy" includes compounds and moieties which contain a carbon connected with a double bond to a sulfur atom.

The term "thioether" includes compounds and moieties which contain a sulfur atom bonded to two different carbon or hetero atoms. Examples of thioethers include, but are not limited to alkthioalkyls, alkthioalkenyls, and alkthioalkynyls. The term "alkthioalkyls" include compounds with an alkyl, alkenyl, or alkynyl group bonded to a sulfur atom which is bonded to an alkyl group. Similarly, the term "alkthioalkenyls" and alkthioalkynyls" refer to compounds or moieties wherein an alkyl alkenyl, or alkynyl group is bonded to a sulfur atom which is covalently bonded to an alkynyl group.

The term "oximyl" includes moieties which comprise an oxime group.

The term "dimeric moiety" includes moieties which comprise a second tetracycline four ring structure. The dimeric moiety may be attached to the substituted tetracycline through a chain of from 1-30 atoms. The chain may be comprised of atoms covalently linked together through single, double and triple bonds. The tetracycline ring structure of the dimeric moiety may further be substituted or unsubstituted. It may be attached at the 1, 2, 3, 4, 5, 6, 7, 8, 9, 10, 11, 11a, 12, 12a, and/or 13 position.

The term "prodrug moiety" includes moieties which can be metabolized *in vivo.* Generally, the prodrugs moieties are metabolized *in vivo* by esterases or by other mechanisms to hydroxyl groups or other advantageous groups. Examples of prodrugs and their uses are well known in the art (See, *e.g*., Berge et al. (1977) "Pharmaceutical Salts", J. Pharm. Sci. 66:1-19). The prodrugs can be prepared *in situ* during the final isolation and purification of the compounds, or by separately reacting the purified compound in its free acid form or hydroxyl with a suitable esterifying agent. Hydroxyl groups can be converted into esters *via* treatment with a carboxylic acid. Examples of prodrug moieties include substituted and unsubstituted, branch or unbranched lower alkyl ester moieties, (*e.g*., propionoic acid esters), lower alkenyl esters, di-lower alkylamino lower-alkyl esters (*e.g*., dimethylaminoethyl ester), acylamino lower alkyl esters (*e.g*., acetyloxymethyl ester), acyloxy lower alkyl esters (*e.g.,* pivaloyloxymethyl ester), aryl esters (phenyl ester), aryl-lower alkyl esters (*e.g.,* benzyl ester), substituted (*e.g.,* with methyl, halo, or methoxy substituents) aryl and aryl-lower alkyl esters, amides, lower-alkyl amides, di-lower alkyl amides, and hydroxy amides. Preferred prodrug moieties are propionoic acid esters and acyl esters. Prodrugs which are converted to active forms through other mechanisms *in vivo* are also included.

The structures of some of the substituted tetracycline compounds used in the methods and compositions of the invention include asymmetric carbon atoms. The isomers arising from the chiral atoms (e.g., all enantiomers and diastereomers) are included within the scope of this invention, unless indicated otherwise. Such isomers can be obtained in substantially pure form by classical separation techniques and by stereochemically controlled synthesis. Furthermore, the structures and other compounds **and** moieties discussed in this application also include all tautomers thereof.

### II. Tetracycline-Inducible Transcriptional Regulators

In the tetracycline regulated gene expression system, transcription of a gene is modulated by a transcriptional regulator, e.g., activated by an activator protein (or reverse transactivator protein) or inhibited by transcriptional silencer proteins. The transactivators and silencers of the invention are fusion proteins or non-covalently associated proteins. Certain methods of the invention thus feature fusion proteins and nucleic acids (e.g., DNA) encoding fusion proteins or non-covalently associated proteins.

In one embodiment, transcription of a gene or gene product is activated by a tetracycline controlled transcriptional activator protein (tTA) or a reverse tetracycline controlled transcriptional activator protein (rtTA), both also referred to herein simply as transactivators. In the absence of a tetracycline a tTA binds to a TRE and activates expression from the target nucleic acid sequence. Conversely, the rtTA only recognizes the TRE in the presence of a tetracycline and, accordingly, transcription of the target nucleic acid sequence is stimulated by the rtTA only in the presence of a tetracycline.

The methods of the invention may also feature transcriptional silencer fusion proteins. The inhibitor fusion proteins of the methods of the invention are constructed similarly to the transcriptional regulator fusion proteins of the invention but instead of containing a polypeptide domain that stimulates transcription in a cell, the inhibitor fusion proteins contain a polypeptide domain that inhibits transcription in eukaryotic cells. The inhibitor fusion proteins are used to downregulate the expression of a gene or gene product operably linked to tetO sequences. For example, when a tetO-linked gene is introduced into a host cell or animal, the level of basal, constitutive expression of the gene may vary depending upon the type of cell or tissue in which the gene is introduced and on the site of integration of the gene. Alternatively, constitutive expression of endogenous genes into which tetO sequences have been introduced may vary depending upon the strength of additional endogenous regulatory sequences in the vicinity. The inhibitor fusion proteins described herein provide compositions that can be used to inhibit the expression of such tetO-linked genes in a controlled manner.

For example, the inhibitor fusion protein of the methods of the invention may comprise a first polypeptide that binds to tet operator sequences in the absence, but not the presence, of a substituted tetracycline compound operatively linked to a heterologous second polypeptide that inhibits transcription in eukaryotic cells. Alternatively, the inhibitor fusion protein may comprise a first polypeptide that binds to tet operator sequences in the presence, but not the absence, of a substituted tetracycline compound operatively linked to a heterologous second polypeptide that inhibits transcription in eukaryotic cells.

### A. The First Polypeptide Of A Transactivator Or Inhibitor Fusion Protein

In one embodiment, a transactivator fusion protein featured in certain methods of the invention is composed, in part, of a first polypeptide which binds to a tet operator sequence in the absence of a substituted tetracycline compound of the invention.

In one embodiment, e.g., when making a tTA fusion protein, the first polypeptide is a wild-type Tet repressor (which binds to tet operator sequences in the absence but not the presence of tetracycline). A wild-type Tet repressor of any class (e.g., A, B, C, D or E) may be used as the first polypeptide. In light of the high degree of sequence conservation (at least 80%) among members of each class of Tet repressor, a single member of each class of Tet repressor is used herein as representative of the entire class. Accordingly, the teaching of the present invention with respect to a specific member of a Tet repressor class is directly applicable to all members of that class.

As used herein, the TetR(A) class is represented by the Tet repressor carried on the Tn1721 transposon (Allmeir et al. (1992) Gene 111(1): 11-20; NCBI (National Library of Medicine, National Center for Biotechnology Information) accession number X61367 and database cross reference number (GI:) for encoded protein sequence GI:48198).

The TetR(B) class is represented by a Tet repressor encoded by a Tn*10* tetracycline resistance determinant (Postle et al. (1984) Nucleic Acids Research 12(12): 4849-63, Accession No. X00694, GI:43052).

The TetR(C) class is represented by the tetracycline repressor of the plasmid pSC101 (Brow et al. (1985) Mol. Biol. Evol. 2(1): 1-12, Accession No. M36272, GI: 150496).

The TetR(D) class is represented by the Tet repressor identified in *Salmonella ordonez* (Allard et al. (1993) Mol. Gen. Genet. 237(1-2): 301-5, Accession No. X65876, GI:49075).

The TetR(E) class is represented by a Tet repressor isolated from a member of *Enterobacteriaceae* (Tovar et al. (1988) Mol. Gen. Genet. 215(1): 76-80, Accession No. M34933, GI:155020).

The TetR(G) class is represented by a Tet repressor identified in *Vibrio anguillarum* (Zhao et al. (1992) Microbiol Immunol 36(10): 1051-60, Accession No. S52438, GI:262929).

The TetR(H) class is represented by a Tet repressor encoded by plasmid pMV111 isolated from *Pasteurella multocida* (Hansen et al. (1993) Antimicrob. Agents. Chemother. 37(12): 2699-705, Accession No. U00792, GI:392872).

The TetR(J) class is represented by a Tet repressor cloned from *Proteus mirabilis* (Magalhaes et al. (1998) Biochim. Biophys. Acta. 1443(1-2): 262-66, Accession No. AF038993, GI:4104706).

The TetR(Z) class is represented by a Tet repressor encoded by the pAG1 plasmid isolated from the gram-positive organism *Corynebacterium glutamicum* (Tauch et al. (2000) Plasmid 44(3): 285-91, Accession No. AAD25064, GI:4583400).

Preferably, the wild-type Tet repressor is a class B tet repressor, e.g., a Tn10-derived Tet repressor.

In another embodiment, a transactivator fusion protein featured in certain methods of the invention is composed, in part, of a first polypeptide which binds to a tet operator sequence in the presence of a substituted tetracycline compound of the invention. Accordingly, in one embodiment, e.g., when making an rtTA fusion protein, the first polypeptide of the fusion protein is a mutated Tet repressor. The amino acid difference(s) between a mutated Tet repressor and a wild-type Tet repressor may be substitution of one or more amino acids, deletion of one or more amino acids or addition of one or more amino acids. Preferably, the mutated Tet repressor of the invention has the following functional properties: 1) the polypeptide can bind to a tet operator sequence, i.e., it retains the DNA binding specificity of a wild-type Tet repressor; and 2) it is regulated in a reverse manner by substituted tetracycline compounds compared to a wild-type Tet repressor, i.e., the mutated Tet repressor binds to a tet operator sequence only the presence of a substituted tetracycline compound rather than in the absence of a substituted tetracycline compound.

In an embodiment, a mutated Tet repressor having the functional properties described above is created by substitution of amino acid residues in the sequence of a wild-type Tet repressor. For example, as described in US 5,789,156, a Tn10-derived Tet repressor having amino acid substitutions at amino acid positions 71, 95, 101 and 102 has the desired functional properties and thus can be used as the first polypeptide in the transcriptional regulator fusion protein of the invention. Mutation of fewer than all four of these amino acid positions may be sufficient to achieve a Tet repressor with the desired functional properties. Accordingly, in the embodiment, a Tet repressor is preferably mutated at at least one of these positions. Other amino acid substitutions, deletions or additions at these or other amino acid positions which retain the desired functional properties of the mutated Tet repressor are within the scope of the methods of the invention. The crystal structure of a Tet repressor-tetracycline complex, as described in Hinrichs, W. et al. (1994) Science 264:418-420, can be used for rational design of mutated Tet repressors. Based upon this structure, amino acid position 71 is located outside the tetracycline binding pocket, suggesting mutation at this site may not be necessary to achieve the desired functional properties of a mutated Tet repressor of the invention. In contrast, amino acid positions 95, 101 and 102 are located within the conserved tetracycline binding pocket. Thus, the tetracycline binding pocket of a Tet repressor may be targeted for mutation to create a mutated Tet repressor of the invention.

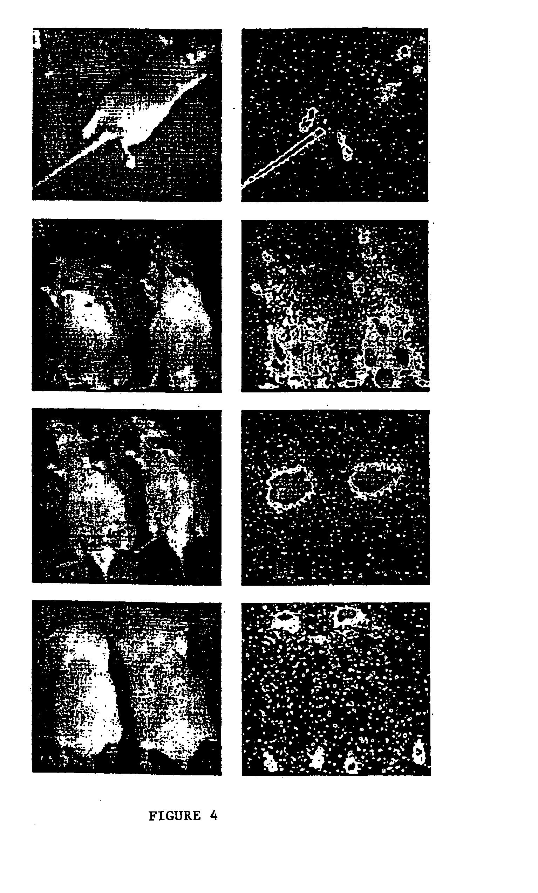

Additional mutated Tet repressors for incorporation into a fusion protein of the methods of the invention can be created according to the teachings of the invention. A number of different classes of Tet repressors have been described, e.g., A, B, C, D and E (of which the Tn10 encoded repressor is a class B repressor). The amino acid sequences of the different classes of Tet repressors share a high degree of homology (i.e., 40-60% across the length of the proteins), including in the region encompassing the above-described mutations. The amino acid sequences of various classes of Tet repressors are shown and compared in US 5,789,156 (FIG. 4), and are also described in Tovar, K. et al. (1988) Mol. Gen. Genet. 215:76-80. Accordingly, equivalent mutations to those described above for the Tn10-derived Tet repressor can be made in other classes of Tet repressors for inclusion in a fusion protein of the invention. For example, amino acid position 95, which is an aspartic acid in all five repressor classes, can be mutated to asparagine in any class of repressor. Similarly, position 102, which is glycine in all five repressor classes, can be mutated to aspartic acid in any class of repressor. Additional suitable equivalent mutations will be apparent to those skilled in the art and can be created and tested for finctionality by procedures described herein. Nucleotide and amino acid sequences of Tet repressors of the A, C, D and E classes are disclosed in Waters, S. H. et al. (1983) Nucl. Acids Res 11:6089-6105, Unger, B. et al. (1984) Gene 3: 103-108, Unger, B. et al. (1984) Nucl Acids Res. 12:7693-7703 and Tovar, K. et al. (1988) Mol. Gen. Genet. 215:76-80, respectively. These wild-type sequences can be mutated according to the teachings of the invention for use in the inducible regulatory system described herein.

Alternative to the above-described mutations, additional suitable mutated Tet repressors (e.g., having the desired functional properties described above) can be created by mutagenesis of a wild type Tet repressor and selection as described in US 5,789,156 (Example 1). The nucleotide and amino acid sequences of wild-type class B Tet repressors are disclosed in Hillen, W. and Schollmeier, K. (1983) Nucl. Acids Res. 11:525-539 and Postle, K. et al. (1984) Nucl. Acids Res. 12:4849-4863. References for the nucleotide and amino acid sequences of wild-type class A, C, D and E type repressors are cited above. A mutated Tet repressor can be created and selected, for example as follows: a nucleic acid (e.g., DNA) encoding a wild-type Tet repressor is subjected to random mutagenesis and the resultant mutated nucleic acids are incorporated into an expression vector and introduced into a host cell for screening. A screening assay, e.g., which allows for selection of a Tet repressor which binds to a Tet operator sequence only in the presence of a substituted tetracycline compound can be used. For example, a library of mutated nucleic acids in an expression vector can be introduced into an E. coli strain in which Tet operator sequences control the expression of a gene encoding a Lac repressor and the Lac repressor controls the expression of a gene encoding an selectable marker (e.g., drug resistance). Binding of a Tet repressor to Tet operator sequences in the bacteria will inhibit expression of the Lac repressor, thereby inducing expression of the selectable marker gene. Cells expressing the marker gene are selected based upon the selectable phenotype (e.g., drug resistance). For wild-type Tet repressors, expression of the selectable marker gene will occur in the absence of tetracycline. A nucleic acid encoding a mutated Tet repressor may be selected using this system based upon the ability of the nucleic acid to induce expression of the selectable marker gene in the bacteria only in the presence of a substituted tetracycline compound.

Another approach for creating a mutated Tet repressor which binds to a class A tet operator is to further mutate the already mutated Tn10-derived Tet repressor described herein (a class B repressor) such that it no longer binds efficiently to a class B type operator but instead binds efficiently to a class A type operator. It has been found that nucleotide position 6 of class A or B type operators is the critical nucleotide for recognition of the operator by its complimentary repressor (position 6 is a G/C pair in class B operators and an A/T pair in class A operators) (see Wissman et al. (1988) J. Mol. Biol. 202:397-406). It has also been found that amino acid position 40 of a class A or class B Tet repressor is the critical amino acid residue for recognition of position 6 of the operator (amino acid position 40 is a threonine in class B repressors but is an alanine in class A repressors). It still further has been found that substitution of Thr40 of a class B repressor with Ala alters its binding specificity such that the repressor can now bind a class A operator (similarly, substitution of Ala40 of a class A repressor with Thr alters its binding specificity such that the repressor can now bind a class B operator) (see Altschmied et al. (1988) EMBO J. 7:4011-4017). Accordingly, one can alter the binding specificity of the mutated Tn10-derived Tet repressor disclosed herein by additionally changing amino acid residue 40 from Thr to Ala by standard molecular biology techniques (e.g., site directed mutagenesis).

A mutated Tet repressor e.g., having specific mutations (e.g., at positions 71, 95, 101 and/or 102, as described above) can be created by introducing nucleotide changes into a nucleic acid encoding a wild-type repressor by standard molecular biology techniques, e.g. site directed mutagenesis or PCR-mediated mutagenesis using oligonucleotide primers incorporating the nucleotide mutations. Alternatively, when a mutated Tet repressor is identified by selection from a library, the mutated nucleic acid can be recovered from the library vector. To create a transcriptional regulator fusion protein of the invention, a nucleic acid encoding a mutated Tet repressor is then ligated in-frame to another nucleic acid encoding a transcriptional activation domain and the fusion construct is incorporated into a recombinant expression vector. The transcriptional regulator fusion protein can be expressed by introducing the recombinant expression vector into a host cell or animal.

### B. The Second Polypeptide Of The Transactivator Fusion Protein

The first polypeptide of the transactivator fusion protein is operatively linked to a second polypeptide which directly or indirectly activates transcription in eukaryotic cells. To operatively link the first and second polypeptides, typically, nucleotide sequences encoding the first and second polypeptides are ligated to each other in-frame to create a chimeric gene encoding a fusion protein. However, the first and second polypeptides can be operatively linked by other means that preserve the function of each polypeptide (e.g., chemically crosslinked). The second polypeptide of the transactivator may itself possess transcriptional activation activity (i.e., the second polypeptide directly activates transcription). The second polypeptide may also activate transcription by an indirect mechanism, through recruitment of a transcriptional activation protein to interact with the fusion protein.

Polypeptides which can function to activate transcription in eukaryotic cells are well known in the art. In particular, transcriptional activation domains of many DNA binding proteins have been described and have been shown to retain their activation function when the domain is transferred to a heterologous protein. A preferred polypeptide for use in the fusion protein of the methods of the invention is the herpes simplex virus virion protein 16 (referred to herein as VP16, the amino acid sequence of which is disclosed in Triezenberg, S. J. et al. (1988) Genes Dev. 2:718-729).

Other polypeptides with transcriptional activation ability in eukaryotic cells can be used in the fusion protein of the methods of the invention. Transcriptional activation domains found within various proteins have been grouped into categories based upon similar structural features. Types of transcriptional activation domains include acidic transcription activation domains, proline-rich transcription activation domains, serine/threonine-rich transcription activation domains and glutamine-rich transcription activation domains. Examples of acidic transcriptional activation domains include the VP 16 regions already described and amino acid residues 753-881 of GAL4. Examples of proline-rich activation domains include amino acid residues 399-499 of CTF/NF1 and amino acid residues 31-76 of AP2. Examples of serine/threonine-rich transcription activation domains include amino acid residues 1-427 of ITF1 and amino acid residues 2-451 of ITF2. Examples of glutamine-rich activation domains include amino acid residues 175-269 of Oct I and amino acid residues 132-243 of Sp1. The amino acid sequences of each of the above described regions, and of other useful transcriptional activation domains, are disclosed in Seipel, K. et al. (EMBO J. (1992) 13:4961-4968).

In addition to previously described transcriptional activation domains, novel transcriptional activation domains, which can be identified by standard techniques, are within the scope of the methods of the invention. The transcriptional activation ability of a polypeptide can be assayed by linking the polypeptide to another polypeptide having DNA binding activity and determining the amount of transcription of a target sequence that is stimulated by the fusion protein. For example, a standard assay used in the art utilizes a fusion protein of a putative transcriptional activation domain and a GAL4 DNA binding domain (e.g., amino acid residues 1-93). This fusion protein is then used to stimulate expression of a reporter gene linked to GAL4 binding sites (see e.g., Seipel, K. et al. (1992) EMBO J. 11:4961-4968 and references cited therein).

The second polypeptide of the fusion protein may indirectly activate transcription by recruiting a transcriptional activator to interact with the fusion protein. For example, a TetR or mutated TetR of the invention can be fused to a polypeptide domain (e.g., a dimerization domain) capable of mediating a protein-protein interaction with a transcriptional activator protein, such as an endogenous activator present in a host cell. It has been demonstrated that functional associations between DNA binding domains and transactivation domains need not be covalent (see e.g., Fields and Song (1989) Nature 340:245-247; Chien et al. (1991) Proc. Natl. Acad. Sci. USA 31:9578-9582; Gyuris et al. (1993) Cell 75:791-803; and Zervos, A. S. (1993) Cell 72:223-232). Accordingly, the second polypeptide of the fusion protein may not directly activate transcription but rather may form a stable interaction with an endogenous polypeptide bearing a compatible protein-protein interaction domain and transactivation domain. Examples of suitable interaction (or dimerization) domains include leucine zippers (Landschulz et al. (1989) Science 243:1681-1688), helix-loop-helix domains (Murre, C. et al. (1989) Cell 58:537-544) and zinc finger domains (Frankel, A. D. et al. (1988) Science 24:70-73). Interaction of a dimerization domain present in the fusion protein with an endogenous nuclear factor results in recruitment of the transactivation domain of the nuclear factor to the fusion protein, and thereby to a tet operator sequence to which the fusion protein is bound.

### C The Second Polypeptide Of The Transcriptional Silencer Fusion Protein

In one embodiment, the first polypeptide of the transcriptional silencer fusion protein is operatively linked to a second polypeptide which directly or indirectly inhibits transcription in eukaryotic cells. As described herein and as known in the art, to operatively link the first and second polypeptides of a fusion protein, typically nucleotide sequences encoding the first and second polypeptides are ligated to each other in-frame to create a chimeric gene encoding the fusion protein. However, the first and second polypeptides can be operatively linked by other means that preserve the function of each polypeptide (e.g., chemically crosslinked). Although the fusion proteins are typically described herein as having the first polypeptide at the amino-terminal end of the fusion protein and the second polypeptide at the carboxy-terminal end of the fusion protein, it will be appreciated by those skilled in the art that the opposite orientation (i.e., the second polypeptide at the amino-terminal end and the first polypeptide at the carboxy-terminal end) is also contemplated by the invention.

Proteins and polypeptide domains within proteins which can function to inhibit transcription in eukaryotic cells have been described in the art (for reviews see, e.g., Renkawitz, R. (1990) Trends in Genetics 6:192-197; and Herschbach, B.M. and Johnson, A. D. (1993) Annu. Rev. Cell. Biol. 9:479-509). Such transcriptional silencer domains have been referred to in the art as "silencing domains" or "repressor domains." Although the precise mechanism by which many of these polypeptide domains inhibit transcription is not known (and the invention is not intended to be limited by mechanism), there are several possible means by which repressor domains may inhibit transcription, including: 1) competitive inhibition of binding of either activator proteins or the general transcriptional machinery, 2) prevention of the activity of a DNA bound activator and 3) negative interference with the assembly of a functional preinitiation complex of the general transcription machinery. Thus, a repressor domain may have a direct inhibitory effect on the transcriptional machinery or may inhibit transcription indirectly by inhibiting the activity of activator proteins. Accordingly, the term "a polypeptide that inhibits transcription in eukaryotic cells" as used herein is intended to include polypeptides which act either directly or indirectly to inhibit transcription. As used herein, "inhibition" of transcription is intended to mean a diminution in the level or amount of transcription of a target nucleic acid sequence compared to the level or amount of transcription prior to regulation by the transcriptional silencer protein. Transcriptional inhibition may be partial or complete. The terms "silencer", "repressor" and "inhibitor" are used interchangeably herein to describe a regulatory protein, or domains thereof, that can inhibit transcription.

A transcriptional "repressor" or "silencer" domain as described herein is a polypeptide domain that retains its transcriptional repressor function when the domain is transferred to a heterologous protein. Proteins which have been demonstrated to have repressor domains that can function when transferred to a heterologous protein include the v-erbA oncogene product (Baniahmad, A. et al. (1992) EMBO J. 11:1015-1023), the thyroid hormone receptor (Baniahmad, supra), the retinoic acid receptor (Baniahmad, supra), and the Drosophila Krueppel (Kr) protein (Licht, J. D. et al. (1990) Nature 346:76-79; Sauer, F. and Jackle, H. (1991) Nature 2:563-566; Licht, J. D. et al. (1994) Mol. Cell. Biol. 14:4057-4066). Non-limiting examples of other proteins which have transcriptional repressor activity in eukaryotic cells include the Drosophila homeodomain protein even-skipped (eve), the S. cerevisiae Ssn6/Tup1 protein complex (see Herschbach and Johnson, supra), the yeast SIR1 protein (see Chien, et al. (1993) Cell 75:531-541), Nepa1 (see Kohne, et al. (1993) J. Mol. Biol 232:747-755), the Drosophila dorsal protein (see Kirov, et al. (1994) Mol. Cell. Biol. 14:713-722; Jiang, et al. (1993) EMBO J. 12:3201-3209), TSF3 (see Chen, et al. (1993) Mol. Cell. Biol. 13:831-840), SF1 (see Targa, et al. (1992) Biochem. Biophys. Res. Comm. 188:416-423), the Drosophila hunchback protein (see Zhang, et al. (1992) Proc. Natl. Acad. Sci. USA 89:7511-7515), the Drosophila knirps protein (see Gerwin, et al. (1994) Mol. Cell. Biol. 14:7899-7908), the WT1 protein (Wilm's tumor gene product) (see Anant, et al. (1994) Oncogene 9:3113-3126; Madden et al., (1993) Oncogene 8:1713-1720), Oct-2.1 (see Lillycrop, et al. (1994) Mol. Cell. Biol. 14:7633-7642), the Drosophila engrailed protein (see Badiani, et al. (1994) Genes Dev. 8:770-782; Han and Manley, (1993) EMBO J. 12:2723-2733), E4BP4 (see Cowell and Hurst, (1994) Nucleic Acids Res. 2:59-65) and ZF5 (see Numoto, et al. (1993) Nucleic Acids Res. 21:3767-3775).

The second polypeptide of the transcriptional silencer fusion protein of the methods of the invention may be a transcriptional silencer domain of the Drosophila Krueppel protein. A C-terminal region having repressor activity can be used such as amino acids 403-466 of the native protein (see Sauer, F. and Jackle, H., supra). This region is referred to as C64KR. Construction of an expression vector encoding a TetR C64KR fusion protein is described in US 5,789,156. Alternatively, an alanine-rich amino terminal region of Kr that also has repressor activity can be used as the second polypeptide of the fusion protein. For example, amino acids 26-110 of Kr (see Licht, J. D. et al., (1990) supra) can be used as the second polypeptide. Alternatively, shorter or longer polypeptide fragments encompassing either of the Kr silencer domains that still retain full or partial inhibitor activity are also contemplated (e.g., amino acids 62 to 92 of the N-terminal silencer domain; see Licht, et al. (1994) supra).

The second polypeptide of the transcriptional silencer fusion protein of the methods of the invention may be a transcriptional silencer domain of the v-erbA oncogene product. The silencer domain of v-erbA has been mapped to approximately amino acid residues 362-632 of the native v-erbA oncogene product (see Baniahmad, et al. supra). Accordingly, a fragment encompassing this region is used as the second polypeptide of the silencer domain. Amino acid residues 364-635 of the native v-erbA protein may be used. Alternatively, shorter or longer polypeptide fragments encompassing the v-erbA silencer region that still retain full or partial inhibitor activity are also contemplated. For example, a.a. residues 346-639, 362-639, 346-632, 346-616 and 362-616 ofv-erbA may be used. Additionally, polypeptide fragments encompassing these regions that have internal deletions yet still retain full or partial inhibitor activity are encompassed by the invention, such as a.a. residues 362-468/508-639 ofv-erbA. Furthermore, two or more copies of the silencer domain may be included in the fusion protein, such as two copies of a.a. residues 362-616 of v-erbA. Suitable silencer polypeptide domains ofv-erbA are described further in Baniahmad, A. et al. (supra).

Other silencer domains may also be used. Non-limiting examples of polypeptide domains that can be used include: amino acid residues 120-410 of the thyroid hormone receptor alpha (THRα), amino acid residues 143-403 of the retinoic acid receptor alpha (RARα), amino acid residues 186-232 of knirps, the N-terminal region of WT 1 (see Anant, supra), the N-terminal region of Oct-2.1 (see Lillycrop, supra), a 65 amino acid domain of E4BP4 (see Cowell and Hurst, supra) and the N-terminal zinc finger domain of ZF5 (see Numoto, supra). Moreover, shorter or longer polypeptide fragments encompassing these regions that still retain full or partial inhibitor activity are also contemplated.

In addition to previously described transcriptional silencer domains, novel transcriptional silencer domains, which can be identified by standard techniques, are within the scope of the methods of the invention. The transcriptional silencer ability of a polypeptide can be assayed by: 1) constructing an expression vector that encodes the test silencer polypeptide linked to another polypeptide having DNA binding activity (i.e., constructing a DNA binding domain-silencer domain fusion protein), 2) cotransfecting this expression vector into host cells together with a reporter gene construct that is normally constitutively expressed in the host cell and also contains binding sites for the DNA binding domain and 3) determining the amount of transcription of the reporter gene construct that is inhibited by expression of the fusion protein in the host cell. For example, a standard assay used in the art utilizes a fusion protein of a GAL4 DNA binding domain (e.g., amino acid residues 1-147) and a test silencer domain. This fusion protein is then used to inhibit expression of a reporter gene construct that contains positive regulatory sequences (that normally stimulate constitutive transcription) and GAL4 binding sites (see e.g., Baniahmad, supra).

### D. Optional Third Polypeptides Of The Transactivator Or Inhibitor Fusion Proteins

In addition to a TetR or mutated TetR and a transcriptional activation or inhibitor domain, a fusion protein of the methods of the invention can contain an operatively linked third polypeptide which promotes transport of the fusion protein to a cell nucleus. Amino acid sequences which, when included in a protein, function to promote transport of the protein to the nucleus are known in the art and are termed nuclear localization signals (NLS). Nuclear localization signals typically are composed of a stretch of basic amino acids. When attached to a heterologous protein (e.g., a fusion protein of the invention), the nuclear localization signal promotes transport of the protein to a cell nucleus. The nuclear localization signal is attached to a heterologous protein such that it is exposed on the protein surface and does not interfere with the function of the protein. Preferably, the NLS is attached to one end of the protein, e.g. the N-terminus. The amino acid sequence of a non-limiting example of an NLS that can be included in a fusion protein of the methods of the invention may be found in US 5,789,156. Preferably, a nucleic acid encoding the nuclear localization signal is spliced by standard recombinant DNA techniques in-frame to the nucleic acid encoding the fusion protein (e.g., at the 5' end).

### III. Target Transcription Units Regulated by a Tetracycline-Regulatable Systems

In one embodiment, the methods of the instant invention feature modulation of fusion protein(s) to regulate the transcription of a target nucleotide sequence. This target nucleotide sequence may be operatively linked to a TRE. Accordingly, another aspect of the invention relates to target nucleic acids (e.g., DNA molecules) comprising a nucleotide sequence to be transcribed operatively linked to a TRE. Such nucleic acid molecules are also referred to herein as tet-regulated transcription units (or simply transcription units).

Within a transcription unit, the "nucleotide sequence to be transcribed" typically includes a minimal promoter sequence which is not itself transcribed but which serves (at least in part) to position the transcriptional machinery for transcription. The minimal promoter sequence is linked to the transcribed sequence in a 5' to 3' direction by phosphodiester bonds (i.e., the promoter is located upstream of the transcribed sequence) to form a contiguous nucleotide sequence. Accordingly, the terms "nucleotide sequence to be transcribed" or "target nucleotide sequence" include both the nucleotide sequence which is transcribed into mRNA and an operatively linked upstream minimal promoter sequence. The term "minimal promoter" includes partial promoter sequences which define the start site of transcription for the linked sequence to be transcribed but which by itself is not capable of initiating transcription efficiently, if at all. Thus, the activity of such a minimal promoter is dependent upon the binding of a transcriptional activator (such as the tetracycline-inducible fusion protein of the invention) to an operatively linked regulatory sequence (such as one or more tet operator sequences). In one embodiment, the minimal promoter is from the human cytomegalovirus (as described in Boshart et al. (1985) Cell 41:521-530). Preferably, nucleotide positions between about +75 to -53 and +75 to -31 are used. Other suitable minimal promoters are known in the art or can be identified by standard techniques. For example, a functional promoter which activates transcription of a contiguously linked reporter gene (e.g., chloramphenicol acetyl transferase, β-galactosidase or luciferase) can be progressively deleted until it no longer activates expression of the reporter gene alone but rather requires the presence of an additional regulatory sequence(s).

Within a transcription unit, the target nucleotide sequence (including the transcribed nucleotide sequence and its upstream minimal promoter sequence) is operatively linked to at least one TRE, e.g., at least one tet operator sequence. In one embodiment, a TRE can include multiple copies (e.g., multimerized or concatemerized copies) of one or more tet operator sequences. In a typical configuration, the tet operator sequence(s) is operatively linked upstream (i.e., 5') of the minimal promoter sequence through a phosphodiester bond at a suitable distance to allow for transcription of the target nucleotide sequence upon binding of a regulatory protein (e.g., the transcriptional regulator fusion protein) to the tet operator sequence. That is, the transcription unit is comprised of, in a 5' to 3' direction: tet operator sequence(s)--a minimal promoter--a transcribed nucleotide sequence. It will be appreciated by those skilled in the art that there is some flexibility in the permissible distance between the tet operator sequence(s) and the minimal promoter, although typically the tet operator sequences will be located within about 200-400 base pairs upstream of the minimal promoter.

Exemplary nucleotide sequences of examples of tet-regulated promoters, containing tet operator sequences linked to a minimal promoter, that can be used in the invention are known in the art. For example, a cytomegalovirus minimal promoter linked to ten tet operator sequences can be used. Alternatively, a herpes simplex virus minimal tk promoter linked to ten tet operator sequences can be used. Exemplary promoters are described, e.g., in Gossen, M. and Bujard, H. (1992) Proc. Natl. Acad. Sci. USA 89:5547-5551.

Alternatively, since regulatory elements have been observed in the art to function downstream of sequences to be transcribed, it is likely that the tet operator sequence(s) can be operatively linked downstream (i.e., 3') of the transcribed nucleotide sequence. Thus, in this configuration, the transcription unit is comprised of, in a 5' to 3' direction: a minimal promoter--a transcribed nucleotide sequence--tet operator sequence(s). Again, it will be appreciated that there is likely to be some flexibility in the permissible distance downstream at which the tet operator sequence(s) can be linked.

A tet-regulated transcription unit can further be incorporated into a recombinant vector (e.g., a plasmid or viral vector) by standard recombinant DNA techniques. The transcription unit, or recombinant vector in which it is contained, can be introduced into a host cell by standard transfection techniques, such as those described above. It should be appreciated that, after introduction of the transcription unit into a population of host cells, it may be necessary to select a host cell clone which exhibits low basal expression of the tet operator-linked nucleotide sequence (i.e., selection for a host cell in which the transcription unit has integrated at a site that results in low basal expression of the tet operator-linked nucleotide sequence). Furthermore, a tet-regulated transcription unit can be introduced, by procedures described herein, into the genome of a non-human animal at an embryonic stage or into plant cells to create a transgenic or homologous recombinant organism carrying the transcription unit in some or all of its cells. Again, it should be appreciated that it may be necessary to select a transgenic or homologous organism in which there is low basal expression of the tet operator-linked nucleotide sequence in cells of interest.

The target nucleotide sequence of the tet-regulated transcription unit can encode a protein of interest. Thus, upon induction of transcription of the nucleotide sequence by the transactivator of the invention and translation of the resultant mRNA, the protein of interest is produced in a host cell or animal. Alternatively, the nucleotide sequence to be transcribed can encode for an active RNA molecule, e.g., an antisense RNA molecule or ribozyme. Expression of active RNA molecules in a host cell or animal can be used to regulate functions within the host (e.g., prevent the production of a protein of interest by inhibiting translation of the mRNA encoding the protein).

A transcriptional regulator of the methods of the invention can be used to regulate transcription of an exogenous nucleotide sequence introduced into the host cell or animal. An "exogenous" nucleotide sequence is a nucleotide sequence which is introduced into the host cell and typically is inserted into the genome of the host. The exogenous nucleotide sequence may not be present elsewhere in the genome of the host (e.g., a foreign nucleotide sequence) or may be an additional copy of a sequence which is present within the genome of the host but which is integrated at a different site in the genome. An exogenous nucleotide sequence to be transcribed and an operatively linked tet operator sequence(s) can be contained within a single nucleic acid molecule which is introduced into the host cell or animal.

Alternatively, a transcriptional regulator of the methods of the invention can be used to regulate transcription of an endogenous nucleotide sequence to which a tet operator sequence(s) has been linked. An "endogenous" nucleotide sequence is a nucleotide sequence which is present within the genome of the host. An endogenous gene can be operatively linked to a tet operator sequence(s) by homologous recombination between a tetO-containing recombination vector and sequences of the endogeneous gene. For example, a homologous recombination vector can be prepared which includes at least one tet operator sequence and a miminal promoter sequence flanked at its 3' end by sequences representing the coding region of the endogenous gene and flanked at its 5' end by sequences from the upstream region of the endogenous gene by excluding the actual promoter region of the endogenous gene. The flanking sequences are of sufficient length for successful homologous recombination of the vector DNA with the endogenous gene. Preferably, several kilobases of flanking DNA are included in the homologous recombination vector. Upon homologous recombination between the vector DNA and the endogenous gene in a host cell, a region of the endogenous promoter is replaced by the vector DNA containing one or more tet operator sequences operably linked to a minimal promoter. Thus, expression of the endogenous gene is no longer under the control of its endogenous promoter but rather is placed under the control of the tet operator sequence(s) and the minimal promoter.

In another embodiment, tet operator sequences can be inserted elsewhere within an endogenous gene, preferably within a 5' or 3' regulatory region, via homologous recombination to create an endogenous gene whose expression can be regulated by a substituted tetracycline compound-regulated fusion protein described herein. For example, one or more tetO sequences can be inserted into a promoter or enhancer region of an endogenous gene such that promoter or enhancer function is maintained (i.e., the tetO sequences are introduced into a site of the promoter/enhancer region that is not critical for promoter/enhancer function). Regions within promoters or enhancers which can be altered without loss of promoter/enhancer function are known in the art for many genes or can be determined by standard techniques for analyzing critical regulatory regions. An endogenous gene having tetO sequences inserted into a non-critical regulatory region will retain the ability to be expressed in its normal constitutive and/or tissue-specific manner but, additionally, can be downregulated by a substituted tetracycline compound-controlled transcriptional silencer protein in a controlled manner. For example, constitutive expression of such a modified endogenous gene can be inhibited by in the presence of a substituted tetracycline compound using an inhibitor fusion protein that binds to tetO sequences in the presence of a substituted tetracycline compound.

### IV. Altered Sensitivity of Transcriptional Regulator Molecules

In one embodiment, a transcriptional regulator of the invention has a novel phenotype such as decreased basal transcriptional activity in the absence of tetracyclines, increased induced transcriptional activity in the presence of tetracyclines, or differential induction by tetracycline and analogs of tetracycline.

In one aspect of the present invention, specific mutations or alterations are introduced into a transcriptional regulatory protein. In another aspect, random mutagenesis techniques, coupled with selection or screening systems, are used to introduce large numbers of mutations into a transcriptional regulatory protein. The resulting collection of randomly mutated proteins is then subjected to a selection for the desired phenotype or a screen in which the desired phenotype can be observed against a background of undesirable phenotypes.

In accordance with the random mutagenesis, in one aspect of the invention one can mutagenize an entire molecule or one can proceed by cassette mutagenesis. In the former instance, the entire coding region of a molecule is mutagenized by one of several methods (chemical, PCR, doped oligonucleotide synthesis), and the resulting collection of randomly mutated molecules is subjected to selection or screening procedures. Random mutagenesis can be applied in this way in cases where the molecule being studied is relatively small and there are powerful and stringent selections or screens available to discriminate between the different classes of mutant phenotypes that will inevitably arise.

Random mutagenesis may be accomplished by many means, including:
1. PCR mutagenesis, in which the error prone Taq polymerase is exploited to generate mutant alleles of transcriptional regulatory proteins, which are assayed directly in yeast for an ability to couple.
2. Chemical mutagenesis, in which expression cassettes encoding transcriptional regulatory proteins are exposed to mutagens and the protein products of the mutant sequences are assayed directly in yeast for an ability to couple.
3. Doped synthesis of oligonucleotides encoding portions of the transcriptional regulatory protein gene.
4. In vivo mutagenesis, in which random mutations are introduced into the coding region of transcriptional regulatory proteins by passage through a mutator strain of E. coli, XL1-Red (mutD5 mutS mutT) (Stratagene, Menasa, Wis.). Substitution of mutant peptide sequences for functional domains in a transcriptional regulatory protein permits the determination of specific sequence requirements for the accomplishment of function.

In accordance with the specific mutagenesis aspect of the invention, discrete regions of a protein, corresponding either to defined structural (i.e..alpha.-helices, .beta.-sheets, turns, surface loops) or functional determinants (e.g., DNA binding determinants, transcription regulatory domains) are subjected to saturating or semi-random mutagenesis. The resulting mutagenized cassettes are re-introduced into the context of the otherwise wild type allele. Cassette mutagenesis is useful when there is experimental evidence available to suggest a particular function for a region of a molecule, and there is a selection and/or screening approach available to discriminate between interesting and uninteresting mutants. Cassette mutagenesis is also useful when the parent molecule is comparatively large and the desire is to map the functional domains of a molecule by mutagenizing the molecule in a step-wise fashion, i.e., mutating one linear cassette of residues at a time and then assaying for function.

Mutagenesis of tTA or rtTA encoding sequences facilitates the identification of transcriptional regulators that interact differentially with different effector molecules. For example, mutagenesis can be restricted to portions of the sequence responsible for forming the effector binding pocket. Such properties can be exploited to control different genes via specific sets of transcriptional regulators and effectors (see Baron et al., 1999). Modification of the effector binding pocket is most likely a prerequisite for the detection of tetracyclines that are not deposited in bone tissue. For gene therapy, it will be advantageous to use transcriptional regulators that are insensitive toward tetracyclines used in human medicine.

In one embodiment, a mutated rtTA protein has altered basal transcriptional activity in the absence of a tetracycline, or an analog thereof. In a preferred embodiment, a rtTA protein has at least one changed amino acid within the DNA binding domain. In a preferred embodiment, the mutation is selected from the group comprising: S 12G, E19G, and T26A. In another embodiment, a mutation within the DNA binding domain confers increased or decreased basal affinity for the tet operator in the absence of a tetracycline, or an analog thereof

In another embodiment, the mutated rtTA protein has increased or decreased induced transcriptional activity in the presence of a tetracycline, or an analog thereof. In a preferred embodiment, a rtTA protein of the invention has at least one amino acid mutation within the tetracycline binding domain. In a preferred embodiment, the mutation is selected from the group comprising: A56P, R87S, deletion C88, D95G, G96R, V99E, D148E, H179R, and E204K. In another embodiment, a mutation within the tetracycline binding domain confers increased or decreased sensitivity towards doxycycline, or an analog thereof.

In another aspect of the invention a transactivator fusion protein of the invention is a sequence variant of a tTA protein. A sequence variant of a tTA protein will contain at least one mutation that confers a novel phenotype upon the protein.

In one embodiment, the mutated tTA protein displays differential induction by tetracycline, and analogs thereof In a preferred embodiment, a tTA protein of the invention has at least one amino acid mutation within the tetracycline binding domain. In a preferred embodiment the mutation is selected from the group comprising: A56V, F78S, S85G, S85R, Y110C, L113H, Y132C, I164L, P167S, L170V, 1174V, I174T, or E183K. In another embodiment, a mutation within the tetracycline binding domain confers either increased or decreased sensitivity towards tetracycline, or an analog thereof.

Exemplary mutations are taught, for example in the patent application published as US20030208783. Other amino acid substitutions, deletions or additions at these or other amino acid positions which retain the desired functional properties of the mutated tTA or rtTA protein are within the scope of the invention.

### V. Expression of Nucleic Acid Molecules

### A. Expression Vectors

A nucleic acid molecule of the invention may encode a transcriptional regulator fusion protein and/or a target nucleic acid sequence operatively linked to a TRE, as described above, and can be incorporated into one or more recombinant expression vector(s) in a form suitable for expression of the fusion protein in a host cell using methods known in the art.

When used in mammalian cells, a recombinant expression vector's control functions are often provided by viral genetic material. For example, commonly used promoters are derived from polyoma, Adenovirus 2, cytomegalovirus and Simian Virus 40. Use of viral regulatory elements to direct expression of the fusion protein can allow for high level constitutive expression of the fusion protein in a variety of host cells. In a preferred recombinant expression vector, the sequences encoding the fusion protein are flanked upstream (i.e., 5') by the human cytomegalovirus IE promoter and downstream (i.e., 3') by an SV40 poly(A) signal. The human cytomegalovirus IE promoter is described in Boshart et al. (1985) Cell 41:521-530. Other ubiquitously expressing promoters which can be used include the HSV-Tk promoter (disclosed in McKnight et al. (1984) Cell 37:253-262) and β-actin promoters (e.g., the human β-actin promoter as described by Ng et al. (1985) Mol. Cell. Biol. 5:2720-2732).

Alternatively, the regulatory sequences of the recombinant expression vector can direct expression of the fusion protein preferentially in a particular cell type, i.e., tissue-specific regulatory elements can be used. Non-limiting examples of tissue-specific promoters which can be used include the albumin promoter (liver-specific; Pinkert et al. (1987) Genes Dev. 1:268-277), lymphoid-specific promoters (Calame and Eaton (1988) Adv. Immunol. 43:235-275), in particular promoters of T cell receptors (Winoto and Baltimore (1989) EMBO J. 8:729-733) and immunoglobulins (Baneiji et al. (1983) Cell 33:729-740; Queen and Baltimore (1983) Cell 33:741-748), neuron-specific promoters. (e.g., the neurofilament promoter, Byrne and Ruddle (1989) Proc. Natl. Acad. Sci. USA 8:5473-5477), pancreas-specific promoters (Edlund et al. (1985) Science 230:912-916), and mammary gland-specific promoters (e.g., milk whey promoter; U.S. Pat. No. 4,873,316 and European Application Publication No. 264,166). Developmentally-regulated promoters are also encompassed, for example the murine hox promoters (Kessel and Gruss (1990) Science 249:374-379) and the α-fetoprotein promoter (Campes and Tilghman (1989) Genes Dev. 3:537-546).

Alternatively, a self-regulating construct encoding a transcriptional regulator fusion protein can be created. To accomplish this, nucleic acid encoding the fusion protein is operatively linked to a minimal promoter sequence and at least one tet operator sequence. When such a nucleic acid is introduced into a cell (e.g., in a recombinant expression vector), a small amount of basal transcription of the transcriptional regulator gene is likely to occur due to "leakiness". In the presence of a substituted tetracycline compound, this small amount of the transcriptional regulator fusion protein will bind to the tet operator sequence(s) upstream of the nucleotide sequence encoding the transcriptional regulator and stimulate additional transcription of the nucleotide sequence encoding the transcriptional regulator, thereby leading to further production of the transcriptional regulator fusion protein in the cell. It will be appreciated by those skilled in the art that such a self-regulating promoter can also be used in conjunction with other tetracycline-regulated transcriptional regulators, such as the wild-type Tet repressor fusion protein (tTA) described in Gossen, M. and Bujard, H. (1992) Proc. Natl. Acad. Sci. USA 89:5547-5551, which binds to tet operators in the absence of tetracycline. When used in conjunction with this transactivator, self-regulated transcription of the nucleotide sequence encoding this transactivator is stimulated in the absence of substituted tetracycline compounds of the invention.

The recombinant expression vector of the invention can be a plasmid, such as that described in US 5,789,156. Alternatively, a recombinant expression vector of the invention can be a virus, or portion thereof, which allows for expression of a nucleic acid introduced into the viral nucleic acid. For example, replication defective retroviruses, adenoviruses and adeno-associated viruses can be used. Protocols for producing recombinant retroviruses and for infecting cells *in vitro* or *in vivo* with such viruses can be found in Current Protocols in Molecular Biology, Ausubel, F. M et al. (eds.) Greene Publishing Associates, (1989), Sections 9.10-9.14 and other standard laboratory manuals. Examples of suitable retroviruses include pLJ, pZIP, pWE and pEM which are well known to those skilled in the art. Examples of suitable packaging virus lines include ψCrip, ψCre, ψ2 and ψAm. The genome of adenovirus can be manipulated such that it encodes and expresses a transcriptional regulator fusion protein but is inactivated in terms of its ability to replicate in a normal lytic viral life cycle. See for example Berkner et al. (1988) BioTechniques 6:616; Rosenfeld et al. (1991) Science 252:431-434; and Rosenfeld et al. (1992) Cell 68:143-155. Suitable adenoviral vectors derived from the adenovirus strain Ad type 5 d1324 or other strains of adenovirus (e.g., Ad2, Ad3, Ad7 etc.) are well known to those skilled in the art. Alternatively, an adeno-associated virus vector such as that described in Tratschin et al. (1985) Mol. Cell. Biol. 5:3251-3260 can be used to express a transcriptional regulator fusion protein.

### B. Host Cells

Tetracycline compounds may be used to regulate transcription in cell or in organisms. In one embodiment, the cell is a eukaryotic cell. In another embodiment, the cell is a mammalian cell. The methods of the invention are broadly applicable and encompass non-mammalian eukaryotic cells and non-eukaryotic cells as well. Some examples include: bacteria, insect (eg., Sp. frugiperda), yeast (e.g., S. cerevisiae, S. pombe, P. pastoris, K. lactis, H. polymorpha; as generally reviewed by Fleer, R (1992) Current Opinion in Biotechnology 3(5):486-496)), fungal and plant cells. Examples of vectors for expression in yeast S. cerivisae include pYepSec1 (Baldari. e al., (1987) Embo J. 6:229-234), pMFa (Kujan and Herskowitz, (1982) Cell 30:933-943), pJRY88 (Schultz et al., (1987) Gene 54:113-123), and pYES2 (Invitrogen Corporation, San Diego, Calif.). The fusion protein can be expressed in insect cells using baculovirus expression vectors (e.g., as described in O'Reilly et al. (1992) Baculovirus Expression Vectors: A Laboratory Manual, Stockton Press). Baculovirus vectors available for expression of proteins in cultured insect cells (e.g., SF 9 cells) include the pAc series (Smith et al., (1983) Mol. Cell Biol. 3:2156-2165) and the pVL series (Lucklow, V. A., and Summers, M. D., (1989) Virology 170:31-39).

In one embodiment, a fusion protein of the methods of the invention is expressed in a cell by introducing nucleic acid encoding the fusion protein into a host cell, wherein the nucleic acid is in a form suitable for expression of the fusion protein in the host cell. For example, a recombinant expression vector of the methods of the invention, encoding the fusion protein, is introduced into a host cell. Alternatively, nucleic acid encoding the fusion protein which is operatively linked to regulatory sequences (e.g., promoter sequences) but without additional vector sequences can be introduced into a host cell.

In addition to cell lines, the methods of the invention are applicable to normal cells, such as cells to be modified for gene therapy purposes or embryonic cells modified to create a transgenic or homologous recombinant animal. Examples of cell types of particular interest for gene therapy purposes include hematopoietic stem cells, myoblasts, hepatocytes, lymphocytes, neuronal cells and skin epithelium and airway epithelium. Additionally, for transgenic or homologous recombinant animals, embryonic stem cells and fertilized oocytes can be modified to contain nucleic acid encoding a transcriptional regulator fusion protein. Moreover, plant cells can be modified to create transgenic plants.

### C Introduction of Nucleic Acid Molecules into a Host Cell

Nucleic acid molecules encoding fusion proteins can be introduced into prokaryotic or eukaryotic cells via conventional transformation or transfection techniques. The terms "transformation" and "transfection" include a variety of art-recognized techniques for introducing foreign nucleic acid (*e.g.,* DNA) into a host cell, including calcium phosphate or calcium chloride co-precipitation, DEAE-dextran-mediated transfection, lipofection, or electroporation. Suitable methods for transforming or transfecting host cells can be found in Sambrook, et al. (Molecular Cloning: A Larboratory Manual. 2nd, ed, Cold Spring Harbor Laboratory, Cold Spring Harbor Laboratory Press, Cold Spring Harbor, NY, 1989), and other laboratory manuals.

The number of host cells transformed with a nucleic acid of the methods of the invention will depend, at least in part, upon the type of recombinant expression vector used and the type of transfection technique used. Nucleic acid molecules can be introduced into a host cell transiently, or more typically, for long term regulation of gene expression, the nucleic acid is stably integrated into the genome of the host cell or remains as a stable episome in the host cell. Plasmid vectors introduced into mammalian cells are typically integrated into host cell DNA at only a low frequency. In order to identify these integrants, a gene that contains a selectable marker (e.g., drug resistance) is generally introduced into the host cells along with the nucleic acid of interest. Preferred selectable markers include those which confer resistance to certain drugs, such as G418 and hygromycin. Selectable markers can be introduced on a separate plasmid from the nucleic acid of interest or, are introduced on the same plasmid. Host cells transfected with a nucleic acid of the invention (e.g., a recombinant expression vector) and a gene for a selectable marker can be identified by selecting for cells using the selectable marker. For example, if the selectable marker encodes a gene conferring neomycin resistance, host cells which have taken up nucleic acid can be selected with G418. Cells that have incorporated the selectable marker gene will survive, while the other cells die.

A host cell transfected with a nucleic acid encoding a fusion protein of the invention can be further transfected with one or more nucleic acids which serve as the target for the fusion protein. The target nucleic acid comprises a nucleotide sequence to be transcribed operatively linked to at least one tet operator sequence.

Nucleic acid molecules can be introduced into eukaryotic cells growing in culture *in vitro* by conventional transfection techniques (e.g., calcium phosphate precipitation, DEAE-dextran transfection, electroporation etc.). Nucleic acid molecules can also be transferred into cells *in vivo,* for example by application of a delivery mechanism suitable for introduction of nucleic acid into cells *in vivo,* such as retroviral vectors (see e.g., Ferry, N et al. (1991) Proc. Natl: Acad. Sci. USA 88:8377-8381; and Kay, M. A. et al. (1992) Human Gene Therapy 3:641-647), adenoviral vectors (see e.g., Rosenfeld, M. A. (1992) Cell 68:143-155; and Herz, J. and Gerard, R.D. (1993) Proc. Natl. Acad. Sci USA 90:2812-2816), receptor-mediated DNA uptake (see e.g., Wu, G. and Wu, C. H. (1988) J. Biol. Chem. 263:14621; Wilson et al. (1992) J. Biol. Chem. 26.963-967; and U.S. Pat. No. 5,166,320), direct injection of DNA (see e.g., Acsadi et al. (1991) Nature 332: 815-818; and Wolffet al. (1990) Science 247:1465-1468) or particle bombardment (see e.g., Cheng, L. et al. (1993) Proc. Natl. Acad. Sci. USA 90:4455-4459; and Zelenin, A. V. et al. (1993) FEBS Letters 315:29-32). Thus, for gene therapy purposes, cells can be modified *in vitro* and administered to a subject or, alternatively, cells can be directly modified *in vivo.*

### D. Transgenic Organisms

Nucleic acid molecules encoding one or more fusion proteins of the invention can be transferred into a fertilized oocyte of a non-human animal to create a transgenic animal which expresses the fusion protein(s) of the invention in one or more cell types. A transgenic animal is an animal having cells that contain a transgene, wherein the transgene was introduced into the animal or an ancestor of the animal at a prenatal, e.g., an embryonic, stage. A transgene is a DNA which is integrated into the genome of a cell from which a transgenic animal develops and which remains in the genome of the mature animal, thereby directing the expression of an encoded gene product in one or more cell types or tissues of the transgenic animal. In one embodiment, the non-human animal is a mouse, although the invention is not limited thereto. In other embodiments, the transgenic animal is a goat, sheep, pig, cow or other domestic farm animal. Such transgenic animals are useful for large scale production of proteins (so called "gene pharming").

A transgenic animal can be created, for example, by introducing a nucleic acid encoding the fusion protein (typically linked to appropriate regulatory elements, such as a constitutive or tissue-specific enhancer) into the male pronuclei of a fertilized oocyte, e.g., by microinjection, and allowing the oocyte to develop in a pseudopregnant female foster animal. Intronic sequences and polyadenylation signals can also be included in the transgene to increase the efficiency of expression of the transgene. Methods for generating transgenic animals, particularly animals such as mice, have become conventional in the art and are described, for example, in U.S. Pat. Nos. 4,736,866 and 4,870,009 and Hogan, B. et al., (1986) A Laboratory Manual, Cold Spring Harbor, N.Y., Cold Spring Harbor Laboratory. A transgenic founder animal can be used to breed additional animals carrying the transgene. Transgenic animals carrying a transgene encoding the fusion protein of the invention can further be bred to other transgenic animals carrying other transgenes, e.g., to a transgenic animal which contains a gene operatively linked to a tet operator sequence.

It will be appreciated that, in addition to transgenic animals, the regulatory system described herein can be applied to other transgenic organisms, such as transgenic plants. Transgenic plants can be made by conventional techniques known in the art. Accordingly, the invention encompasses non-human transgenic organisms, including animals and plants, that contains cells which express the transcriptional regulator fusion protein of the invention (i.e., a nucleic acid encoding the transcriptional regulator is incorporated into one or more chromosomes in cells of the transgenic organism).

### E. Homologous Recombinant Organisms

The methods of the invention also feature a homologous recombinant non-human organism expressing the fusion protein of the invention, to which substituted tetracycline compounds may be administered. The term "homologous recombinant organism" includes organisms, e.g. animal or plant, containing a gene which has been modified by homologous recombination between the gene and a DNA molecule introduced into a cell of the animal, e.g., an embryonic cell of the animal. In one embodiment, the non-human animal is a mouse, although the invention is not limited thereto. An animal can be created in which nucleic acid encoding the fusion protein has been introduced into a specific site of the genome, i.e., the nucleic acid has homologously recombined with an endogenous gene.

To create such a homologous recombinant animal, a vector is prepared which contains DNA encoding the fusion protein flanked at its 5' and 3' ends by additional nucleic acid of a eukaryotic gene at which homologous recombination is to occur. The additional nucleic acid flanking that encoding the fusion protein is of sufficient length for successful homologous recombination with the eukaryotic gene. Typically, several kilobases of flanking DNA (both at the 5' and 3' ends) are included in the vector (see e.g., Thomas, K. R. and Capecchi, M. R. (1987) Cell 51:503 for a description of homologous recombination vectors). The vector is introduced into an embryonic stem cell line (e.g., by electroporation) and cells in which the introduced DNA has homologously recombined with the endogenous DNA are selected (see e.g., Li, E. et al.. (1992) Cell 6:915). The selected cells are then injected into a blastocyst of an animal (e.g., a mouse) to form aggregation chimeras (see e.g., Bradley, A. in Teratocarcinomas and Embryonic Stem Cells: A Practical Approach, E. J. Robertson, ed. (IRL, Oxford, 1987) pp. 113-152). A chimeric embryo can then be implanted into a suitable pseudopregnant female foster animal and the embryo brought to term. Progeny harbouring the homologously recombined DNA in their germ cells can be used to breed animals in which all cells of the animal contain the homologously recombined DNA. These "germline transmission" animals can further be mated to animals carrying a gene operatively linked to at least one tet operator sequence.

In addition to the homologous recombination approaches described above, enzyme-assisted site-specific integration systems are known in the art and can be applied to the components of the regulatory system of the methods of the invention to integrate a DNA molecule at a predetermined location in a second target DNA molecule. Examples of such enzyme-assisted integration systems include the Cre recombinase-lox target system (e.g., as described in Baubonis, W. and Sauer, B. (1993) Nucl. Acids Res. 21:2025-2029; and Fukushige, S. and Sauer, B. (1992) Proc. Natl. Acad. Sci. USA 89:7905-7909) and the FLP recombinase-FRT target system (e.g., as described in Dang, D. T. and Perrimon, N. (1992) Dev. Genet. 13:367-375; and Fiering, S. et al. (1993) Proc. Natl. Acad. Sci. USA 9:8469-8473).

### VI. Regulation of Expression of Target Nucleotide Sequences

Expression of target nucleotide sequences is regulated by transcriptional regulator proteins such as those described above. Thus, the fusion protein and the target nucleic acid molecule are both present in a host cell or organism. The presence of both the transcriptional regulator fusion protein and the target transcription unit in the same host cell or organism can be achieved in a number of different ways. For example, one nucleic acid of the expression system (e.g., encoding the transcriptional regulator fusion protein) can be introduced into a host cell and then the other nucleic acid molecule can be introduced into the same host cell. Two distinct selectable markers can be used for selection, e.g., uptake of the first nucleic acid can be selected with G418 and uptake of the second nucleic acid can be selected with hygromycin. Alternatively, a single population of cells can be transfected with nucleic acid corresponding to both components of the system.

Accordingly, the methods of the invention provide a nucleic acid composition comprising:
- a first nucleic acid encoding a fusion protein which modulates transcription, the fusion protein comprising a first polypeptide which binds to a tet operator sequence in the presence or absence of a substituted tetracycline compound operatively linked to a second polypeptide which activates transcription in eukaryotic cells; and
- a second nucleic acid comprising a nucleotide sequence to be transcribed operatively linked to at least one tet operator sequence.

The two nucleic acids may exist on two separate molecules (e.g., two different vectors). In this case, a host cell is cotransfected with the two nucleic acid molecules or successively transfected first with one nucleic acid molecule and then the other nucleic acid molecule. In another embodiment, the two nucleic acids are linked (i.e., colinear) in the same molecule (e.g., a single vector). In this case a host cell is transfected with the single nucleic acid molecule.

The host cell may be a cell cultured *in vitro* or a cell present *in vivo* (e.g., a cell targeted for gene therapy). The host cell can further be a fertilized oocyte, embryonic stem cell or any other embryonic cell used in the creation of non-human transgenic or homologous recombinant animals. Transgenic or homologous recombinant animals which comprise both nucleic acid components of the expression system can be created by introducing both nucleic acids into the same cells at an embryonic stage, or more preferably, an animal which carries one nucleic acid component of the system in its genome is mated to an animal which carries the other nucleic acid component of the system in its genome. Offspring which have inherited both nucleic acid components can then be identified by standard techniques.

### A. Coordinate Regulation of Expression of Two Nucleotide Sequences

In addition to providing a system for the regulated expression of a single transcribed nucleotide sequence, the methods of the invention further permit coordinated regulation of the expression of two nucleotide sequences operatively linked to the same tet operator sequence(s). Accordingly, the methods of the invention may also pertain to a novel tet-regulated transcription unit for coordinate regulation of two genes. In this transcription unit, the same tet operator sequence(s) regulates the expression of two operatively linked nucleotide sequences that are transcribed in opposite directions from the common tet operator sequence(s). Accordingly, one nucleotide sequence is operatively linked to one side of the tet operator sequence (e.g., the 5' end on the top strand of DNA) and the other nucleotide sequence is operatively linked to the opposite side of the tet operator sequence (e.g., the 3' end on the top strand of DNA). Additionally, it should be understood that each nucleotide sequence to be transcribed includes an operatively linked minimal promoter sequence which is located between the nucleotide sequence to be transcribed and the tet operator sequence(s).

A representative example of such a transcription unit is diagrammed schematically in Figure 6 of US 5,789,176. In this vector, the two nucleotide sequences, operatively linked to the same tet operator sequence(s), are transcribed in opposite directions relative to the tet operator sequence(s) (i.e., the sequences are transcribed in a divergent manner upon activation by a transactivator fusion protein of the invention). By "transcribed in opposite directions relative to the tet operator sequence(s)", it is meant that the first nucleotide sequence is transcribed 5' to 3' from one strand of the DNA (e.g., the bottom strand) and the second nucleotide sequence is transcribed 5' to 3' from the other stand of the DNA (e.g., the top strand), resulting in bidirectional transcription away from the tet operator sequence(s).

Accordingly, the methods of the invention may feature a recombinant vector for coordinately-regulated, bidirectional transcription of two nucleotide sequence. The vector may comprise a nucleotide sequence linked by phosphodiester bonds comprising, in a 5' to 3' direction: a first nucleotide sequence to be transcribed, operatively linked to at least one tet operator sequence, operatively linked to a second nucleotide sequence to be transcribed, wherein transcription of the first and second nucleotide sequences proceeds in opposite directions from the at least one tet operator sequence(s) (i.e., the first and second nucleotide sequences are transcribed in a divergent manner).

The vector may also not include the first and second nucleotide sequence to be transcribed but instead may contain cloning sites which allow for the introduction into the vector of nucleotide sequences of interest. Accordingly, the vector may comprise a nucleotide sequence comprising in a 5' to 3' direction: a first cloning site for introduction of a first nucleotide sequence to be transcribed, operatively linked to at least one tet operator sequence, operatively linked to a second cloning site for introduction of a second nucleotide sequence to be transcribed, wherein transcription of a first and second nucleotide sequence introduced into the vector proceeds in opposite directions from the at least one tet operator sequence(s). It will be appreciated by those skilled in the art that this type of "cloning vector" may be in a form which also includes minimal promoter sequences such that a first nucleotide sequence introduced into the first cloning site is operatively linked to a first minimal promoter and a second nucleotide sequence introduced into the second cloning site is operatively linked to a second minimal promoter. Alternatively, the "cloning vector" may be in a form which does not include minimal promoter sequences and instead, nucleotide sequences including linked minimal promoter sequences are introduced into the cloning sites of the vector.

The term "cloning site" includes at least one restriction endonuclease site. Typically, multiple different restriction endonuclease sites (e.g., a polylinker) are contained within the nucleic acid.

The vector for coordinate, bidirectional transcription of two nucleotide sequences may also contain a first nucleotide to be transcribed, such as that encoding a detectable marker (e.g., luciferase or β-galactosidase), and a cloning site for introduction of a second nucleotide sequence of interest.

Suitable examples of bidirectional promoter regions for use in a vector for coordinate regulation of two nucleotide sequences to be transcribed are described in US 5,789,156.

### B Independent Regulation of Expression of Multiple Nucleotide Sequences

The methods of the invention still further permit independent and opposite regulation of two or more nucleotide sequences to be transcribed. Accordingly, a tet-regulated transcription unit for independent regulation of two or more genes may be used. To independently regulate the expression of two nucleotide sequences to be transcribed, one nucleotide sequence may be operatively linked to a tet operator sequence(s) of one class type while the other nucleotide sequence is operatively linked to a tet operator sequence(s) of another class type.

Accordingly, a vector for independent regulation of transcription of two nucleotide sequences may be used. Such vector(s) may comprise: a first nucleotide sequence to be a transcribed operatively linked to at least one tet operator sequence of a first class type; and a second nucleotide sequence to be a transcribed operatively linked to at least one tet operator sequence of a second class type. The two independently regulated transcription units can be included on a single vector, or alternatively, on two separate vectors. The recombinant vector(s) containing the nucleotide sequences to be transcribed can be introduced into a host cell or animal as described previously.

The vector(s) may also not include the first and second nucleotide sequence to be transcribed but instead contain cloning sites which allow for the introduction into the vector of nucleotide sequences of interest. Accordingly, the vector(s) may comprise:
- a first cloning site for introduction of a first nucleotide sequence to be transcribed operatively linked to at least one tet operator sequence of a first class type; and
- a second cloning site for introduction of a second nucleotide sequence to be transcribed operatively linked to at least one tet operator sequence of a second class type.

This cloning vector(s) may be in a form that already includes first and second minimal promoters operatively linked, respectively, to the first and second cloning sites: Alternatively, nucleotide sequences to be transcribed which include an operatively linked minimal promoter can be introduced into the cloning vector.

The vector for independent regulation of two nucleotide sequences may also contain a first nucleotide to be transcribed, such as that encoding a detectable marker or a suicide gene, operatively linked to at least one tet operator sequence of a first class type and a cloning site for introduction of a second nucleotide sequence of interest such that it is operatively linked to at least one tet operator sequence of a second class type.

It will be appreciated by those skilled in the art that various combinations of classes of tet operator sequences can be used for independent regulation of two nucleotide sequences. For example, the first tet operator sequence(s) can be of the class A type and the second can be of the class B type, or the first tet operator sequence can be of the class B type and the second can be of the class C type, etc. Preferably, one to the two tet operators used is a class B type operator.

Independent transcription of the first and second nucleotide sequences is regulated in a host cell by further introducing into the host cell one or more nucleic acids encoding two different transcriptional regulator fusion proteins which bind independently to tet operator sequences of different class types. The first fusion protein comprises a polypeptide which binds to a tet operator sequence in the presence of tetracycline or a tetracycline analogue, operatively linked to a polypeptide which activates transcription in eukaryotic cells (e.g., a transactivator fusion protein of the invention, such as a mutated Tn10-derived Tet repressor linked to a VP16 activation region). The second fusion protein comprises a polypeptide which binds to a tet operator sequence in the absence of tetracycline or a tetracycline analogue, operatively linked to a polypeptide which activates transcription in eukaryotic cells (e.g., a wild-type Tn10-derived Tet repressor linked to a VP16 activation region, such as the tTA described in Gossen, M. and Bujard, H. (1992) Proc. Natl. Acad. Sci. USA 8:5547-5551). The first fusion protein may bind to the tet operator sequence of the first class type used in the transcription unit and the second fusion protein may bind to the tet operator sequence of the second class type used in the transcription unit. Alternatively, in another embodiment, the first fusion protein binds to the second class type of tet operator and the second fusion protein binds to the first class type of tet operator.

For example, the first nucleotide sequence to be transcribed may be linked to a class A tet operator and the first fusion protein may bind to class A operators, whereas the second nucleotide sequence to be transcribed may be linked to a class B tet operator and the second fusion protein may bind to class B operators. Thus, in this embodiment, transcription of the first nucleotide sequence is activated in the presence of tetracycline (or analogue thereof) while transcription of the second nucleotide sequence is activated in the absence of tetracycline (or analogue thereof). Alternatively, in another embodiment, the first fusion protein binds to class B operators and the second fusion protein binds to class A operators. In this case, transcription of the second nucleotide sequence is activated in the presence of tetracycline (or analogue thereof) while transcription of the first nucleotide sequence is activated in the absence of tetracycline (or analogue thereof). Appropriate transactivator proteins for use in this system can be designed as is known in the art, e.g., as in Gossen and Bujard (1992) cited herein. In order to inhibit heterodimerization between the two different types of Tet repressor fusion proteins present in the same cell, it may be necessary to mutate the dimerization region of one or both of the transcriptional regulator fusion proteins. Mutations can be targeted to the C-terminal region of TetR known to be involved in dimerization. The dimerization region has been described in detail based upon the crystal structure of TetR (see Hinrichs, W. et al. (1994) Science 264:418-420).

This system allows for independent and opposite regulation of the expression of two genes by substituted tetracycline compounds. Use of different substituted tetracycline compounds as inducing agents may further allow for high, low or intermediate levels of expression of the different sequences. The transcription unit of the methods of the invention for independently regulating the expression of two genes, described above, can be used in situations where two gene products are to be expressed in the same cell but where it is desirable to express one gene product while expression of the other gene product is turned "off", and vice versa. For example, this system is particularly useful for expressing in the same host cell either a therapeutic gene or a suicide gene (i.e., a gene which encodes a product that can be used to destroy the cell, such as ricin or herpes simplex virus thymidine kinase). In many gene therapy situations, it is desirable to be able to express a gene for therapeutic purposes in a host cell but also to have the capacity to destroy the host cell once the therapy is completed. This can be accomplished using the above-described system by linking the therapeutic gene to one class of tet operator and the suicide gene to another class of tet operator. Thus, expression of the therapeutic gene in a host cell can be stimulated by a substituted tetracycline compound (in which case expression of the suicide gene is absent). Then, once the therapy is complete, the substituted tetracycline compound is removed, which turns off expression of the therapeutic gene and turns on expression of the suicide gene in the cell.

### C Combined Coordinate and Independent Regulation of Multiple Nucleotide Sequences

It is further possible to regulate the expression of four nucleotide sequences by combining the system described in two of the systems described above such that two pairs of sequences are coordinately regulated while one pair is independently regulated from the other pair. Accordingly, two target transcription units can be designed comprising:
- a first nucleic acid comprising in a 5' to 3' direction: a first nucleotide sequence to be transcribed, a tet operator sequence(s) of a first class type, and a second nucleotide sequence to be transcribed
- a second nucleic acid comprising in a 5' to 3' direction: a third nucleotide sequence to be transcribed, a tet operator sequence(s) of a second class type, and a fourth nucleotide sequence to be transcribed.

Transcription of the first and second nucleotide sequences in the first nucleic acid proceeds in a divergent manner from the first class of tet operator sequence(s). Likewise, transcription of the third and fourth nucleotide sequences in the second nucleic acid proceeds in a divergent manner from the second class of tet operator sequence(s). Thus, expression of the first and second nucleotide sequences is coordinately regulated and expression of the third and fourth nucleotide sequences is coordinately regulated. However, expression of the first and second sequences is independently (and oppositely) regulated compared to the third and fourth sequences through the use of two different transactivator fusion proteins, as described above, one which activates transcription in the presence of a substituted tetracycline compound and the other which activates transcription in the absence of a substituted tetracycline compound. One transactivator is designed to bind to a tet operators of the first class type and the other is designed to bind to a tet operators of the second class type. In other embodiments, rather than already containing first, second, third and/or fourth nucleotide sequences to be transcribed, these transcription units can contain cloning sites which allow for the introduction of first, second, third and/or fourth nucleotide sequences to be transcribed.

### VII. Kits of the Invention

Another aspect of the invention pertains to kits which include the components of the inducible regulatory system of the invention. Such a kit can be used to regulate the expression of a gene of interest (i.e., a nucleotide sequence of interest to be transcribed) which can be cloned into a target transcription unit. The kit may include nucleic acid encoding a transcriptional activator fusion protein or a transcriptional silencer fusion protein or both. Alternatively, eukaryotic cells which have nucleic acid encoding a transactivator and/or inhibitor fusion protein stably incorporated therein, such that the transactivator and/or inhibitor fusion protein are expressed in the eukaryotic cell, may be provided in the kit.

In one embodiment, the kit includes a carrier means having in close confinement therein at least two container means: a first container means which contains a first nucleic acid (e.g., DNA) encoding a transcriptional regulator fusion protein of the invention (e.g., a recombinant expression vector encoding a first polypeptide which binds to a tet operator sequence in the presence of tetracycline operatively linked to a second polypeptide which activates transcription in eukaryotic cells), and a second container means which contains a second target nucleic acid (e.g., DNA) for the transcriptional regulator into which a nucleotide sequence of interest can be cloned. The second nucleic acid typically comprises a cloning site for introduction of a nucleotide sequence to be transcribed (optionally including an operatively linked minimal promoter sequence) and at least one operatively linked tet operator sequence. The term "cloning site" is intended to encompass at least one restriction endonuclease site. Typically, multiple different restriction endonuclease sites (e.g., a polylinker) are contained within the nucleic acid.

To regulate expression of a nucleotide sequence of interest using the components of the kit, the nucleotide sequence is cloned into the cloning site of the target vector of the kit by conventional recombinant DNA techniques and then the first and second nucleic acids are introduced into a host cell or animal. The transcriptional regulator fusion protein expressed in the host cell or animal then regulates transcription of the nucleotide sequence of interest in the presence of the substited tetracycline compound.

Alternatively, in another embodiment, the kit includes a eukaryotic cell which is stably transfected with a nucleic acid encoding a transcriptional regulator fusion protein of the invention such that the transcriptional regulator is expressed in the cell. Thus, rather than containing nucleic acid alone, the first container means described above can contain a eukaryotic cell line into which the first nucleic acid encoding the transcriptional regulator has been stably introduced (e.g., by stable transfection by a conventional method such as calcium phosphate precipitation or electroporation, etc.). In this embodiment, a nucleotide sequence of interest is cloned into the cloning site of the target vector of the kit and then the target vector is introduced into the eukaryotic cell expressing the transcriptional regulator fusion protein.

Alternatively or additionally, a recombinant vector of the invention for coordinate regulation of expression of two nucleotide sequences can also be incorporated into a kit of the invention. The vector can be included in the kit in a form that allows for introduction into the vector of two nucleotide sequences of interest. Thus, in another embodiment, a kit of the invention includes 1) a first nucleic acid encoding a transcriptional regulator fusion protein of the invention (or a eukaryotic cell into which the nucleic acid has been stably introduced) and 2) a second nucleic acid comprising a nucleotide sequence comprising in a 5' to 3' direction: a first cloning site for introduction of a first nucleotide sequence of interest operatively linked to at least one tet operator sequence operatively linked to a second cloning site for introduction of a second nucleotide sequence of interest, wherein transcription of the first and second nucleotide sequences proceeds in opposite directions from the at least one tet operator sequence. Optionally, the vector can include operatively linked minimal promoter sequences. In another embodiment, the vector can be in a form that already contains one nucleotide sequence to be transcribed (e.g., encoding a detectable marker such as luciferase, β-galactosidase or CAT) and a cloning site for introduction of a second nucleotide sequence of interest to be transcribed.

The transcription units and transcriptional regulators of the invention for independent regulation of expression of two nucleotide sequences to be transcribed can also be incorporated into a kit of the invention. The target transcription units can be in a form which allows for introduction into the transcription units of nucleotide sequences of interest to be transcribed. Thus, in another embodiment, a kit of the invention includes 1) a first nucleic acid encoding a transcriptional regulator which binds to a tet operator of a first class type in the presence of a substituted tetracycline compound, 2) a second nucleic acid comprising a first cloning site for introduction of a first nucleotide sequence to be transcribed operatively linked to at least one tet operator of a first class type, 3) a third nucleic acid encoding a transcriptional regulator which binds to a tet operator of a second class type in the absence of a substituted tetracycline compound, and 4) a fourth nucleic acid comprising a second cloning site for introduction of a second nucleotide sequence to be transcribed operatively linked to at least one tet operator of a second class type. (Optionally, minimal promoter sequences are included in the second and fourth nucleic acids). In another embodiment, one nucleotide sequence to be transcribed (e.g., encoding a suicide gene) is already contained in either the second or the fourth nucleic acid. In yet another embodiment, the nucleic acids encoding the transcriptional regulators (e.g., the first and third nucleic acids described above) can be stably introduced into a eukaryotic cell line which is provided in the kit.

In yet another embodiment, a kit of the invention includes a first container means containing a first nucleic acid encoding a transcriptional silencer fusion protein of the invention (e.g., the fusion protein inhibits transcription in eukaryotic cells either only in the presence of tetracycline or only the absence of tetracycline) and a second container means containing a second nucleic acid comprising a cloning site for introduction of a nucleotide sequence to be transcribed operatively linked to at least one tet operator sequence. The kit may further include a third nucleic acid encoding a transactivator fusion protein that binds to tetO sequences either only in the presence of tetracycline or only in the absence of tetracycline. Alternatively, the first and/or third nucleic acids (i.e., encoding the inhibitor or transactivator fusion proteins) may be stably incorporated into a eukaryotic host cell which is provided in the kit.

In still another embodiment, a kit of the invention may include at least one substituted tetracycline compound. For example, the kit may include a container means which contains a substituted tetracycline compound described herein.

### VIII. Regulation of Gene Expression by Substituted Tetracycline Compounds

### A. Stimulation of Gene Expression by Transactivator Fusion Proteins

In a host cell which carries nucleic acid encoding a transactivator fusion protein of the invention and a nucleotide sequence operatively linked to the tet operator sequence(i.e., gene of interest to be transcribed), high level transcription of the nucleotide sequence operatively linked to the tet operator sequence(s) does not occur in the absence of the substituted tetracycline compounds of the invention. The level of basal transcription of the nucleotide sequence may vary depending upon the host cell and site of integration of the sequence, but is generally quite low or even undetectable in the absence of a substituted tetracycline compound of the invention. In order to induce transcription in a host cell, the host cell is contacted with the substituted tetracycline compounds of the invention. The substituted tetracycline compounds may be administered to a subject containing the cell.

To induce gene expression in a cell *in vitro,* the cell is contacted with the substituted tetracycline compound by culturing the cell in a medium containing the substituted tetracycline compound. When culturing cells *in vitro* in the presence of the substituted tetracycline compound, a preferred concentration range for the inducing agent is between about 10 and about 1000 ng/ml. The substituted tetracycline compound can be directly added to media in which cells are already being cultured, or more preferably for high levels of gene induction, cells are harvested from substituted tetracycline compound-free media and cultured in fresh media containing the desired substituted tetracycline compound.

To induce gene expression in vivo, cells within in a subject are contacted with the substituted tetracycline compound of the invention by administering the compound to the subject. In an exemplary embodiment, when the inducing agent is administered to a human or animal subject, the dosage is adjusted to preferably achieve a serum concentration between about 0.0005 and 1.0 µg/ml The substituted tetracycline compound can be administered to a subject by any means effective for achieving an *in vivo* concentration sufficient for gene induction. Examples of suitable modes of administration include oral administration (e.g., dissolving the inducing agent in the drinking water), slow release pellets and implantation of a diffusion pump. To administer the substituted tetracycline compounds of the invention to a transgenic plant, the inducing agent can be dissolved in water administered to the plant.

Featured in the methods of the invention are substituted tetracycline compounds that further enhance the precision with which such expression systems may be implemented. The ability to use different tetracycline analogues as inducing agents in this system allows for modulation of the level of expression of a tet operator-linked nucleotide sequence. As demonstrated in US 5,789,156, anhydrotetracycline and doxycycline have been found to be strong inducing agents. The increase in transcription of the target sequence is typically as high as 1000- to 2000-fold, and induction factors as high as 20,000 fold can be achieved. Tetracycline, chlorotetracycline and oxytetracycline have been found to be weaker inducing agents, i.e., in this case, the increase in transcription of a target sequence is in the range of about 10-fold. Thus, an appropriate substituted tetracycline compound is chosen as an inducing agent based upon the desired level of induction of gene expression. It is also possible to change the level of gene expression in a host cell or animal over time by changing the substituted tetracycline compound used as the inducing agent. For example, there may be situations where it is desirable to have a strong burst of gene expression initially and then have a sustained lower level of gene expression. Accordingly, a substituted tetracycline compound which stimulates a high levels of transcription can be used initially as the inducing agent and then the inducing agent can be switched to an analogue which stimulates a lower level of transcription. Moreover, when regulating the expression of multiple nucleotide sequences (e.g., when one sequence is regulated by a one of class tet operator sequence(s) and the other is regulated by another class of tet operator sequence(s), as described herein it may be possible to independently vary the level of expression of each sequence depending upon which transactivator fusion protein is used to regulate transcription and which substituted tetracycline compound is used as the inducing agent. Different transactivator fusion proteins are likely to exhibit different levels of responsiveness to substituted tetracycline compounds. The level of induction of gene expression by a particular combination of transactivator fusion protein and inducing agent (tetracycline or tetracycline analogue) can be determined by techniques described herein, (e.g., see Example 2). Additionally, the level of gene expression can be modulated by varying the concentration of the inducing agent. Thus, the expression system of the methods of the invention provides a mechanism not only for turning gene expression on or off, but also for "fine tuning" the level of gene expression at intermediate levels depending upon the type and concentration the substituted tetracycline comopound used.

### B. Inhibition of Gene Expression by Transcriptional silencer Fusion Proteins

The methods of the invention also feature inhibition of gene expression using transcriptional silencer fusion proteins. These methods can be used to down-regulate basal, constitutive or tissue-specific transcription of a tetO-linked gene of interest. For example, a gene of interest that is operatively linked to tetO sequences and additional positive regulatory elements (e.g., consitutive or tissue-specific enhancer sequences) will be transcribed in host cells at a level that is primarily determined by the strength of the positive regulatory elements in the host cell. Moreover, a gene of interest that is operatively linked to tetO sequences and only a minimal promoter sequence may exhibit varying degrees of basal level transcription depending on the host cell or tissue and/or the site of integration of the sequence. In a host cell containing such a target sequence and expressing an inhibitor fusion protein of the invention, transcription of the target sequence can be down regulated in a controlled manner by altering the concentration of the substituted tetracycline compound in contact with the host cell. For example, when the inhibitor fusion protein binds to tetO in the absence of a substituted tetracycline compound, the concentration of the substituted tetracycline compound in contact with the host cell is reduced to inhibit expression of the target nucleic acid sequence. Preferably, a host cell is cultured in the absence of substituted tetracycline compounds to keep target nucleic acid sequence expression repressed. Likewise, the substituted tetracycline compounds are not administered to a host organism to keep target nucleic acid sequence expression repressed. Alternatively, when the inhibitor fusion protein binds to tetO in the presence of a substituted tetracycline compound, the concentration of the substituted tetracycline compound in contact with the host cell is increased to inhibit expression of the target nucleic acid sequence. For example, the substituted tetracycline compound is added to the culture medium of a host cell or the substituted tetracycline compound is administered to a host organism to repress target nucleic acid sequence expression.

The inhibitor fusion proteins can inhibit a tetO-linked gene of interest in which the tetO sequences are positioned 5' of a minimal promoter sequence (e.g., substituted tetracycline compound-regulated transcription units as described). Furthermore, the inhibitor fusion protein may be used to inhibit expression of a gene of interest in which tetO-linked sequences are located 3' of the promoter sequence but 5; of the transcription start site. Still further, the inhibitor fusion protein may be used to inhibit expression of a gene of interest in which tetO-linked sequences are located 3' of the transcription start site.

### C Combined Positive and Negative Regulation of Gene Expression

In addition to regulating gene expression using either a transcriptional activator or inhibitor fusion protein alone, the two types of fusion proteins can be used in combination to allow for both positive and negative regulation of expression of one or more target nucleic acid sequences in a host cell. Thus, a transcriptional silencer protein that binds to tetO either (i) in the absence, but not the presence, of a substituted tetracycline compound, or (ii) in the presence, but not the absence, of a substituted tetracycline compound, can be used in combination with a transactivator protein that binds to tetO either (i) in the absence, but not the presence, of a substituted tetracycline compound, or (ii) in the presence, but not the absence, of a substituted tetracycline compound. Transactivator proteins that bind to tetO in the absence, but not the presence, of unsubstituted tetracycline (e.g., wild-type TetR-activator fusion proteins) are described in further detail in U.S. Ser. No. 08/076,726, U.S. Ser. No. 08/076,327 and U.S. Ser. No. 08/260,452. Transactivator fusion proteins that bind to tetO in the presence, but not the absence, of substituted tetracycline compounds (e.g., mutated TetR-activator fusion proteins) are known in the art.

As described herein, when more than one TetR fusion protein is expressed in a host cell or organism, additional steps may be taken to inhibit heterodimerization between the different TetR fusion proteins. For example, a transactivator composed of a TetR of one class may be used in combination with a transcriptional silencer composed of a TetR of a second, different class that does not heterodimerize with the first class of TetR Alternatively, amino acid residues of the TetR involved in dimerization may be mutated to inhibit heterodimerization. However, even if some heterodimerization between transactivator and inhibitor fusion proteins occurs in a host cell, sufficient amounts of homodimers should be produced to allow for efficient positive and negative regulation as described herein.

It will be appreciated by those skilled in the art that various combinations of activator and inhibitor proteins can be used to regulate a single tetO-linked gene of interest in both a positive and negative manner or to regulate multiple tetO-linked genes of interest in a coordinated manner or in an independent manner using the teachings described herein. The precise regulatory components utilized will depend upon the genes to be regulated and the type of regulation desired. Several non-limiting examples of how the transactivator and inhibitor fusion proteins may be used in combination are described further below. However, many other possible combinations will be evident to the skilled artisan in view of the teachings herein and are intended to be encompassed by the invention:

### 1X. Exemplary Applications of the Invention

The invention is widely applicable to a variety of art recognized situations where it is desirable to be able to turn gene expression on and off, or regulate the level of gene expression, in a rapid, efficient and controlled manner without causing pleiotropic effects or cytotoxicity. Thus, the system of the methods of the invention has widespread applicability to the study of cellular development and differentiation in eukaryotic cells, plants and animals. For example, expression of oncogenes can be regulated in a controlled manner in cells to study their function. Additionally, the system can be used to regulate the expression of site-specific recombinases, such as CRE or FLP, to thereby allow for irreversible modification of the genotype of a transgenic organism under controlled conditions at a particular stage of development. For example, drug resistance markers inserted into the genome of transgenic plants that allow for selection of a particular transgenic plant could be irreversibly removed via a substituted tetracycline compound-regulated site specific recombinase. Other applications of the regulatory system of the methods of the invention include:

### A. Gene Therapy

The methods of the invention may be used in gene therapy approaches, in treatments for either genetic or acquired diseases. The general approach of gene therapy involves the introduction of nucleic acid into cells such that one or more gene products encoded by the introduced genetic material are produced in the cells to restore or enhance a functional activity. For reviews on gene therapy approaches see Anderson, W. F. (1992) Science 256:808-813; Miller, A. D. (1992) Nature 357:455-460; Friedmann, T. (1989) Science 244:1275-1281; and Cournoyer, D., et al. (1990) Curr. Opin. Biotech. 1:196-208. However, current gene therapy vectors typically utilize constitutive regulatory elements which are responsive to endogenous transcriptions factors. These vector systems do not allow for the ability to modulate the level of gene expression in a subject. In contrast, the inducible regulatory system of the methods of the invention provides this ability.

The substituted tetracycline compound-controlled regulatory system of the invention has numerous advantageous properties that make it particularly suitable for application to gene therapy. For example, the system provides an "on"/"off' switch for gene expression that allows for regulated dosage of a gene product in a subject. There are several situations in which it may be desirable to be able to provide a gene product at specific levels and/or times in a regulated manner, rather than simply expressing the gene product constitutively at a set level. For example, a gene of interest can be switched "on" at fixed intervals (e.g., daily, alternate days, weekly, etc.) to provide the most effective level of a gene product of interest at the most effective time. The level of gene product produced in a subject can be monitored by standard methods (e.g., direct monitoring using an immunological assay such as ELISA or RIA or indirectly by monitoring of a laboratory parameter dependent upon the function of the gene product of interest, e. g., blood glucose levels and the like). This ability to turn "on" expression of a gene at discrete time intervals in a subject while also allowing for the gene to be kept "off" at other times avoids the need for continued administration of a gene product of interest at intermittent intervals. This approach avoids the need for repeated injections of a gene product, which may be painful and/or cause side effects and would likely require continuous visits to a physician. In contrast, the system of the invention avoids these drawbacks. Moreover, the ability to turn "on" expression of a gene at discrete time intervals in a subject allows for focused treatment of diseases which involve "flare ups" of activity (e.g., many autoimmune diseases) only at times when treatment is necessary during the acute phase when pain and symptoms are evident. At times when such diseases are in remission, the expression system can be kept in the "off" state.

Gene therapy applications that may particularly benefit from this ability to modulate gene expression during discrete time intervals include the following non-limiting examples:

Rheumatoid arthritis--genes which encode gene products that inhibit the production of inflammatory cytokines (e.g., TNF, IL-1 and IL-12) can be expressed in subjects. Examples of such inhibitors include soluble forms of a receptor for the cytokine. Additionally or alternatively, the cytokines IL-10 and/or IL-4 (which stimulate a protective Th2-type response) can be expressed. Moreover, a glucocorticomimetic receptor (GCMR) can be expressed.

Hypopituitarism--the gene for human growth hormone can be expressed in such subjects only in early childhood, when gene expression is necessary, until normal stature is achieved, at which time gene expression can be downregulated.

Wound healing/Tissue regeneration--Factors (e.g., growth factors, angiogenic factors, etc.) necessary for the healing process can be expressed only when needed and then downregulated.

Anti-Cancer Treatments--Expression of gene products useful in anti-cancer treatment can be limited to a therapeutic phase until retardation of tumor growth is achieved, at which time expression of the gene product can be downregulated. Possible systemic anti-cancer treatments include use of tumor infiltrating lymphocytes which express immunostimulatory molecules (e.g., IL-2, IL-12 and the like), angiogenesis inhibitors (PF4, IL-12, etc.), Herregulin, Leukoregulin (see PCT Publication No. WO 85/04662), and growth factors for bone marrow support therapy, such as G-CSF, GM-CSF and M-CSF. Regarding the latter, use of the regulatory system of the invention to express factors for bone marrow support therapy allows for simplified therapeutic switching at regular intervals from chemotherapy to bone marrow support therapy (similarly, such an approach can also be applied to AIDS treatment, e.g., simplified switching from anti-viral treatments to bone marrow support treatment). Furthermore, controlled local targeting of anti-cancer treatments are also possible. For example, expression of a suicide gene by a regulator of the invention, wherein the regulator itself is controlled by, for example, a tumor-specific promoter or a radiation-induced promoter.

The ability to express a suicide gene (e.g., an apoptosis gene, TK gene, etc) in a controlled manner using the regulatory system of the methods of the invention adds to the general safety and usefulness of the system. For example, at the end of a desired therapy, expression of a suicide gene can be triggered to eliminate cells carrying the gene therapy vector, such as cells in a bioinert implant, cells that have disseminated beyond the intended original location, etc. Moreover, if a transplant becomes tumorous or has side effects, the cells can be rapidly eliminated by induction of the suicide gene. The use of more than one substituted tetracycline compound -controlled "on"/"off' switch in one cell allows for completely independent regulation of a suicide gene compared to regulation of a gene of therapeutic interest (as described in detail herein).

### B. Production of Proteins in Vitro

Large scale production of a protein of interest can be accomplished using cultured cells in vitro which have been modified to contain 1) a nucleic acid encoding a transcriptional regulator fusion protein of the invention in a form suitable for expression of the transcriptional regulator in the cells and 2) a gene encoding the protein of interest operatively linked to a tet operator sequence(s). For example, mammalian, yeast or fungal cells can be modified to contain these nucleic acid components as described herein. The modified cells can then be cultured by standard fermentation techniques in the presence of a substituted tetracycline compound to induce expression of the gene and produce the protein of interest. Accordingly, the methods of the invention may be used to manipulate a production process for isolating a protein of interest. In the process, a host cell (e.g., a yeast or fungus), into which has been introduced both a nucleic acid encoding a transcriptional regulator fusion protein of the methods of the invention and a nucleic acid encoding the protein of the interest operatively linked to at least one tet operator sequence, is grown at production scale in a culture medium in the presence of a substituted tetracycline compound to stimulate transcription of the nucleotides sequence encoding the protein of interest (i.e., the nucleotide sequence operatively linked to the tet operator sequence(s)) and the protein of interest is isolated from harvested host cells or from the culture medium. Standard protein purification techniques can be used to isolate the protein of interest from the medium or from the harvested cells.

### C Production of Proteins in Vivo

The methods of the invention also may enhance large scale production of a protein of interest in animals, such as in transgenic farm animals. Advances in transgenic technology have made it possible to produce transgenic livestock, such as cattle, goats, pigs and sheep (reviewed in Wall, R. J. et al. (1992) J. Cell. Biochem. 49:113-120; and Clark, A. J. et al. (1987) Trends in Biotechnology 5:20-24). Accordingly, transgenic livestock carrying in their genome the components of the inducible regulatory system of the methods of the invention can be constructed, wherein a gene encoding a protein of interest is operatively linked to at least one tet operator sequence. Gene expression, and thus protein production, is induced by administering a substituted tetracycline compound to the transgenic animal. Protein production can be targeted to a particular tissue by linking the nucleic acid encoding the transcriptional regulator fusion protein to an appropriate tissue-specific regulatory element(s) which limits expression of the transcriptional regulator to certain cells. For example, a mammary gland-specific regulatory element, such as the milk whey promoter (U.S. Pat. No. 4,873,316 and European Application Publication No. 264,166), can be linked to the transcriptional regulator transgene to limit expression of the transcriptional regulator to mammary tissue. Thus, in the presence of a substituted tetracycline compound, the protein of interest will be produced in the mammary tissue of the transgenic animal. The protein can be designed to be secreted into the milk of the transgenic animal, and if desired, the protein can then be isolated from the milk.

### D. Imaging of Regulated Gene Expression in Vivo

The methods of the invention can be employed in combination with invasive or more preferably, non-invasive imaging techniques, to monitor regulated gene expression in cells, cell lines and/or living subjects. For example, both a reporter gene (e.g., luciferase, GFP, CAT, etc.) and a nucleotide sequence of interest may be placed under the control of a bidirectional tet operator (e.g., P_{tetbi}-1), thereby rendering expression of the reporter gene and nucleotide sequence of interest responsive to a substituted tetracycline compound-controlled transactivators (tTA or rtTA). Through use of such genetic constructs, transgenic animals and cell lines may be derived within which expression and/or activity of a reporter gene such as luciferase serves as an indirect, non-invasive marker of the expression of the tet operator-linked nucleotide sequence. Use of such methods for implementation of non-invasive imaging in living subjects is described in Hasan, MT et al. Genesis 29(3):116-22.

### E. Animal Models of Human Disease

The transcriptional activator and inhibitor proteins of the methods of the invention can be used alone or in combination to stimulate or inhibit expression of specific genes in animals to mimic the pathophysiology of human disease to thereby create animal models of human disease. For example, in a host animal, a gene of interest thought to be involved in a disease can be placed under the transcriptional control of one or more tet operator sequences (e.g., by homologous recombination, as described herein). Such an animal can be mated to a second animal carrying one or more transgenes for a transactivator fusion protein and/or an inhibitor fusion protein to create progeny that carry both a substituted tetracycline compound-regulated fusion protein(s) gene and a tet-regulated target sequence. Expression of the gene of interest in these progeny can be modulated using a substituted tetracycline compound. For example, expression of the gene of interest can be downmodulated using a transcriptional silencer fusion protein to examine the relationship between gene expression and the disease. Such an approach may be advantageous over gene "knock out" by homologous recombination to create animal models of disease, since the tet-regulated system described herein allows for control over both the levels of expression of the gene of interest and the timing of when gene expression is down- or up-regulated.

### F. Production of Stable Cell Lines, for Gene Cloning and Other Uses

The transcriptional silencer system used in the methods of the invention can keep gene expression "off" (i.e., expressed) to thereby allow production of stable cell lines that otherwise may not be produced. For example, stable cell lines carrying genes that are cytotoxic to the cells can be difficult or impossible to create due to "leakiness" in the expression of the toxic genes. By repressing gene expression of such toxic genes using the transcriptional silencer fusion proteins of the invention, stable cell lines carrying toxic genes may be created. Such stable cell lines can then be used to clone such toxic genes (e.g., inducing the expression of the toxic genes under controlled conditions using a substituted tetracycline compound). General methods for expression cloning of genes, to which the transcriptional silencer system of the invention can be applied, are known in the art (see e.g., Edwards, C. P. and Aruffo, A. (1993) Curr. Opin. Biotech. 4:558-563) Moreover, the transcriptional silencer system can be applied to inhibit basal expression of genes in other cells to create stable cell lines, such as in embryonic stem (ES) cells. Residual expression of certain genes introduced into ES stems may result in an inability to isolate stably transfected clones. Inhibition of transcription of such genes using the transcriptional silencer system described herein may be useful in overcoming this problem.

### G. Induction of Substituted Tetracycline Compound-Regulated Gene Expression in the Brain

Certain substituted tetracycline compounds of the methods of the invention improve upon use of, e.g., doxycycline as preferred agents with which to modulate the inducible regulatory system featured in the methods of the invention. Doxycycline has been described to show limited penetration of the blood-brain barrier, thus necessitating elevation of doxycycline to extremely high levels in the peripheral blood of a treated subject in order to elicit modulation of an inducible regulatory system in the brain (refer to Mansuy and Bujard Curr. Op. Neurobiol. 10:593-96, incorporated herein by reference). Through implementation of the methods of the present invention with certain of the substituted tetracycline compounds described herein, improved functionality of the featured inducible regulatory system can be achieved in the brain and spinal cord of a living subject.

### H. Expression of Inhibitory RNAs

In another embodiment, the invention relates to recombinant vectors for inducible and/or tissue specific expression of nucleic acid molecules, e.g., double-stranded RNA molecules, that interfere with the expression of a target gene using methods known in the art. In certain embodiments, the invention relates to the use of Tet (tetracycline)-responsive RNA Polymerase II (Pol II) promoters (e.g., TetON or TetOFF) to direct inducible knockdown in certain cells of an integrated or an endogenous gene. The invention also relates to a method for producing transgenic animals (e.g., mice) expressing tetracycline-regulated reversible, and/or tissue-specific nucleic acid molecules, e.g., double-stranded RNA molecules that interfere with the expression of a target gene.

### X. Advantages

- The methods described herein allow for enhanced use of the featured inducible regulatory system. Substituted tetracycline compounds of the methods of the invention allow for induction of substituted tetracycline compound-triggered responses at concentrations as low as ten-fold less than those used for doxycycline. In certain embodiments, the methods of the invention can also for *in vivo* induction of a gene at a 100-fold lower effector concentrations than doxycycline.

Certain compounds featured in the methods of the invention also exhibit improved partitioning across the blood-brain barrier, allowing for realization of an induction response in a subject at lower concentrations of compound administration. This advantage is both economic and therapeutic, as lower circulating concentrations of the featured compounds of the invention present less likelihood of inducing undesirable side-effects following administration. Certain of the featured compounds of the invention are also non-antibiotic in nature, as compared to the antibiotic effects of e.g., doxycycline, thus presenting an additional advantage for practice of the methods of the invention in humans.

This invention is further illustrated by the following examples which should not be construed as limiting. The contents of all references, patents and published patent applications cited throughout this application are hereby incorporated by reference.

### EXAMPLES

### Example 1: Induction of Luciferase Activity by Subsituted Tetracycline Compounds

The ability of substituted tetracycline compounds to induce luciferase expression in HR5-C11 cells was examined. Cell line HRS-CL11 cells possess a luciferase gene and the rtTA gene, but not the tTA gene. HR5-C11 cells were plated at a density of about 3x10⁴ cells/35 mm dish (about 80% confluency). After full attachment of the cells, the tetracycline derivatives were admistered to the cells at concentrations of 0, 30 through 3000 ng/mL. The luciferase activity was measured after three days incubation.

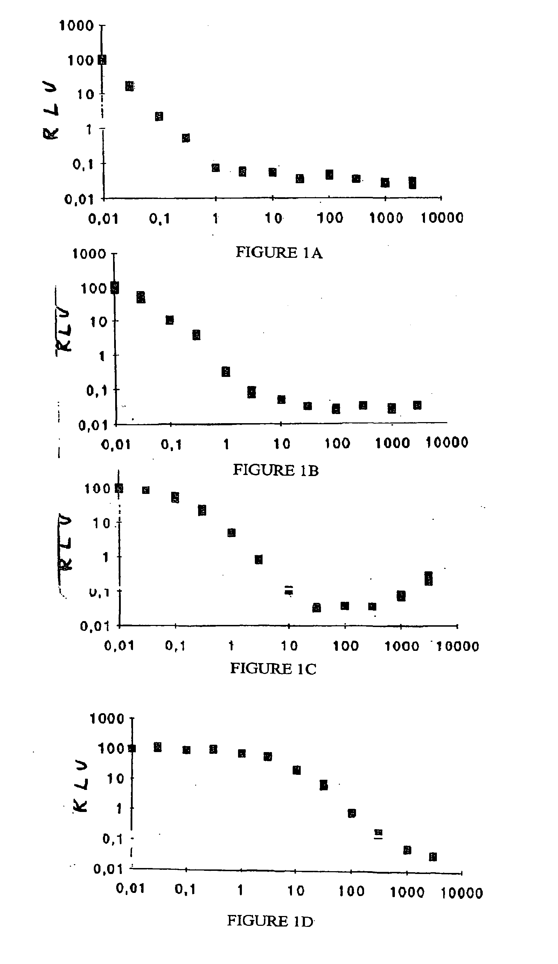

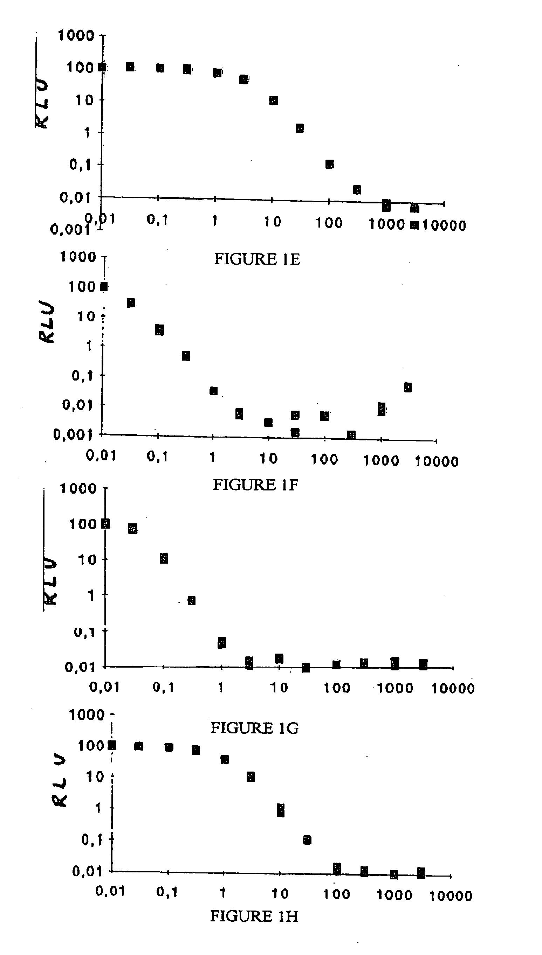

It was found that all of the tetracycline compounds increased *luc* activity. It was found that 9-t-butyl doxycycline resulted in the highest increase in *luc* expression, followed by pentacycline, 9-1'methylcyclopentyl doxycycline, 5-esters of doxcycline, 7,9-disubstituted doxcyclines, and 9-amino-substituted doxycycline. The dose response curves are shown in Figures 1A-1H (Doxycycline (Figure 1A); 5-cyclobutanoate doxycycline (Figure 1B); 5-cyclohexanoate doxycycline (Figure 1C); 5-propionyl-7-cyclopentylacetylamino doxycycline (Figure 1D); 7-acetylamino doxycycline (Figure 1E); 9-1'-methylcyclopentyl doxycycline (Figure 1F); 9-1'-methylcyclobutyl doxycycline (Figure 1G); 9-t-butyl-7-methyl doxycycline (Figure 1H)).

### Example 2: rtTA-Mediated Gene Activation using Substituted Tetracycline Compounds

Two luciferase positive cell lines 34R and MT2 produced new transactivators rtTA2-34R and rtTA2-MT2 respectively. These mutants are characterized by a very low level of residual DNA-binding in the presence of tetracycline compounds. With the rtTA2-MT2 system, 9-t-butyl doxycycline increased RtTA-mediated gene activation by 100 fold. 9-t-butyl doxycycline activated the system at concentrations between 30 and 100 ng/mL. It was found that 9-t-butyl doxycycline induced all rtTA's at a 10 fold lower concentration than doxycycline *in vitro.* Full induction of the system occured at a concentration of 10 ng/mL of 9-t-butyl doxycycline. It was also found that 5-phenylcarbamate doxcycline is also two fold better activiator of rtTA2s-M2 than doxycycline.

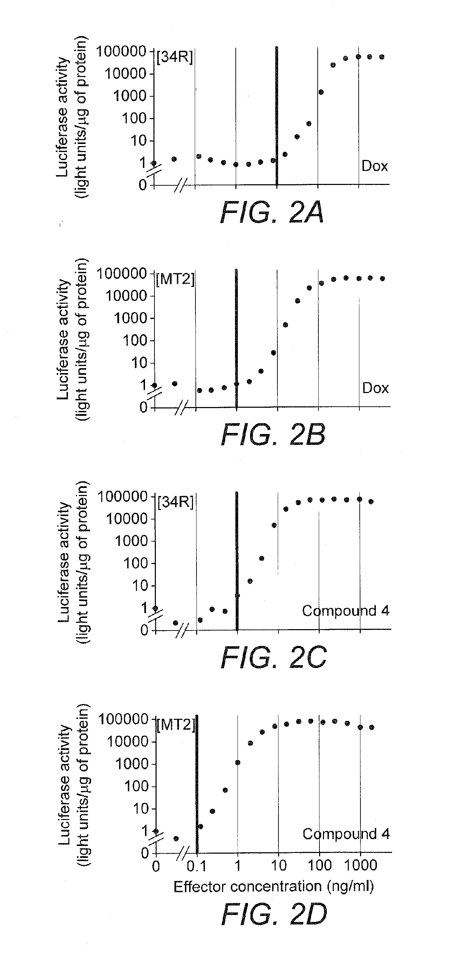

Figures 2A-2D show a comparison of doxycycline and 9-t-butyl doxcycline in 34R and MT2 rtTA mutants. Figures 2A and 2B show the effect of doxycyline on 34R and MT2 mutants, respectively. Figures 2C and 2D show the effect of 9-t-butyl doxycycline on 34R and MT2 mutants, respectively. Although doxycycline demonstrated greater specificity and luiciferase activity with MT2, 9-t-butyl doxcycline was ten times more active in both systems.

### Example 3: Dose Response Studies using X1/5 Cells and Subsituted Tetracycline Compounds

The ability of substituted tetracycline compounds on tTA and rtTA. transactivation using dose-response analysis with X1/5 cells were studied. Cell line X1/5 cells possess chromosomally integrated copies of the tTA gene and a luciferase gene controlled by a tetracycline-inducible promoter. After full attachment of the cells, the tetracycline derivatives were admistered to the cells at concentrations of 0, 30 through 3000 ng/mL. The luciferase activity was measured after three days incubation.

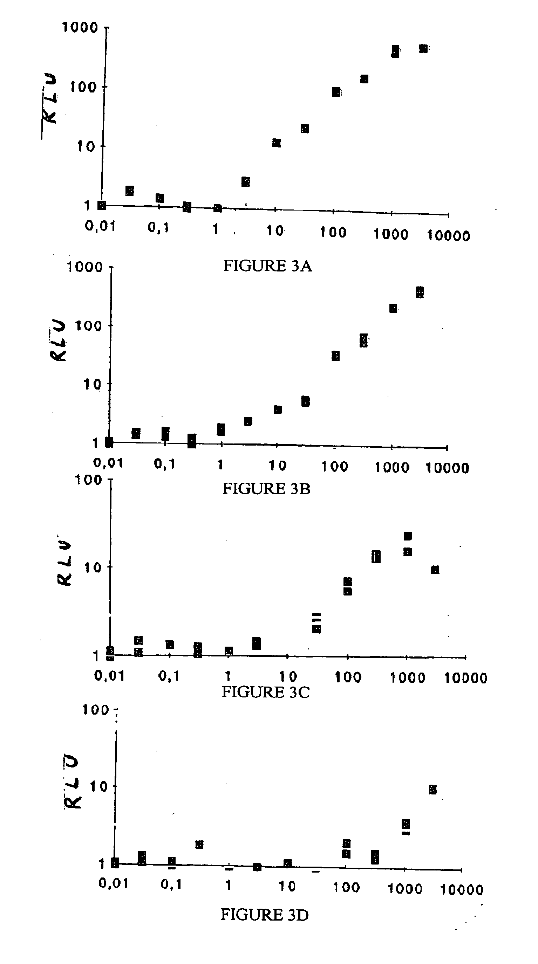

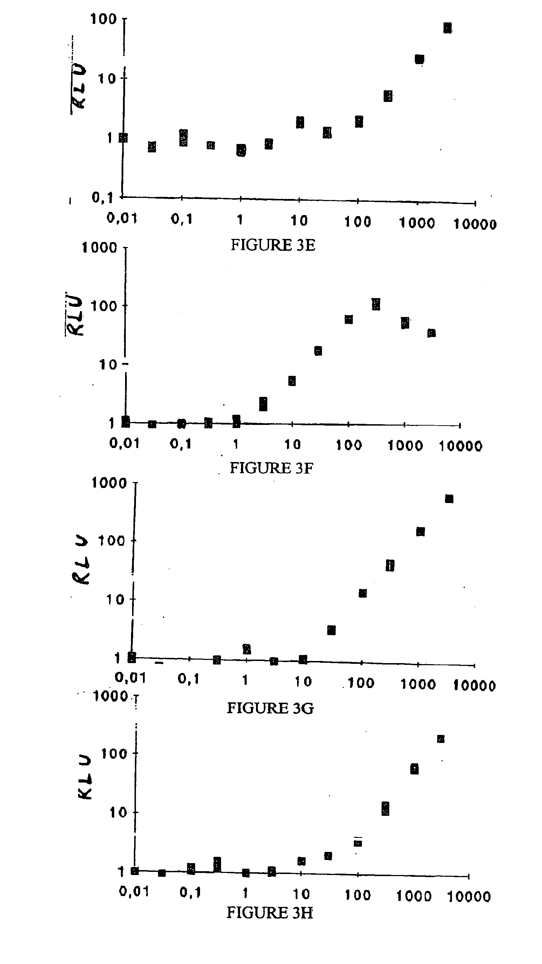

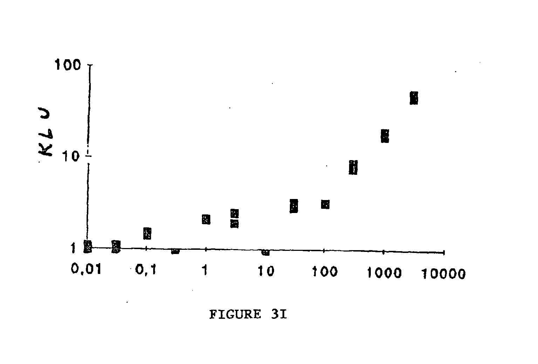

It was found that as the concentration of doxycycline was increased, the switch turned off the *luc* gene. All the tested tetracycline compounds decreased the luciferase activity. 9-t-butyl doxycycline showed efficacy as measured by *luc* expression at the lowest concentrations, followed by pentacycline, 9-1'methylcyclopentyl doxycycline, doxycycline, 5-butanoate doxcycline, 5-cyclohexanoate doxycycline, 5,9-disubstituted doxcyclines, 7,9-disubstituted doxyclines and 9-amino substituted doxycycline. The dose response curves are shown in Figures 3A-3I (Doxycycline (Figure 3A); 5-cyclobutanoate doxycycline (Figure 3B); 5-cyclohexanoate doxycycline (Figure 3C); 5-propionyl-7-cyclopentylacetylamino doxycycline (Figure 3D); 7-acetylamino doxycycline (Figure 3E); 9-1'-methylcyclopentyl doxycycline (Figure 3F); 9-1'-methylcyclobutyl doxycycline (Figure 3G); 9-t-butyl-7-methylthiomethyl doxycycline (Figure 3H); and 9-t-butyl-7-methyl doxycycline (Figure 3I)).

### Example 4: Activation of rtTA2s-M2 gene in vivo by Substituted Tetracycline Compounds

9-t-butyl doxycycline was tested in bitransgenic mice with rTA M2CaMK-1/LC1. The mice expressed the rtTA2s-M2 gene under control of the forebrain specific α-CamKII promoter. In addition, the LC1 mouse had *luciferase* and *cre* genes under the control of the bi-directional promoter. The mice were adminstered doxycycline (2 mg/mL) for seven days in the drinking water with 5% sucrose or with 9-t-butyl doxycycline at (0.2 and 2 mg/mL). The mice were anaesthetized with avertin and injected with luciferase (IP) and placed in the bioluminescence chamber and measured 5 minutes later. The results showed that 9-t-butyl doxycycline at 0.2 mg/mL had a better activation response than did doxycycline at 2 mg/mL. Figure 4 shows an image of the luciferase expression in the mice.

### Example 5: Dose Response Analysis of Tetracycline Compounds with the JE305K+pTetLua1 tet Detection System

The substituted tetracycline compounds were examined in a dose response analysis using the JE305K+pTetLuxl tet detection system. JE305K is a strain that has disrupted acrB and waaP genes. pTetLux1 is a plasmid which contains tetR, and has the luxCDABE operon (from P. *luminescens*) under the regulation of the tetA promotor/operator. The plasmid was obtained from the University of Turku, Finland.

The following minocycline derivatives with 9-position phenyls, alkyl, alkenyl or alkynyl groups were found to be potent and non-antibacterial.

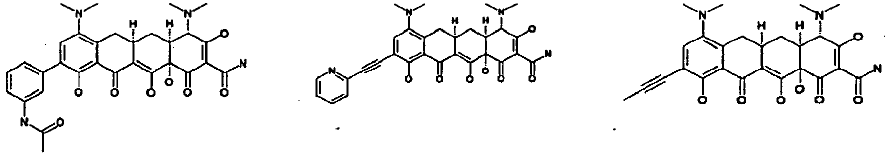

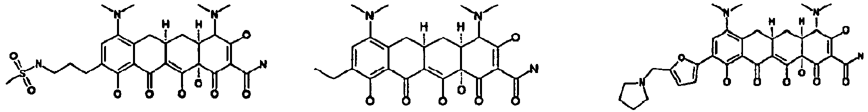

Substitution by alkyl, benzyl or similar substituents at the 10-position resulted in increased activation and a loss of antibacterial activity.

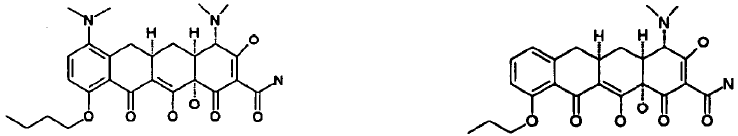

It was found that 7-alkynyl, alkenyl, alkyl and phenyl substituted sancycline compounds were active in the asasay. These derivatives possess varying degrees of antibacterial activity. An example of an active 7-alkynyl sancycline compound is shown below.

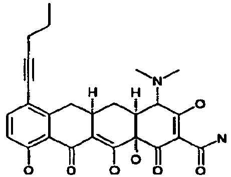

In addition, compounds A-Q showed excellent activity (e.g., a Klux of greater than about 70 about a concentration of about 13µg/mL); compounds R-BW showed good activity (e.g., a Klux of between about 51 and 70 at a concentration of about 13µg/mL) and compounds BX-LE showed little activity (e.g., a Klux of less than 51).

### EQUIVALENTS

Those skilled in the art will recognize, or be able to ascertain using no more than routine experimentation, many equivalents to the specific embodiments of the invention described herein. Such equivalents are intended to be encompassed by the following claims.

For the avoidance of doubt, the present invention includes the embodiments described in the following numbered paragraphs (referred to as "para.").
1. A method for regulating expression of a tet operator-linked nucleotide sequence in a cell containing (i) a target nucleotide sequence operatively-linked to a tetracycline responsive promoter element (TRE) and (ii) a fusion protein comprising a first polypeptide which binds to the TRE in the presence or absence of a substituted tetracycline compound operatively-linked to a second polypeptide which regulates transcription in cells, comprising modulating the concentration of the substituted tetracycline compound in the cell, such that expression of the target nucleotide sequence in the cell is regulated, wherein the substituted tetracycline compound is of formula (I): 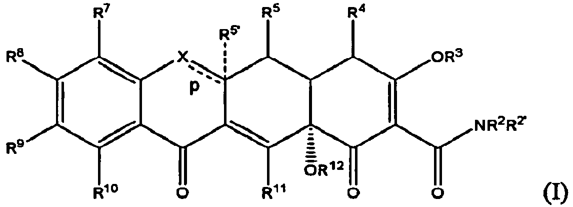 wherein
   p is a single or double bond;
   X is CHC(R¹³Y'Y), CR^{6'}R⁶, C=CR^{6'}R⁶, S, NR⁶, or O; and R^{5'} is hydrogen when p is a single bond;
   X is CR^{6"} and R^{5'} is absent when p is a double bond;
   R², R^{2'}, R^{4'}, and R^{4"} are each independently hydrogen, alkyl, alkenyl, alkynyl, alkoxy, alkylthio, alkylsulfinyl, alkylsulfonyl, alkylamino, arylalkyl, aryl, heterocyclic, heteroaromatic or a prodrug moiety;
   R³ and R¹² are each hydrogen or a pro-drug moiety;
   R⁴ is NR^{4'}R^{4"}, alkyl, alkenyl, alkynyl, hydroxyl, halogen, or hydrogen;
   R⁵ is hydroxyl, hydrogen, thiol, alkanoyl, aroyl, alkaroyl, aryl, heteroaromatic, alkyl, alkenyl, alkynyl, alkoxy, alkylthio, alkylsulfinyl, alkylsulfonyl, alkylamino, arylalkyl, alkyl carbonyloxy, or aryl carbonyloxy;
   R⁶ and R^{6'} are each independently hydrogen, methylene, absent, hydroxyl, halogen, thiol, alkyl, alkenyl, alkynyl, aryl, alkoxy, alkylthio, alkylsulfinyl, alkylsulfonyl, alkylamino, or an arylalkyl;
   R⁷ is hydrogen, hydroxyl, halogen, cyano, oximyl, alkoxycarbonyl, alkylcarbonyl, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃(NR^{7c})₀₋₁C(=W')WR^{7a};
   R⁸ is hydrogen, hydroxyl, halogen, cyano, oximyl, alkoxycarbonyl, alkylcarbonyl, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃(NR^{8c})₀₋₁C(=E')ER^{8a};
   R⁹ is hydrogen, hydroxyl, halogen, cyano, oximyl, alkoxycarbonyl, alkylcarbonyl, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃(NR^{9c})₀₋₁C(=Z')ZR^{9a};
   R¹⁰ is hydrogen, hydroxyl, halogen, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic or thionitroso;
   R¹¹ is hydrogen, hydroxyl or alkoxyl.
   R^{7a}, R^{7b}, R^{7c}, R^{7d}, R^{7e}, R^{7f}, R^{8a}, R^{8b}, R^{8c}, R^{8d}, R^{8e}, R^{8f}, R^{9a}, R^{9b}, R^{9c}, R^{9d}, R^{9e}, and R^{9f} are each independently hydrogen, acyl, alkyl, alkenyl, alkynyl, alkoxy, alkylthio, alkylsulfinyl, alkylsulfonyl, alkylamino, arylalkyl, aryl, heterocyclic, heteroaromatic or a prodrug moiety;
   R¹³ is hydrogen, hydroxy, alkyl, alkenyl, alkynyl, alkoxy, alkylthio, aryl, alkylsulfinyl, alkylsulfonyl, alkylamino, or an arylalkyl;
   E is CR^{8d}R^{8c} S, NR^{8b} or O;
   E' is O, ,NR^{8f}, or S;
   W is CR^{7d}R^{7e}, S, NR^{7b} or O;
   W' is O, NR^{7f}, or S;
   Z is CR^{9d}R^{9c}, S, NR^{9b} or O_{;}
   Z' is O, S, or NR^{9f};
   Y' and Y are each independently hydrogen, halogen, hydroxyl, cyano, sulfhydryl, amino, alkyl, alkenyl, alkynyl, alkoxy, alkylthio, alkylsulfinyl, alkylsulfonyl, alkylamino, or an arylalkyl; and pharmaceutically acceptable salts, esters and enantiomers thereof
2. The method of para. 1, wherein the target nucleotide sequence encodes a protein.
3. The method of any one of para. 1-2, wherein the first polypeptide binds to the TRE in the presence of said substituted tetracycline compound.
4. The method of any one of para. 1-2, wherein the first polypeptide binds to the TRE in the absence, but not the presence of said substituted tetracycline compound.
5. The method of any one of para. 1-4, wherein the first polypeptide of the fusion protein is a mutated Tet repressor.
6. The method of para. 5, wherein the mutated Tet repressor is a class B repressor.
7. The method of para. 6, wherein the mutated Tet repressor is a Tn10-derived Tet repressor having an amino acid substitution at at least one amino acid position selected from the group consisting of amino acid position 71, position 95, position 101 and position 102.
8. The method of para. 6, wherein the mutated Tet repressor is a Tn10-derived Tet repressor having an amino acid substitution at at least two amino acid positions selected from the group consisting of amino acid position 71, position 95, position 101 and position 102
9. The method of any one of para. 1-8, wherein the second polypeptide of the fusion protein comprises a transcription activation domain of herpes simplex virion protein 16.
10. The method of any one of para. 1-9, wherein the nucleic acid molecule encoding the fusion protein is integrated randomly in a chromosome of the cell.
11. The method of any one of para. 1-10, wherein the nucleic acid molecule encoding the fusion protein is integrated at a predetermined location within a chromosome of the cell.
12. The method of any one of para. 1-11, wherein the nucleic acid molecule encoding the fusion protein is introduced into the cell ex *vivo,* the method further comprising administering the cell to a subject.
13. The method of any one of para. 1-12, wherein the tet operator-linked nucleic acid is an endogenous nucleic acid of the cell which has been operatively linked to at least one tet operator sequence.
14. The method of any one of para. 1-13, wherein the tet operator-linked nucleic acid molecule is an exogenous nucleic acid molecule which has been introduced into the cell.
15. The method of any one of para. 1-14, wherein the cell further contains a second target nucleic acid molecule.
16. The method of any one of para. 1-15, wherein the tetracycline compound does not have antibiotic activity.
17. The method of para. 1, wherein R², R^{2˙}, R³ and R¹² are each hydrogen; R⁴ is NR⁴R^{4'}; R⁴ and R^{4'} are each alkyl; and R¹¹ is hydroxyl.
18. The method of para. 17, wherein p is a single bond; X is CR⁶R^{6'}; R⁶ is alkyl; R^{6'} is hydrogen and R⁵ is hydroxyl.
19. The method of para. 28, wherein R⁷ and R⁸ are each hydrogen.
20. The method of para. 19, wherein R¹⁰ is hydroxyl.
21. The method of para. 20, wherein R⁹ is aminoalkyl, alkenyl, alkoxycarbonyl or a heterocyclic moiety.
22. The method of para. 21, wherein said alkoxycarbonyl is methoxycarbonyl.
23. The method of para. 21, wherein said heterocyclic moiety is morpholine.
24. The method of para. 21, wherein said aminoalkyl is aminomethyl.
25. The method of para. 21, wherein said alkenyl is aminocarbonylalkyl.
26. The method of para. 19, wherein R⁹ is hydrogen.
27. The method of para. 26, wherein R¹⁰ is alkoxy.
28. The method of para. 27, wherein said alkoxy is butoxy.
29. The method of para. 17, wherein p is a single bond; X is CR⁶R^{6'}; R⁵, R⁶ and R^{6'} are each hydrogen.
30. The method of para. 29, wherein R⁸ is hydrogen.
31. The method of para. 3 0, wherein R⁷ is amino.
32. The method of para. 31, wherein said amino is dialkylamino.
33. The method of para. 37, wherein said dialkylamino is dimethylamino.
34. The method of para. 31, wherein R¹⁰ is hydroxyl.
35. The method of para. 34, wherein R⁹ is halogen, alkyl, aminoalkyl, alkoxycarbonyl, alkylcarbonyl, alkenyl, alkynyl, aryl or heterocyclic moiety.
36. The method of para. 35, where said halogen is fluorine.
37. The method of para. 36, wherein said alkoxycarbonyl is methoxycarbonyl.
38. The method of para. 36, wherein said alkylcarbonyl is acyl.
39. The method of para. 36, wherein said heterocyclic moiety is morpholine.
40. The method of para. 39, wherein said aryl is heteroaryl.
41. The method of para. 40, wherein said heteroaryl is furanyl.
42. The method of para. 41, wherein said furanyl is alkyl substituted furnayl.
43. The method of para. 35, wherein said alkyl is propyl.
44. The method of para. 43, said aminoalkyl is aminomethyl.
45. The method of para. 44, wherein said aminomethyl is alkylaminomethyl or arylaminomethyl.
46. The method of para. 35, wherein said alkenyl is alkoxycarbonylalkenyl or aminoalkenyl.
47. The method of para. 46, wherein said aminoalkenyl is alkylaminoalkenyl.
48. The method of para. 46, wherein said alkoxycarbonylalkenyl is ethoxycarbonylalkenyl.
49. The method of para. 31, wherein R⁹ is hydrogen and R¹⁰ is alkoxy.
50. The method of para. 49, wherein said alkoxy is butoxy.
51. The method of para. 30, wherein R¹⁰ is hydroxyl.
52. The method of para. 51, wherein R⁷ is halogen
53. The method of para. 52, wherein said halogen is iodine.
54. The method of para. 52, wherein R⁹ is alkyl.
55. The method of para. 51, wherein R⁷ is aryl.
56. The method of para. 55, wherein said aryl is heteroaryl.
57. The method of para. 56, wherein said heteroaryl is pyridinyl.
58. The method of para. 55, wherein R⁹ is alkyl.
59. The method of para. 58, wherein said alkyl is methyl.
60. The method of para. 51, wherein R⁷ is alkenyl.
61. The method of para. 60, wherein said alkenyl is substituted with one or more halogens.
62. The method of para. 61, wherein said halogen is fluorine.
63. The method of para. 60, wherein R⁹ is amino or nitro.
64. The method of para. 51, wherein R⁷ is alkyl.
65. The method of para. 64, wherein said alkyl is ethyl.
66. The method of para. 64, wherein R⁹ is aminoalkyl.
67. The method of para. 66, wherein said aminoalkyl is alkylaminoalkyl.
68. The method of para. 30, wherein R⁹ is hydrogen and R¹⁰ is hydroxyl.
69. The method of para. 68, wherein R⁷ is amino, aryl, alkyl, alkenyl, aminoalkyl, cyano, a heterocyclic moiety or oximyl.
70. The method of para. 69, wherein said amino is alkylamino or alkylcarbonylamino
71. The method of para. 70, wherein said amino is dialkylamino.
72. The method of para. 71, wherein said dialkylamino is diethylamino.
73. The method of para. 69, wherein said aryl is phenyl or heteroaryl.
74. The method of para. 73, wherein said heteroaryl is pyridinyl, furanyl, isoxazolyl, or pyrazolyl.
75. The method of para. 74, wherein said pyridinyl is substituted with one or more halogens.
76. The method of para. 75, wherein said halogen is chlorine or fluorine.
77. The method of para. 74, wherein said isoxazolyl is alkyl substituted isoxazolyl.
78. The method of para. 69, wherein said alkyl is methyl, isobutyl, isopropyl, isopropenyl or alkoxy substituted alkyl.
79. The method of para. 69, wherein said heterocyclic moiety is morpholine.
80. The method of para. 69, wherein said alkenyl is substituted with one or more halogens, cyano, alkoxycarbonyl or alkylcarbonyl.
81. The method of para. 80, wherein said halogen is fluorine.
82. The method of para. 80, wherein said alkoxycarbonyl is methoxycarbonyl.
83. The method of para. 80, wherein said alkylcarbonyl is acyl.
84. The method of para. 69, wherein said aminoalkyl is aminomethyl.
85. The method of para. 84, wherein said aminomethyl is substituted with a heterocyclic moiety.
86. The method of para. 85, wherein said heterocyclic moiety is piperidine.
87. The method of para. 29, wherein R¹⁰ is hydroxyl.
88. The method of para. 87, wherein R⁷ is hydrogen and R⁸ is halogen or aryl.
89. The method of para. 88, wherein said halogen is chlorine or bromine.
90. The method of para. 88, wherein said aryl is phenyl or pyridinyl.
91. The method of para. 90, wherein said pyridinyl is substituted with halogen.
92. The method of para. 91, wherein said halogen is fluorine.
93. The method of para. 88, wherein R⁹ is hydrogen or amino.
94. The method of para. 93, wherein said amino is substituted with alkylcarbonyl.
95. The method of para. 94, wherein said alkylcarbonyl is acyl.
96. The method of para. 87, wherein R⁷ is amino, R⁸ is halogen and R⁹ is hydrogen.
97. The method of para. 96, wherein said halogen is chlorine.
98. The method of para. 29, wherein R⁷ is hydrogen and R¹⁰ is hydroxyl.
99. The method of para. 98, wherein R⁹ is a heterocyclic moiety or aminoalkyl. -
100. The method of para. 99, wherein said heterocyclic moiety is piperidine or morpholine.
101. The method of para. 99, wherein said aminoalkyl is aminomethyl.
102. The method of para. 101, wherein said aminomethyl is substituted with trifluoroalkyl
103. The method of para. 30, wherein R⁷ and R⁹ are hydrogen; and R¹⁰ is alkoxy.
104. The method of para. 103, wherein said alkoxy is propoxy or butoxy.
105. The method of para. 103, wherein said alkoxy is substituted with hydroxyl.
106. The method of para. 17, wherein p is a double bond; X is CR^{6"}; and R^{6"} is hydrogen.
107. The method of para. 106, wherein R⁸ and R⁹ are hydrogen and R¹⁰ is hydroxyl.
108. The method of para. 107, wherein R⁷ is halogen.
109. The method of para. 108, wherein said halogen is chlorine.
110. A method for regulating expression of a tet operator-linked nucleotide sequence in a cell containing (i) a target nucleotide sequence operatively-linked to a tetracycline responsive promoter element (TRE) and (ii) a fusion protein comprising a first polypeptide which binds to the TRE in the presence or absence of a substituted tetracycline compound operatively-linked to a second polypeptide which regulates transcription in cells, comprising modulating the concentration of the substituted tetracycline compound in the cell, such that expression of the target nucleotide sequence in the cell is regulated, wherein the substituted tetracycline compound is of formula (II): 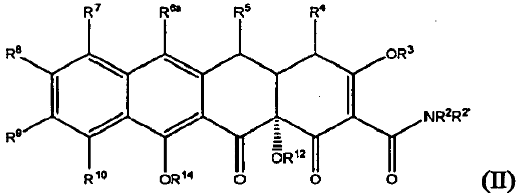 wherein
   R², R^{2'}, R^{4'}, and R^{4"} are each independently hydrogen, alkyl, alkenyl, alkynyl, alkoxy, alkylthio, alkylsulfinyl, alkylsulfonyl, alkylamino, arylalkyl, aryl, heterocyclic, heteroaromatic or a prodrug moiety;
   R³, R¹² and R¹⁴ are each hydrogen or a pro-drug moiety;
   R⁴ is NR^{4'}R^{4"}, alkyl, alkenyl, alkynyl, hydroxyl, halogen, or hydrogen;
   R⁵ is hydroxyl, hydrogen, thiol, alkanoyl, aroyl, alkaroyl, aryl, heteroaromatic, alkyl alkenyl, alkynyl, alkoxy, alkylthio, alkylsulfinyl, alkylsulfonyl, alkylamino, arylalkyl, alkyl carbonyloxy, or aryl carbonyloxy;
   R^{6a} is hydrogen, hydroxyl, halogen, thiol, alkyl, alkenyl, alkynyl, aryl, alkoxy, alkylthio, alkylsulfinyl, alkylsulfonyl, alkylamino, or an arylalkyl;
   R⁷ is hydrogen, hydroxyl, halogen, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃(NR^{7c})₀₋₁C(=W')WR^{7a};
   R⁸ is hydrogen, hydroxyl, halogen, thiol, nitro, alkyl, alkenyl, alkynyl, aryl, alkoxy, alkylthio, alkylsulfinyl, alkylsulfonyl, alkylamino, amino, arylalkenyl, arylalkynyl, acyl, aminoalkyl, heterocyclic, thionitroso, or -(CH₂)₀₋₃(NR^{8c})₀₋₁C(=E')ER^{8a}_{;}
   R⁹ is hydrogen, hydroxyl, halogen, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃(NR⁷)₀₋₁C(=Z')ZR^{9a};
   R¹⁰ is hydrogen, hydroxyl, halogen, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic or thionitroso;
   R^{7a}, R^{7b}, R^{7c}, R^{7d}, R^{7e}, R^{7f}, R^{8a}, R^{8b}, R^{8c}, R^{8d}, R^{8e}, R^{8f}, R^{9a}, R^{9b}, R^{9c}, R^{9d}, R^{9e}, and R^{9f} are each independently hydrogen, acyl, alkyl, alkenyl, alkynyl, alkoxy, alkylthio, alkylsulfinyl, alkylsulfonyl, alkylamino, arylalkyl, aryl, heterocyclic, heteroaromatic or a prodrug moiety;
   W is CR^{7d}R^{7e} S, NR^{7b} or O;
   W' is O, NR^{7f}, or S;
   E is CR^{8d}R^{8e}, S, NR^{8b} or O;
   E' is O, NR^{8f}, or S;
   Z is CR^{9d}R^{9e} S, NR^{9b} or O;
   Z' is O, S, or NR^{9f}; and pharmaceutically acceptable salts, esters and enantiomers thereof.
111. The method of para. 110, wherein R², R^{2'}, R³, R⁵, R⁸, R⁹, R¹¹,R¹² and R¹⁴ are each hydrogen; R⁴ is NR⁴R^{4'}; R⁴, R^{4'} are R^{6a} are each alkyl; and R¹⁰ is hydroxyl.
112. The method of para. 111, wherein and are each R⁷ is hydrogen.
113. The method of para. 111, wherein R⁷ is halogen.
114. The method of para. 113, wherein said halogen is chlorine.
115. The method of para. 1, wherein said substituted tetracycline compound is selected from the group consisting of: 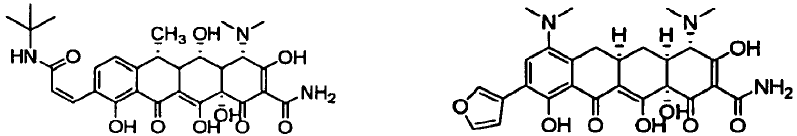 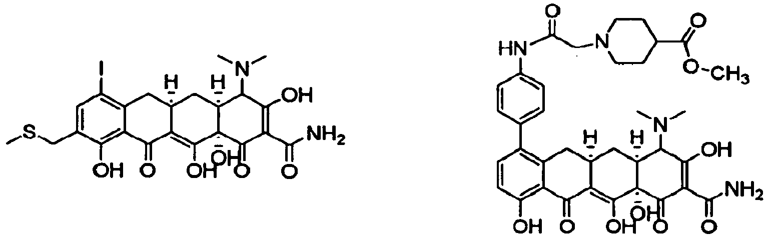 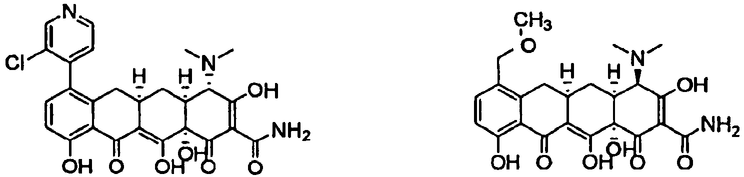 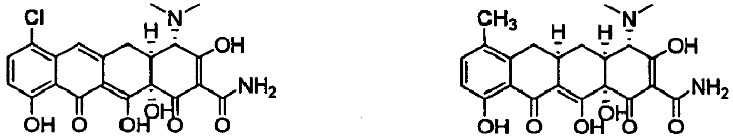 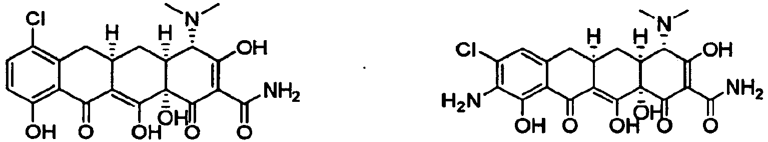 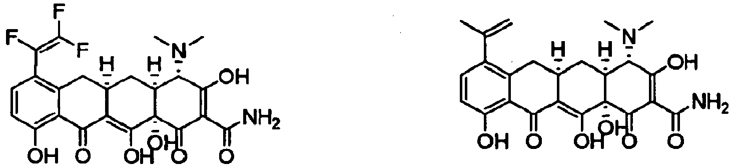 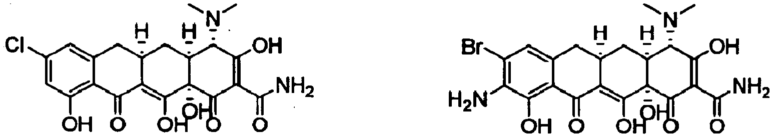 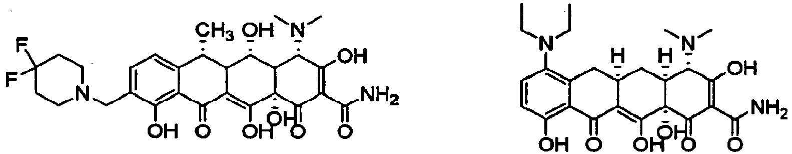 and pharmaceutically acceptable salts thereof.
116. The method of para. 1, wherein the compound is selected from the group consisting of: 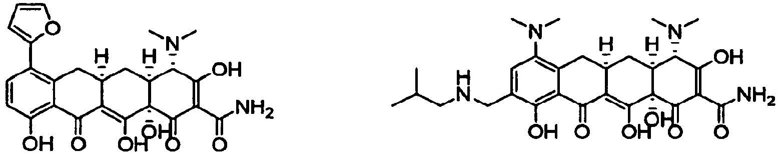 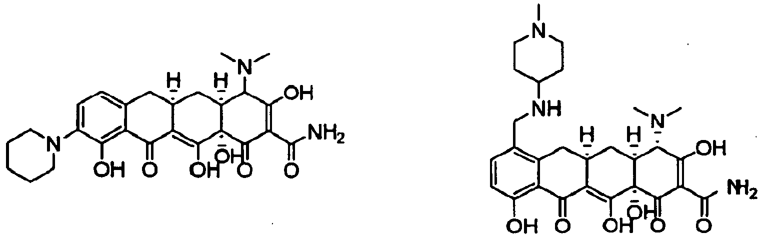 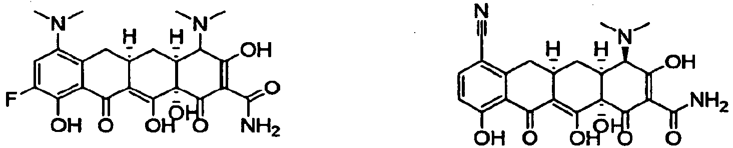 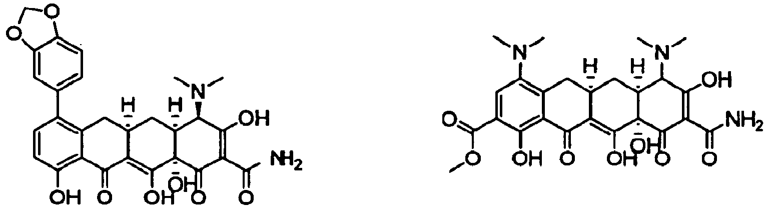 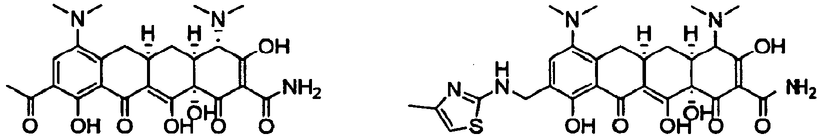 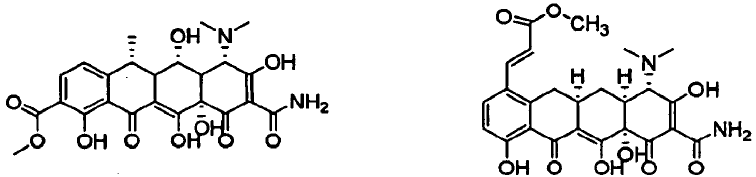 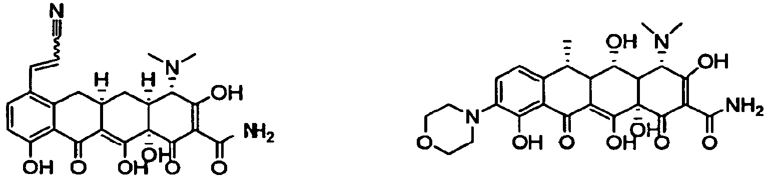 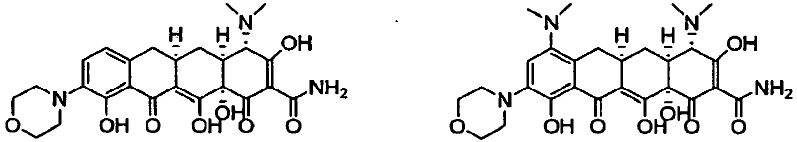 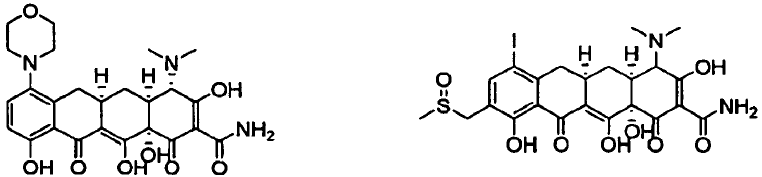 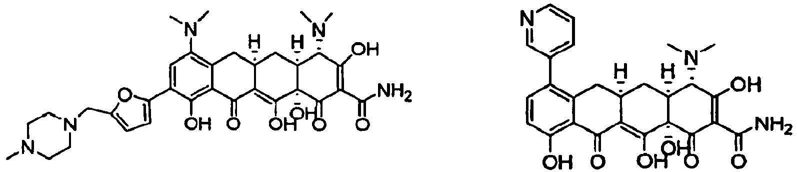 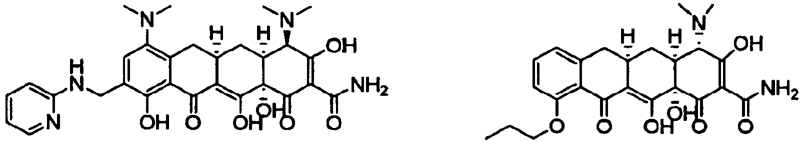 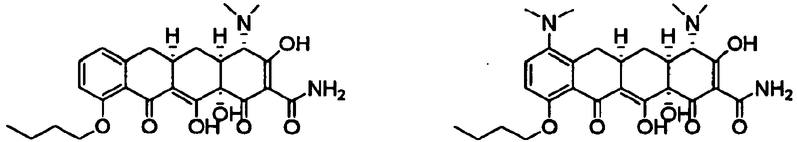 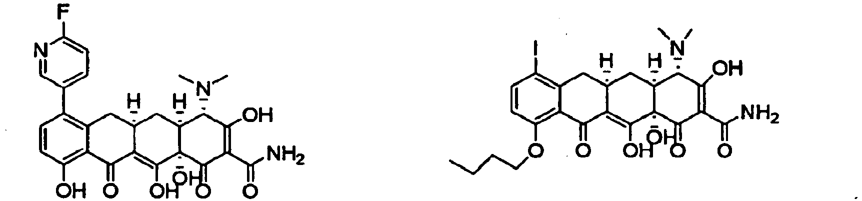 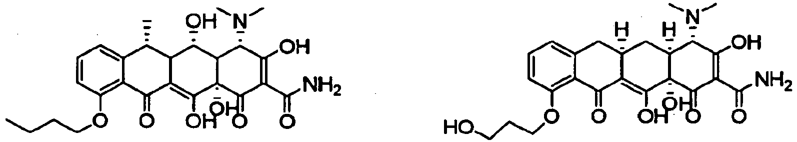 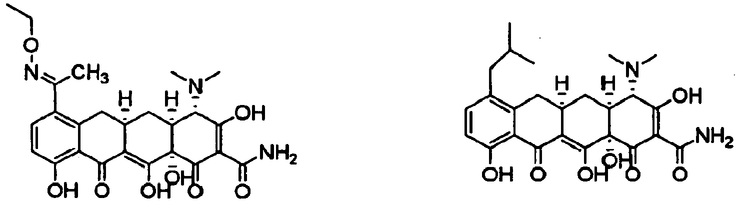 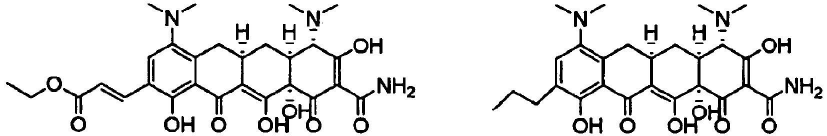 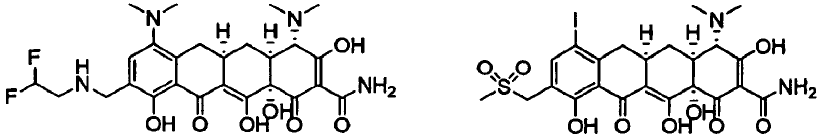 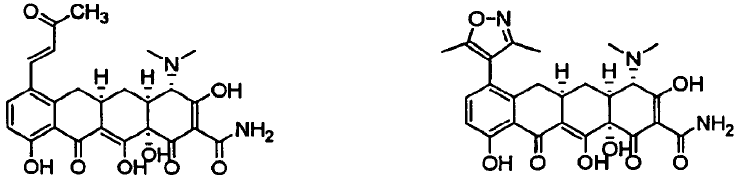 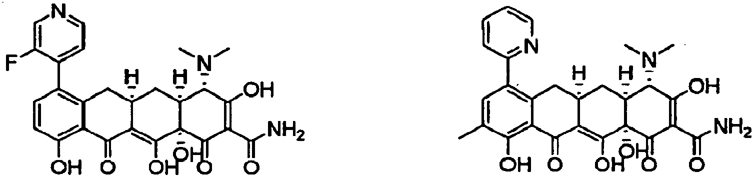 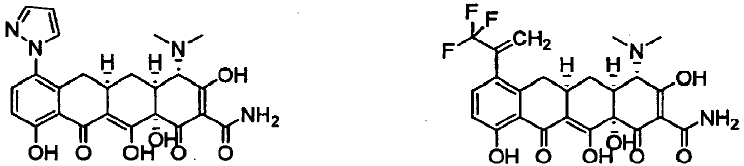 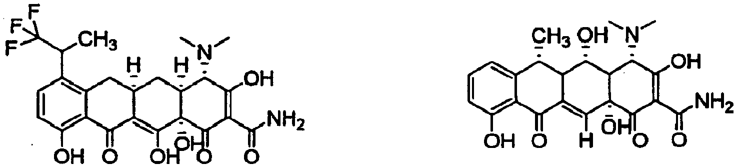 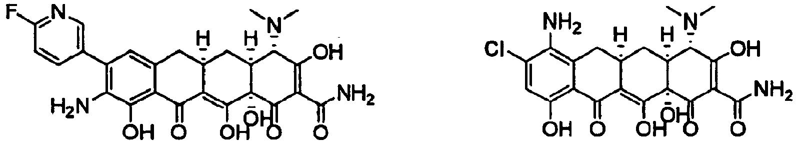 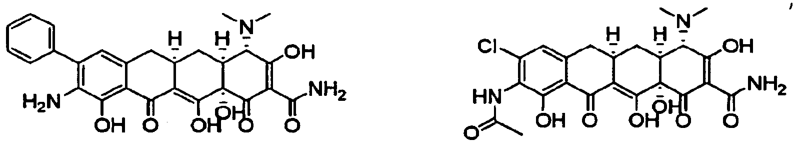 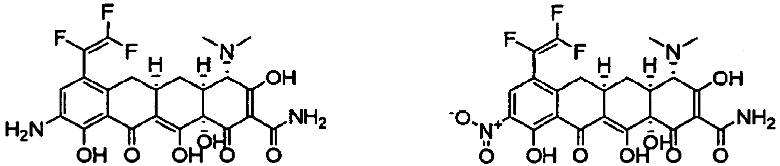 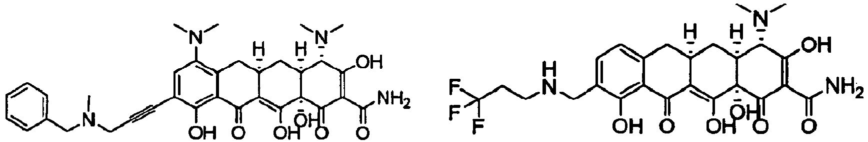 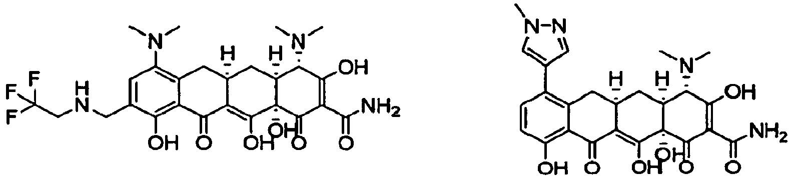 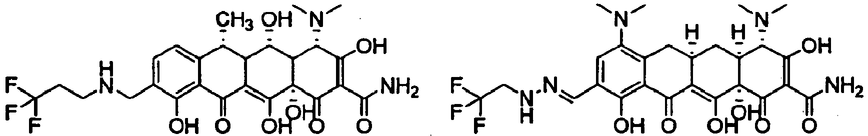 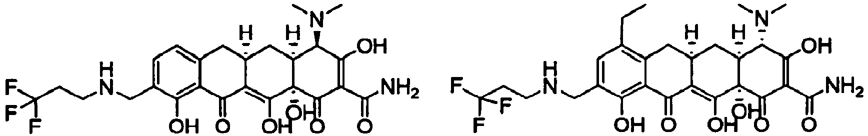 and pharmaceutically acceptable salts thereof
117. The method of para.1, wherein the compound is selected from the group consisting of: 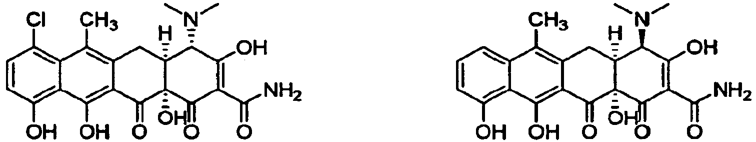 and pharmaceutically acceptable salts thereof.
118. The method of para. 54, wherein said alkyl is substituted with alkylthio, alkylsulfonyl or alkylsulfinyl.
119. A method for regulating expression of a tet operator-linked nucleotide sequence in a cell containing (i) a target nucleotide sequence operatively-linked to a tetracycline responsive promoter element (TRE) and (ii) a mutated Tet repressor which binds to the TRE in the presence but not in the absence of a substituted tetracycline compound, comprising modulating the concentration of the substituted tetracycline compound in the cell, such that expression of the target nucleotide sequence in the cell is regulated, wherein the mutated Tet repressor is selected such that said mutated Tet repressor bins selectively to the TRE in the presence of the substituted tetracycline compound of formula (I): 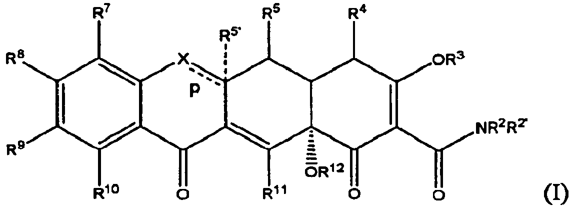 wherein
   p is a single or double bond;
   X is CHC(R¹³Y'Y), CR^{6'}R⁶, C=CR^{6'}R⁶, S, NR⁶, or O; and R^{5'} is hydrogen when p is a single bond;
   X is CR^{6"} and R^{5'} is absent when p is a double bond;
   R², R^{2'}, R^{4'}, and R^{4"} are each independently hydrogen, alkyl, alkenyl, alkynyl, alkoxy, alkylthio, alkylsulfinyl, alkylsulfonyl, alkylamino, arylalkyl, aryl, heterocyclic, heteroaromatic or a prodrug moiety;
   R³ and R¹² are each hydrogen or a pro-drug moiety;
   R⁴ is NR^{4'}R^{4"}, alkyl, alkenyl, alkynyl, hydroxyl, halogen, or hydrogen;
   R⁵ is hydroxyl, hydrogen, thiol, alkanoyl, aroyl, alkaroyl, aryl, heteroaromatic, alkyl, alkenyl, alkynyl, alkoxy, alkylthio, alkylsulfinyl, alkylsulfonyl, alkylamino, arylalkyl, alkyl carbonyloxy, or aryl carbonyloxy;
   R⁶ and R^{6'} are each independently hydrogen, methylene, absent, hydroxyl, halogen, thiol, alkyl, alkenyl, alkynyl, aryl, alkoxy, alkylthio, alkylsulfinyl, alkylsulfonyl, alkylamino, or an arylalkyl;
   R⁷ is hydrogen, hydroxyl, halogen, cyano, oximyl, alkoxycarbonyl, alkylcarbonyl, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃(NR^{7c})₀₋₁C(=W')WR^{7a};
   R⁸ is hydrogen, hydroxyl, halogen, cyano, oximyl, alkoxycarbonyl, alkylcarbonyl, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃(NR^{8c})₀₋₁C(=E')ER^{8a};
   R⁹ is hydrogen, hydroxyl, halogen, cyano, oximyl, alkoxycarbonyl, alkylcarbonyl, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃(NR^{9c})₀₋₁C(=Z')ZR^{9a};
   R¹⁰ is hydrogen, hydroxyl, halogen, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic or thionitroso;
   R¹¹ is hydrogen, hydroxyl or alkoxyl.
   R^{7a}, R^{7b}, R^{7c}, R^{7d}, R^{7e}, R7^{f}, R^{8a}, R^{8b}, R^{8c}, R^{8d}, R^{8e}, R^{8f}, R^{9a}, R^{9b}, R^{9c}, R^{9d}, R^{9e}, and R^{9f} are each independently hydrogen, acyl, alkyl, alkenyl, alkynyl, alkoxy, alkylthio, alkylsulfinyl, alkylsulfonyl, alkylamino, arylalkyl, aryl, heterocyclic, heteroaromatic or a prodrug moiety;
   R¹³ is hydrogen, hydroxy, alkyl, alkenyl, alkynyl, alkoxy, alkylthio, aryl, alkylsulfinyl, alkylsulfonyl, alkylamino, or an arylalkyl;
   E is CR^{8d}R^{8e}, S, NR^{8b} or O;
   E' is O, NR^{8f}, or S;
   W is CR^{7d}R^{7e}, S, NR^{7b} or O;
   W' is O, NR^{7f}, or S;
   Z is CR^{9d}R^{9e}, S, NR^{9b} or O;
   Z' is O, S, or NR^{9f}_{;}
   Y' and Y are each independently hydrogen, halogen, hydroxyl, cyano, sulfhydryl, amino, alkyl, alkenyl, alkynyl, alkoxy, alkylthio, alkylsulfinyl, alkylsulfonyl, alkylamino, or an arylalkyl; and pharmaceutically acceptable salts, esters and enantiomers thereof.
120. A method for regulating expression of a tet operator-linked nucleotide sequence in a cell containing (i) a target nucleotide sequence operatively-linked to a tetracycline responsive promoter element (TRE) and (ii) a mutated Tet repressor which binds to the TRE in the presence but not in the absence of a substituted tetracycline compound, comprising modulating the concentration of the substituted tetracycline compound in the cell, such that expression of the target nucleotide sequence in the cell is regulated, wherein the mutated Tet repressor is selected such that said mutated Tet repressor bins selectively to the TRE in the presence of the substituted tetracycline compound of formula (II): 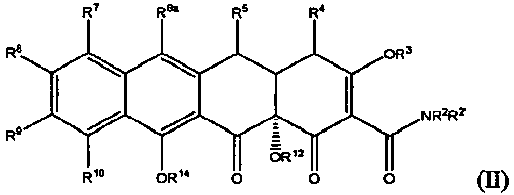 wherein
   R², R^{2'}, R^{4'}, and R^{4"} are each independently hydrogen, alkyl, alkenyl, alkynyl, alkoxy, alkylthio, alkylsulfinyl, alkylsulfonyl, alkylamino, arylalkyl, aryl, heterocyclic, heteroaromatic or a prodrug moiety;
   R³, R¹² and R¹⁴ are each hydrogen or a pro-drug moiety;
   R⁴ is NR^{4'}R^{4"}, alkyl, alkenyl, alkynyl, hydroxyl, halogen, or hydrogen;
   R⁵ is hydroxyl, hydrogen, thiol, alkanoyl, aroyl, alkaroyl, aryl, heteroaromatic, alkyl, alkenyl, alkynyl, alkoxy, alkylthio, alkylsulfinyl, alkylsulfonyl, alkylamino, arylalkyl, alkyl carbonyloxy, or aryl carbonyloxy;
   R^{6a} is hydrogen, hydroxyl, halogen, thiol, alkyl, alkenyl, alkynyl, aryl, alkoxy, alkylthio, alkylsulfinyl, alkylsulfonyl, alkylamino, or an arylalkyl;
   R⁷ is hydrogen, hydroxyl, halogen, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃(NT^{7c})₀₋₁C(=W')WR^{7a};
   R⁸ is hydrogen, hydroxyl, halogen, thiol, nitro, alkyl, alkenyl, alkynyl, aryl, alkoxy, alkylthio, alkylsulfinyl, alkylsulfonyl, alkylamino, amino, arylalkenyl, arylalkynyl, acyl, aminoalkyl, heterocyclic, thionitroso, or -(CH₂)₀₋₃(NR^{8c})₀₋₁C(=E')ER^{8a}_{;}
   R⁹ is hydrogen, hydroxyl, halogen, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃ (NR^{9c})₀₋₁C(=Z')ZR^{9a};
   R¹⁰ is hydrogen, hydroxyl, halogen, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic or thionitroso;
   R^{7a}, R^{7b}, R^{7c}, R^{7d}, R^{7e}, R^{7f}, R^{8a}, R^{8b}, R^{8c}, R^{8d}, R^{8e}, R^{8f}, R^{9a}, R^{9b}, R^{9c}, R^{9d}, R^{9e}, and R^{9f} are each independently hydrogen, acyl, alkyl, alkenyl, alkynyl, alkoxy, alkylthio, alkylsulfinyl, alkylsulfonyl, alkylamino, arylalkyl, aryl, heterocyclic, heteroaromatic or a prodrug moiety;
   W is CR^{7d}R^{7e}, S, NR^{7b} or O;
   W' is O, NR^{7f}, or S;
   E is CR^{8d}R^{8e}, S, NR^{8b} or O;
   E' is O, NR^{8f}, or S;
   Z is CR^{9d}R^{9e}, S, NR^{9b} or O;
   Z' is O, S, or NR^{9f}, and pharmaceutically acceptable salts, esters and enantiomers thereof.
121. A method for regulating expression of a tet operator-linked nucleotide sequence in a cell containing (i) a target nucleotide sequence operatively-linked to a tetracycline responsive promoter element (TRE) and (ii) a mutated Tet repressor which binds to the TRE in the absence but not in the presence of a substituted tetracycline compound, comprising modulating the concentration of the substituted tetracycline compound in the cell, such that exprese target nucleotide sequence in the cell is regulated, wherein the mutated Tet repressor is selected such that said mutated Tet repressor binds selectively to the TRE only in the absence of the substituted tetracycline compound of formula (I): 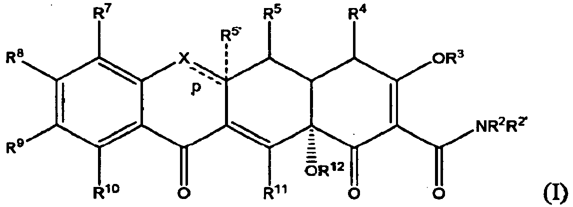 wherein
   p is a single or double bond;
   X is CHC(R¹³Y'Y), CR^{6'}R⁶, C=CR^{6'}R⁶, S, NR⁶, or O; and R^{5'} is hydrogen when p is a single bond;
   X is CR^{6"} and R^{5'} is absent when p is a double bond;
   R², R^{2'}, R^{4'}, and R^{4"} are each independently hydrogen, alkyl, alkenyl, alkynyl, alkoxy, alkylthio, alkylsulfinyl, alkylsulfonyl, alkylamino, arylalkyl, aryl, heterocyclic, heteroaromatic or a prodrug moiety;
   R³ and R¹² are each hydrogen or a pro-drug moiety;
   R⁴ is NR^{4'}R^{4"}, alkyl, alkenyl, alkynyl, hydroxyl, halogen, or hydrogen;
   R⁵ is hydroxyl, hydrogen, thiol, alkanoyl, aroyl, alkaroyl, aryl, heteroaromatic, alkyl, alkenyl, alkynyl, alkoxy, alkylthio, alkylsulfinyl, alkylsulfonyl, alkylamino, arylalkyl, alkyl carbonyloxy, or aryl carbonyloxy;
   R⁶ and R^{6'} are each independently hydrogen, methylene, absent, hydroxyl, halogen, thiol, alkyl, alkenyl, alkynyl, aryl, alkoxy, alkylthio, alkylsulfinyl, alkylsulfonyl, alkylamino, or an arylalkyl;
   R⁷ is hydrogen, hydroxyl, halogen, cyano, oximyl, alkoxycarbonyl, alkylcarbonyl, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃(NR^{7c})₀₋₁C(=W')WR^{7a};
   R⁸ is hydrogen, hydroxyl, halogen, cyano, oximyl, alkoxycarbonyl, alkylcarbonyl, thiol, nitro, alkyl alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃(NR^{8c})₀₋₁C(=E')ER^{8a};
   R⁹ is hydrogen, hydroxyl, halogen, cyano, oximyl, alkoxycarbonyl, alkylcarbonyl, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃(NR^{9c})₀₋₁C(=Z')ZR^{9a};
   R¹⁰ is hydrogen, hydroxyl, halogen, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic or thionitroso;
   R¹¹ is hydrogen, hydroxyl or alkoxyl.
   R^{7a}, R^{7b}, R^{7c}, R^{7d}, R^{7e}, R^{7f}, R^{8a}, R^{8b}, R^{8c}, R^{8d}, R^{8e}, R^{8f}, R^{9a}, R^{9b}, R^{9c}, R^{9d}, R^{9e}, and R^{9f} are each independently hydrogen, acyl, alkyl, alkenyl, alkynyl, alkoxy, alkylthio, alkylsulfinyl, alkylsulfonyl, alkylamino, arylalkyl, aryl, heterocyclic, heteroaromatic or a prodrug moiety;
   R¹³ is hydrogen, hydroxy, alkyl, alkenyl, alkynyl, alkoxy, alkylthio, aryl, alkylsulfinyl, alkylsulfonyl, alkylamino, or an arylalkyl;
   E is CR^{8d}R^{8e}, S, NR^{8b} or O;
   E' is O, NR^{8f}, or S;
   W is CR^{7d}R^{7e}, S, NR^{7b} or O;
   W' is O, NR^{7f}, or S;
   Z is CR^{9d}R^{9e}, S, NR^{9b} or O;
   Z' is O, S, or NR^{9f},
   Y' and Y are each independently hydrogen, halogen, hydroxyl, cyano, sulfhydryl, amino, alkyl, alkenyl, alkynyl, alkoxy, alkylthio, alkylsulfinyl, alkylsulfonyl, alkylamino, or an arylalkyl; and pharmaceutically acceptable salts, esters and enantiomers thereof.
122. A method for regulating expression of a tet operator-linked nucleotide sequence in a cell containing (i) a target nucleotide sequence operatively-linked to a tetracycline responsive promoter element (TRE) and (ii) a mutated Tet repressor which binds to the TRE in the absence but not in the presence of a substituted tetracycline compound, comprising modulating the concentration of the substituted tetracycline compound in the cell, such that exprese target nucleotide sequence in the cell is regulated, wherein the mutated Tet repressor is selected such that said mutated Tet repressor binds selectively to the TRE only in the absence of the substituted tetracycline compound of formula (II): 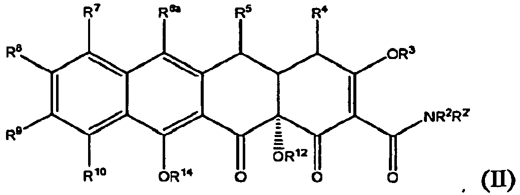 wherein
   R², R^{2'}, R^{4'}, and R^{4"} are each independently hydrogen, alkyl, alkenyl, alkynyl, alkoxy, alkylthio, alkylsulfinyl, alkylsulfonyl, alkylamino, arylalkyl, aryl, heterocyclic, heteroaromatic or a prodrug moiety;
   R³, R¹² and R¹⁴ are each hydrogen or a pro-drug moiety;
   R⁴ is NR^{4'}R^{4"}, alkyl, alkenyl, alkynyl, hydroxyl, halogen, or hydrogen;
   R⁵ is hydroxyl, hydrogen, thiol, alkanoyl, aroyl, alkaroyl, aryl, heteroaromatic, alkyl, alkenyl, alkynyl, alkoxy, alkylthio, alkylsulfinyl, alkylsulfonyl, alkylamino, arylalkyl, alkyl carbonyloxy, or aryl carbonyloxy;
   R^{6a} is hydrogen, hydroxyl, halogen, thiol, alkyl, alkenyl, alkynyl, aryl, alkoxy, alkylthio, alkylsulfinyl, alkylsulfonyl, alkylamino, or an arylalkyl;
   R⁷ is hydrogen, hydroxyl, halogen, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or-(CH₂)₀₋₃(NR^{7c})₀₋₁C(=W')WR^{7a};
   R⁸ is hydrogen, hydroxyl, halogen, thiol, nitro, alkyl, alkenyl, alkynyl, aryl, alkoxy, alkylthio, alkylsulfinyl, alkylsulfonyl, alkylamino, amino, arylalkenyl, arylalkynyl, acyl, aminoalkyl, heterocyclic, thionitroso, or -(CH₂)₀₋₃(NR^{8c})₀₋₁C(=E')ER^{8a}_{;}
   R⁹ is hydrogen, hydroxyl, halogen, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃ (NR^{9c})₀₋₁C(=Z')ZR^{9a};
   R¹⁰ is hydrogen, hydroxyl, halogen, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic or thionitroso;
   R^{7a}, R^{7b}, R^{7c}, R^{7d}, R^{7e}, R^{7f}, R^{8a}, R^{8b}, R^{8c}, R^{8d}, R^{8e}, R^{8f}, R^{9a}, R^{9b}, R^{9c}, R^{9d}, R^{9e}, R^{9f} are each independently hydrogen, acyl, alkyl, alkenyl, alkynyl, alkoxy, alkylthio, alkylsulfinyl, alkylsulfonyl, alkylamino, arylalkyl, aryl, heterocyclic, heteroaromatic or a prodrug moiety;
   W is CR^{7d}R^{7e}, S, NR^{7b} or O;
   W' is O, NR^{7f}, or S;
   E is CR^{8d}R^{8e}, S, NR^{8b} or O;
   E' is O, NR^{8f}, or S;
   Z is CR^{9d}R^{9e}, S, NR^{9b} or O;
   Z' is O, S, or NR^{9f}; and pharmaceutically acceptable salts, esters and enantiomers thereof.
123. The method of any one of para. 119-122, wherein said substituted tetracycline compound is a compound of Table 2.
124. The method of any one of para. 1-123, wherein the substituted tetracycline compound exhibits a Klux of greater than 70 at a concentration of at least about 13 µg/mL.
125. The method of para. 124, wherein the tetracycline compound exhibits a Klux of between about 51 and about 70 at a concentration of at least about 13 µg/mL.

## Claims

1. A method for regulating expression of a tet operator-linked nucleotide sequence in a cell containing (i) a target nucleotide sequence operatively-linked to a tetracycline responsive promoter element (TRE) and (ii) a fusion protein comprising a first polypeptide which binds to the TRE in the presence or absence of a substituted tetracycline compound operatively-linked to a second polypeptide which regulates transcription in cells, comprising modulating the concentration of the substituted tetracycline compound in the cell, such that expression of the target nucleotide sequence in the cell is regulated, wherein the substituted tetracycline compound is of formula (I): 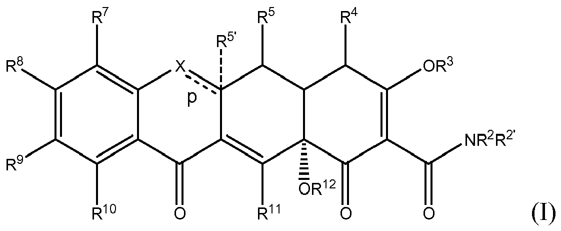 wherein
p is a single or double bond;
X is CHC(R¹³Y'Y)_{,} CR^{6'}R⁶, C=CR^{6'}R⁶, S, NR⁶, or O; and R^{5'} is hydrogen when p is a single bond;
X is CR^{6"} and R^{5'} is absent when p is a double bond;
R², R^{2'}, R^{4'}, and R^{4"} are each independently hydrogen, alkyl, alkenyl, alkynyl, alkoxy, alkylthio, alkylsulfinyl, alkylsulfonyl, alkylamino, arylalkyl, aryl, heterocyclic, heteroaromatic or a prodrug moiety;
R³ and R¹² are each hydrogen or a pro-drug moiety;
R⁴ is NR^{4'}R^{4"}, alkyl, alkenyl, alkynyl, hydroxyl, halogen, or hydrogen;
R⁵ is hydroxyl, hydrogen, thiol, alkanoyl, aroyl, alkaroyl, aryl, heteroaromatic, alkyl, alkenyl, alkynyl, alkoxy, alkylthio, alkylsulfinyl, alkylsulfonyl, alkylamino, arylalkyl, alkyl carbonyloxy, or aryl carbonyloxy;
R⁶ and R^{6'} are each independently hydrogen, methylene, absent, hydroxyl, halogen, thiol, alkyl, alkenyl, alkynyl, aryl, alkoxy, alkylthio, alkylsulfinyl, alkylsulfonyl, alkylamino, or an arylalkyl;
R⁷ is halogen;
R⁸ is hydrogen, hydroxyl, halogen, cyano, oximyl, alkoxycarbonyl, alkylcarbonyl, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃(NR^{8c})₀₋₁C(=E')ER^{8a};
R⁹ is hydrogen, hydroxyl, halogen, cyano, oximyl, alkoxycarbonyl, alkylcarbonyl, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic, thionitroso, or -(CH₂)₀₋₃(NR^{9c})₀₋₁C(=Z')ZR^{9a}_{;}
R¹⁰ is hydrogen, hydroxyl, halogen, thiol, nitro, alkyl, alkenyl, alkynyl, aryl, alkoxy, alkylthio, alkylsulfinyl, alkylsulfonyl, arylalkyl, amino, arylalkenyl, arylalkynyl, acyl, aminoalkyl, heterocyclic or thionitroso;
R¹¹ is hydrogen, hydroxyl or alkoxyl.
R^{7a}, R^{7b}, R^{7c}, R^{7d}, R^{7e}, R^{7f}, R^{8a}, R^{8b}, R^{8c}, R^{8d}, R^{8e}, R^{8f}, R^{9a}, R^{9b}, R^{9c}, R^{9d}, R^{9e}, and R^{9f} are each independently hydrogen, acyl, alkyl, alkenyl, alkynyl, alkoxy, alkylthio, alkylsulfinyl, alkylsulfonyl, alkylamino, arylalkyl, aryl, heterocyclic, heteroaromatic or a prodrug moiety;
R¹³ is hydrogen, hydroxy, alkyl, alkenyl, alkynyl, alkoxy, alkylthio, aryl, alkylsulfinyl, alkylsulfonyl, alkylamino, or an arylalkyl;
E is CR^{8d}R^{8e}, S, NR^{8b} or O;
E' is O, NR^{8f}, or S;
W is CR^{7d}R^{7e}, S, NR^{7b} or O;
W' is O, NR^{7f}, or S;
Z is CR^{9d}R^{9e}, S, NR^{9b} or O;
Z' is O, S, or NR^{9f}; and
Y' and Y are each independently hydrogen, halogen, hydroxyl, cyano, sulfhydryl, amino, alkyl, alkenyl, alkynyl, alkoxy, alkylthio, alkylsulfinyl, alkylsulfonyl, alkylamino, or an arylalkyl; and pharmaceutically acceptable salts, esters and enantiomers thereof.

2. The method of claim 1, wherein the target nucleotide sequence encodes a protein.

3. The method of claim 1 or 2, wherein the tet operator-linked nucleic acid molecule is an exogenous nucleic acid molecule which has been introduced into the cell.

4. The method of any one of claims 1-3, wherein the cell further contains a second target nucleic acid molecule.

5. The method of claim 1, wherein R², R^{2'}, R³ and R¹² are each hydrogen; R⁴ is NR⁴R^{4'}; R⁴ and R^{4'} are each alkyl; and R¹¹ is hydroxyl.

6. The method of claim 5, wherein p is a single bond; X is CR⁶R^{6'}; R⁶ is alkyl; R^{6'} is hydrogen and R⁵ is hydroxyl.

7. The method of claim 5, wherein p is a single bond; X is CR⁶R^{6'}; R⁵, R⁶ and R^{6'} are each hydrogen.

8. The method of claim 7, wherein R⁸ is hydrogen.

9. The method of claim 8, wherein R¹⁰ is hydroxyl.

10. The method of claim 9, wherein R⁷ is iodine.

11. The method of claim 5, wherein p is a double bond; X is CR^{6"}; and R^{6"} is hydrogen.

12. The method of claim 11, wherein R⁸ and R⁹ are hydrogen and R¹⁰ is hydroxyl.

13. The method of claim 12, wherein said halogen is chlorine.

14. The method of claim 1, wherein the substituted tetracycline compound is selected from the group consisting of: 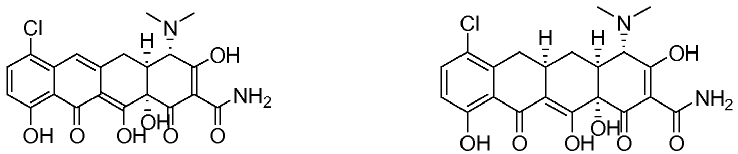 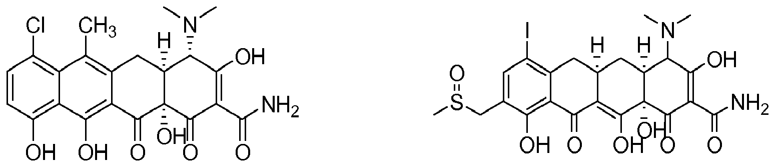 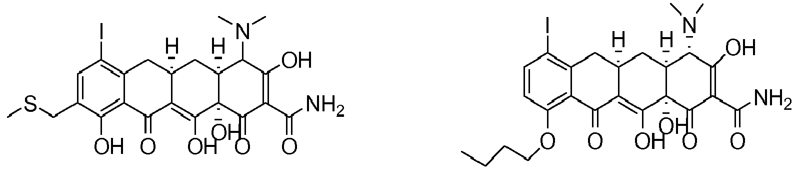 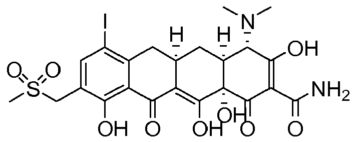 and pharmaceutically acceptable salts thereof.

15. The method of claim 14, wherein the compound is
